# EUROPEAN PATENT SPECIFICATION

(11) **EP 2 569 335 B1**
(45) Date of publication and mention of the grant of the patent: **22.08.2018**
(21) Application number: 11743614.7
(22) Date of filing: 13.05.2011
(51) Int. Cl.: C07K 16/24

(54) **METHODS OF TREATING AND/OR PREVENTING CELL PROLIFERATION DISORDERS WITH IL-17 ANTAGONISTS**
VERFAHREN ZUR BEHANDLUNG UND/ODER PRÄVENTION VON ZELLPROLIFERATIONSSTÖRUNGEN MIT IL-17-ANTAGONISTEN
MÉTHODES DE TRAITEMENT ET/OU DE PRÉVENTION DE TROUBLES DE PROLIFÉRATION CELLULAIRE À L'AIDE D'ANTAGONISTES DE IL-17

(30) Priority: 14.05.2010 US 334979 P
(43) Date of publication of application: 20.03.2013
(73) Proprietor: Orega Biotech, 69130 Ecully (FR); Institut National de la Santé et de la Recherche Médicale, 75654 Paris Cedex 13 (FR)
(72) Inventor: BASTID, Jérémy, 69290 Craponne (FR); BONNEFOY-BERARD, Nathalie, 69003 Lyon (FR); DOREAU-BASTID, Agnès, 69290 Craponne (FR); ELIAOU, Jean-François, 34070 Montpellier (FR)
(74) Representative: Cabinet Laurent & Charras
(86) International application number: PCT/IB2011/001448
(87) International publication number: WO 2011/141823

(56) References cited:
- WO-A1-2007/070750
- WO-A1-2008/118930
- WO-A1-2008/133684
- WO-A2-2007/117749
- WO-A2-2010/115092
- US-A1- 2010 055 108
- ZOU WEIPING ET AL: "T(H)17 cells in tumour immunity and immunotherapy", NATURE REVIEWS IMMUNOLOGY, vol. 10, no. 4, April 2010 (2010-04), pages 248-256, XP002661453,
- CHAE WOOK-JIN ET AL: "Ablation of IL-17A abrogates progression of spontaneous intestinal tumorigenesis", PROCEEDINGS OF THE NATIONAL ACADEMY OF SCIENCES OF THE UNITED STATES OF AMERICA, vol. 107, no. 12, March 2010 (2010-03), XP002661454, ISSN: 0027-8424
- WANG LIN ET AL: "IL-17 can promote tumor growth through an IL-6-Stat3 signaling pathway", JOURNAL OF EXPERIMENTAL MEDICINE, vol. 206, no. 7, July 2009 (2009-07), pages 1457-1464,S1-S2, XP002661455, ISSN: 0022-1007
- NUMASAKI MUNEO ET AL: "Interleukin-17 promotes angiogenesis and tumor growth", BLOOD, vol. 101, no. 7, 1 April 2003 (2003-04-01) , pages 2620-2627, XP002367324, AMERICAN SOCIETY OF HEMATOLOGY, US ISSN: 0006-4971, DOI: 10.1182/BLOOD-2002-05-1461
- DOREAU AGNES ET AL: "Interleukin 17 acts in synergy with B cell-activating factor to influence B cell biology and the pathophysiology of systemic lupus erythematosus", NATURE IMMUNOLOGY, vol. 10, no. 7, July 2009 (2009-07), pages 778-785+METHOD, XP002661456, ISSN: 1529-2908
- KAWAGOE HIROYUKI ET AL: "Overexpression of N-Myc rapidly causes acute myeloid leukemia in mice", CANCER RESEARCH, vol. 67, no. 22, November 2007 (2007-11), pages 10677-10685, XP002661457, ISSN: 0008-5472
- SHIOTA M ET AL: "Twist and p53 reciprocally regulate target genes via direct interaction", ONCOGENE, vol. 27, no. 42, September 2008 (2008-09), pages 5543-5553, XP002661458, ISSN: 0950-9232
- ANSIEAU STEPHANE ET AL: "Induction of EMT by twist proteins as a collateral effect of tumor-promoting inactivation of premature senescence", CANCER CELL, vol. 14, no. 1, July 2008 (2008-07), pages 79-89, XP002661459, ISSN: 1535-6108
- DATABASE BIOSIS [Online] BIOSCIENCES INFORMATION SERVICE, PHILADELPHIA, PA, US; November 2009 (2009-11), JAIN PREETESH ET AL: "Th-17/IL-17 Axis in Chronic Lymphocytic Leukemia", XP002661460, Database accession no. PREV201000353351 & BLOOD, vol. 114, no. 22, November 2009 (2009-11), pages 930-931, 51ST ANNUAL MEETING OF THE AMERICAN-SOCIETY-OF-HEMATOLOGY; NEW ORLEANS, LA, USA; DECEMBER 05 -08, 2009 ISSN: 0006-4971(print)
- PRABHALA RAO H ET AL: "Elevated IL-17 produced by T(H)17 cells promotes myeloma cell growth and inhibits immune function in multiple myeloma", BLOOD, vol. 115, no. 26, 15 April 2010 (2010-04-15), pages 5385-5392, XP002661461,
- DAS ROY LOPAMUDRA ET AL: "Breast cancer-associated metastasis is significantly increased in a model of autoimmune arthritis", BREAST CANCER RESEARCH, CURRENT SCIENCE, LONDON, GB, vol. 11, no. 4, 30 July 2009 (2009-07-30), page R56, XP021061321, ISSN: 1465-5411, DOI: 10.1186/BCR2345
- PANG ET AL: "Celecoxib induces apoptosis in COX-2 deficient human gastric cancer cells through Akt/GSK3beta/NAG-1 pathway", CANCER LETTERS, NEW YORK, NY, US, vol. 251, no. 2, 3 May 2007 (2007-05-03), pages 268-277, XP022059615, ISSN: 0304-3835, DOI: 10.1016/J.CANLET.2006.11.032
- VITAL-REYES V ET AL: "Celecoxib Inhibits Cellular Growth, Decreases Ki-67 Expression and Modifies Apoptosis in Ovarian Cancer Cell Lines", ARCHIVES OF MEDICAL RESEARCH, INSTITUTO MEXICANO DEL SEGURO SOCIAL, MEXICO, MX, vol. 37, no. 6, 1 August 2006 (2006-08-01) , pages 689-695, XP027958951, ISSN: 0188-4409 [retrieved on 2006-08-01]
- LIU H F ET AL: "Celecoxib induces p53-PUMA pathway for apoptosis in human colorectal cancer cells", CHEMICO-BIOLOGICAL INTERACTIONS, ELSEVIER SCIENCE IRLAND, IR, vol. 176, no. 1, 22 October 2008 (2008-10-22), pages 48-57, XP025474212, ISSN: 0009-2797, DOI: 10.1016/J.CBI.2008.07.012 [retrieved on 2008-08-08]
- AMERICAN CANCER SOCIETY: 'How is chemotherapy used to treat cancer?', [Online] Retrieved from the Internet: <URL:https://www.cancer.org/treatment/treat ments-and-side-effects/treatment-types/chem otherapy/how-is-chemotherapy-used-to-treat- cancer.html> [retrieved on 2017-05-26]
- DATABASE BIOSIS [Online] BIOSCIENCES INFORMATION SERVICE, PHILADELPHIA, PA, US September 2013 CHUNG ALICIA S ET AL: 'An interleukin-17-mediated paracrine network promotes tumor resistance to anti-angiogenic therapy' Database accession no. PREV201400690391 & NATURE MEDICINE, vol. 19, no. 9, September 2013 (2013-09), pages 1114-1123, ISSN: 1078-8956(print), DOI: 10.1038/NM.3291
- DATABASE BIOSIS [Online] BIOSCIENCES INFORMATION SERVICE, PHILADELPHIA, PA, US December 2013 COCHAUD STEPHANIE ET AL: 'IL-17A is produced by breast cancer TILs and promotes chemoresistance and proliferation through ERK1/2' Database accession no. PREV201400068109 & SCIENTIFIC REPORTS, vol. 3, December 2013 (2013-12), ISSN: 2045-2322(print), DOI: 10.1038/SREP03456
- DATABASE BIOSIS [Online] BIOSCIENCES INFORMATION SERVICE, PHILADELPHIA, PA, US December 2013 LOTTI FIORENZA ET AL: 'Chemotherapy activates cancer-associated fibroblasts to maintain colorectal cancer-initiating cells by IL-17A' Database accession no. PREV201400103834 & JOURNAL OF EXPERIMENTAL MEDICINE, vol. 210, no. 13, December 2013 (2013-12), pages 2851-2872, ISSN: 0022-1007(print), DOI: 10.1084/JEM.20131195

## Description

### BACKGROUND OF THE INVENTION

### Field of the Invention

The present disclosure relates generally to methods of treating and/or preventing cell proliferation diseases, such as cancers, using antagonists of IL-17. The present disclosure also relates to methods and kits for identifying subjects who are likely to respond to treatment and/or prevention of cell proliferation diseases with antagonists of IL-17.

### Background

Interleukin-17 ("IL-17"), also known as IL-17A and CTLA-8, is a pro-inflammatory cytokine that stimulates secretion of various other cytokines in a variety of cell types. For example, IL-17 can induce IL-6, IL-8, G-CSF, TNF-α, IL-1β, and IFN-γ, as well as numerous chemokines and other effectors. *See, e.g.,* Gaffen, S.L., Arthritis Research & Therapy 6: 240-247 (2004).

IL-17 is expressed by T_{H}17 cells, which are involved in the pathology of inflammation and autoimmunity. It is also expressed by CD8⁺ T cells, γδ cells, NK cells, NKT cells, macrophages and dendritic cells. IL-17 and Th17 are linked to pathogenesis of diverse autoimmune and inflammatory diseases, but are essential to host defense against many microbes, particularly extracellular bacteria and fungi. IL-17 can form homodimers or heterodimers with its family member, IL-17F. IL-17 binds to both IL-17 RA and IL-17 RC to mediate signaling. IL-17, signaling through its receptor, activates the NF-κB transcription factor, as well as various MAPKs. *See, e.g.,* Gaffen, S., Nature Rev. Immunol. 9: 556-567 (2009).

IL-17 can act in cooperation with other inflammatory cytokines such as TNF-α, IFN-γ, and IL-1β to mediate pro-inflammatory effects. *See, e.g.,* Gaffen, S.L., Arthritis Research & Therapy 6: 240-247 (2004). Increased levels of IL-17 have been implicated in numerous diseases, including rheumatoid arthritis (RA), bone erosion, intraperitoneal abscesses, inflammatory bowel disease, allograft rejection, psoriasis, angiogenesis, atheroscloerosis, and multiple sclerosis. *See, e.g.,* Gaffen, S.L., Arthritis Research & Therapy 6: 240-247 (2004); US Publ. No. 2008/-0269467 A1, published Oct. 30, 2008. IL-17 was found in higher serum concentrations in patients with systemic lupus erythematosus (SLE) and was recently determined to act either alone or in synergy with B-cell activating factor (BAFF) to control B-cell survival, proliferation, and differentiation into immunoglobulin producing cells. Doreau et al., Nature Immunology 7:778-785 (2009)

IL-17 and IL-17-producing T_{H}17 cells have recently been implicated in certain cancers, Ji and Zhang, Cancer Immunol Immunother 59: 979-987 (2010). For example, IL-17-expressing T_{H}17 cells were shown to be involved in multiple myeloma, Prabhala et al., Blood, online DOI 10.1182/blood-2009-10-246660, April 15, 2010, and to correlate with poor prognosis in patients with HCC, Zhang et al., J. Hepatology 50: 980-89 (2009). Also, IL-17 was found to be expressed by breast-cancer-associated macrophages, Zhu et al., Breast Cancer Research 10:R95 (2008). However, the role of IL-17 in cancer, in many cases, has been unclear. In particular, IL-17 and IL-17-producing T_{H}17 cells have been identified as having both a positive and a negative role in tumor immunity, sometimes in the same type of cancer. For a review, *see,* Ji and Zhang, Cancer Immunol Immuother 59: 979-987 (2010).

WO2007/117749 describes an anti-IL-17 antibody (AIN457) used for the treatment of cancers, possibly in combination with a chemotherapeutic agent. Das Roy Lopamudra et al. (Breast Cancer Research 11(4): R56 (2009)) describes the treatment with anti-IL-17 and celecoxib (an anti-inflammatory drug) in the context of metastatic breast cancer.

BAFF is a member of the TNF family that is expressed in T cells, macrophages, monocytes, and dendritic eels. It is involved in stimulation of T cell and B cell function (including proliferation and maturation of peripheral B cells). The receptors for BAFF include TACI, BCMAQ, and BAFF-R.

Accordingly, it would be beneficial to have direct indicators of the detrimental effects of IL-17 expression or BAFF and IL-17 expression in cell proliferation disorders, that can be used to identify subjects who are at increased risk for developing a cell proliferation disorder and to identify subjects who are likely to respond to treatments with an IL-17 antagonist or an IL-17 antagonist and a BAFF antagonist, as well as using these indicators in methods of treating and/or preventing cell proliferation disorders or other IL-17-related diseases. Also, it would be beneficial to target the direct effects of IL-17 or IL-17 and BAFF, through the induction of damage and other detrimental effects on cells and in subjects.

### BRIEF SUMMARY OF THE DISCLOSURE

This invention is defined in the the appended claims and any other aspects set forthherein are for information only. In one aspect, it is disclosed a method of treating a cell proliferation disorder in a subject, the method comprising: (a) measuring the amount of IL-17 in a sample from said subject; (b)comparing the measured amount of IL-17 to a reference amount of IL-17 to determine if said subject is likely to respond to treatment of said cell proliferation disorder with an anti-IL17 antagonist, wherein an amount of IL-17 that is greater than said reference amount indicates that the subject is likely to respond; and (c) administering to said subject an IL-17 antagonist in an amount effective to treat said cell proliferation disorder. In one embodiment, the method further comprises determining if Activation Induced Deaminase (AID) expression is increased in a sample from said subject compared to a reference level of AID expression to determine if said subject is likely to respond to treatment of said cell proliferation disorder with an IL-17 antagonist, wherein increased AID expression indicates that said subject is likely to respond.

In another aspect, it is disclosed a method of treating a cell proliferation disorder in a subject, said method comprising: (a) determining if AID expression is increased in a sample from said subject compared to a reference level of AID to identify if said subject is likely to respond to treatment with an anti-IL17 antagonist, wherein increased AID expression indicates that said subject is likely to respond; and (b) administering to said subject an IL-17 antagonist in an amount effective to treat said cell proliferation disorder. In one embodiment, the method of further comprises measuring the amount of IL-17 in a sample from said subject and comparing the measured amount of IL-17 to a reference amount of IL-17 to determine if said subject is likely to respond to treatment with an anti-IL17 antagonist, wherein an amount of IL-17 that is greater than said reference amount indicates that said subject is likely to respond.

In another aspect, it is disclosed a method of preventing a cell proliferation disorder in a subject at increased risk for a cell proliferation disorder, said method comprising: (a) measuring the amount of IL-17 in a sample from said subject; (b) comparing the amount of IL-17 in said sample to a reference amount of IL-17, wherein an amount of IL-17 that is greater than said reference amount indicates an increased risk for a cell proliferation disorder; and (c) administering to said subject an IL-17 antagonist in an amount effective to prevent IL-17-induced transformation of cells into abnormally proliferating cells, thereby preventing said cell proliferation disorder. In one embodiment, the method further comprises determining if AID expression is increased in a sample from said subject compared to a reference level of AID expression to identify if said subject is at increased risk for a cell proliferation disorder, wherein increased AID expression indicates that said subject at increased risk.

In another aspect, it is disclosed a method of preventing a cell proliferation disorder in a subject at increased risk for a cell proliferation disorder, said method comprising: (a) determining if AID expression is increased in a sample from said subject compared to a reference level of AID to identify if said subject is at increased risk for a cell proliferation disorder, wherein increased AID expression indicates an increased risk; and (b) administering to said subject an IL-17 antagonist in an amount effective to prevent an IL-17-induced increase in expression of AID and transformation of cells, thereby preventing said cell proliferation disorder. In one embodiment, the method further comprises measuring the amount of IL-17 in a sample from said subject and comparing the amount of IL-17 in said sample to a reference amount of IL-17, wherein an amount of IL-17 that is greater than the reference amount indicates an increased risk for a cell proliferation disorder.

The invention is directed to a pharmaceutical composition comprising a chemotherapeutic agent and an IL-17 neutralizing antibody or an antigen binding fragment thereof for increasing the sensitivity of cancer cells to a chemotherapeutic agent, for use in the treatment of cancer in a subject having a cancer associated with increased expression or production of IL-17. The invention also concerns products containing a chemotherapeutic agent and an IL-17 neutralizing antibody or an antigen binding fragment thereof for increasing the sensitivity of cancer cells to a chemotherapeutic agent, as a combined preparation for simultaneous, separate or sequential use in the treatment of cancer in a subject having a cancer associated with increased expression or production of IL-17. According to the invention, said chemotherapeutic agent is selected from the group consisting of: docetaxel, doxorubicin, and paclitaxel.

In another aspect, it is disclosed a method of preventing tumor metastases in a subject having a cell proliferation disorder, said method comprising: (a) measuring the amount of IL-17 in a sample from said subject; (b) comparing the amount of IL-17 in said sample to a reference amount of IL-17 to identify if said subject is likely to respond to prevention of said tumor metastases with an IL-17 antagonist, wherein an amount of IL-17 that is greater than said reference amount indicates that said subject is likely to respond; and (c) administering an amount of an IL-17 antagonist effective to prevent tumor metastases in said subject. In one embodiment, the method further comprises determining if TWIST-1 expression is increased in a sample from said subject compared to a reference level of TWIST-1 expression to identify if said subject is at increased risk for tumor metastases, wherein TWIST-1 expression indicates an increased risk for tumor metastases in said subject. In another embodiment, the method further comprises determining if said sample comprises cells with the characteristics of mesenchymal cells.

In another aspect, it is disclosed a method of preventing or reducing IL-17-induced DNA damage in one or more cells of a human subject having or at risk of developing a cell proliferation disorder, said method comprising: (a) measuring the amount of IL-17 in a sample from said subject; (b) comparing the measured amount of IL-17 to a reference amount of IL-17 to determine if said subject is likely to respond to treatment with an anti-IL17 antagonist; and (c) administering to said subject an IL-17 antagonist in an amount effective to prevent or reduce said IL-17-induced DNA damage. In one embodiment, the method of further comprises determining if AID expression is increased in a sample from said subject compared to a reference level of AID expression to identify if said subject is at increased risk for IL-17-induced DNA damage, wherein increased AID expression indicates that said subject at increased risk for IL-17-induced DNA damage.

In another aspect, it is disclosed a method of preventing or reversing IL-17-induced inhibition of the p53 suppressor pathway in one or more cells of a human subject having or at risk of developing a cell proliferation disorder, said method comprising: (a) measuring the amount of IL-17 in a sample from said subject; (b) comparing the measured amount of IL-17 to a reference amount of IL-17 to determine if said subject is likely to respond to treatment with an anti-IL17 antagonist; and (c) administering to said subject an IL-17 antagonist in an amount effective to prevent or reverse said IL-17-induced inhibition of the p53 suppressor pathway. In one embodiment, the method of further comprises determining if TWIST-1 expression is increased in a sample from said subject compared to a reference level of TWIST-1 expression to identify if said subject is at increased risk for IL-17 induced inhibition of the p53 suppressor pathway, wherein TWIST-1 expression indicates an increased risk for IL-17 induced inhibition of the p53 tumor suppressor pathway in said subject.

In a particular embodiment, the cancer is a solid tumor. Advantageously, the solid tumor is selected from the group consisting of breast cancer, hepatocellular carcinoma, ovarian cancer, lung cancer, colorectal cancer, oesophageal cancer, head and neck cancer, renal cell carcinoma, cervical carcinoma, fibrosarcoma, gastric cancer, prostate cancer, and melanoma. In another embodiment, the cancer is a lymphoproliferative disease, advantageously a hematological malignancy, more advantageously selected from the group consisting of lymphoma, acute myeloid leukemia, chronic lymphocytic leukemia, chronic myelogenous leukemia, myeloma, and hairy cell leukemia. In a particular embodiment, the hematological malignancy is a B-cell malignancy. In a more specific embodiment, said B-cell malignancy is non-Hodgkin's lymphoma.

In one aspect, the method disclosed therein further comprises measuring the amount of BAFF and/or IL-6 and/or IL10 in a sample from said subject and comparing the measured amount of BAFF and/or IL-6 and/or IL10 to a reference amount of BAFF and/or IL-6 and/or IL10 to determine if said subject is likely to respond to treatment with an anti-BAFF antagonist and/or an IL-6 antagonist and/or an IL10 antagonist, or a combination of any of an anti-IL17 antagonist and/or an IL-6 antagonist and/or an IL10 antagonist, wherein an amount of BAFF and/or IL-6 and/or IL10 that is greater than said reference amount indicates that said subject is likely to respond; and administering to said subject an anti-BAFF antagonist in an amount to treat said B-cell malignancy. In one embodiment, the method further comprises measuring the amount of BAFF and/or IL-6 and/or IL10 in a sample from said subject and comparing the measured amount of BAFF and/or IL-6 and/or IL10 to a reference amount of BAFF and/or IL-6 and/or IL10 to determine if said subject is at increased risk for B-cell malignancy, wherein an amount greater than the reference amount indicates increased risk. In another embodiment, the method further comprises administering to said subject an anti-BAFF antagonist and/or an IL-6 antagonist and/or an IL10 antagonist, or a combination of any of an anti-IL17 antagonist and/or an IL-6 antagonist and/or an IL10 antagonist in an amount to treat said B-cell malignancy.

In some disclosures, the subject has an anomaly of the immune system. In a specific embodiment, the subject is immunocompromised. In another specific embodiment, the subject is a tissue or organ transplant recipient. In another specific embodiment, the subject has an autoimmune disorder. In a more specific embodiment, the autoimmune disorder is systemic lupus erythematosus or rheumatoid arthritis.

In some disclosures, the sample is selected from the group consisting of an organ sample, a tissue sample, a cell sample, and a blood sample. In some embodiments the sample comprises cancer cells.

In some disclosures, IL-17 antagonist prevents a virus-induced transformation of hepatocytes in a subject. In a more specific embodiment, the virus is selected from the group consisting of HBV and HCV.

In some disclosures, the IL-17 antagonist is selected from the group consisting of a small molecule, an IL-17-specific antigen binding molecule, a nucleic acid antagonist, and a protein antagonist. In a more specific embodiment, the antigen binding molecule is selected from the group consisting of an antibody and an antigen binding antibody fragment.

In one aspect, it is disclosed a method of preventing or reducing IL-17-induced DNA damage in a mammalian cell, more specifically a human cell, said method comprising contacting said cell with an IL-17 antagonist in an amount effective to prevent or reduce said IL-17-induced DNA damage. In a specific embodiment, the IL-17-induced DNA damage is a result of overexpression of AID. In another specific embodiment, the IL-17-induced DNA damage is a result of overexpression of TWIST-1. In another specific embodiment, the IL-17-induced DNA damage is a result of inhibition of the p53 tumor suppressor pathway. In another specific embodiment, the IL-17-induced DNA damage is a result of a combination of overexpression of IL-17, AID, and/or TWIST-1. In one embodiment, the mammalian cell is a mammary cell. In another embodiment, the mammalian cell is a hepatocyte. In another embodiment, the hepatocyte is a hepatocellular carcinoma cell. In another embodiment, the hepatocyte is infected with a virus. In a more specific embodiment, the virus is selected from the group consisting of HBV and HCV. In one embodiment, the IL-17 antagonist prevents a virus-induced transformation of said hepatocyte. In another embodiment, the mammalian cell is a B-cell. In a more specific embodiment, the B-cell is a cancerous B-cell. In an even more specific embodiment, the cancerous B-cell is a non-Hodgkins lymphoma cell. In one embodiment, said cell is in a subject who is at increased risk for a B-cell cancer. In another embodiment, the subject suffers from an autoimmune disorder. In a more specific embodiment, the autoimmune disorder is selected from the group consisting of systemic lupus erythematosus and rheumatoid arthritis. In one embodiment, the method further comprises contacting said cell with a BAFF antagonist. In a specific embodiment, the BAFF antagonist is selected from the group consisting of a small molecule, a BAFF-specific antigen binding molecule, a nucleic acid antagonist, and a protein antagonist. In a more specific embodiment, the antigen binding molecule is a selected from the group consisting of an antibody and an antigen binding antibody fragment.

In another aspect, it is disclosed a method of preventing or reverting IL-17-induced transformation of a mammalian cell, said method comprising contacting said cell with an IL-17 antagonist in an amount effective to prevent or revert said IL-17-induced transformation. In another aspect, it is disclosed a method of preventing or reverting IL-17-induced abnormal survival of a mammalian cell, said method comprising contacting said cell with an IL-17 antagonist in an amount effective to prevent or revert said IL-17-induced survival. In another aspect, it is disclosed a method of inducing cell death of an IL-17-expressing cancer cell in a subject, said method comprising contacting said cell with an IL-17 antagonist in an amount effective to induce cell death. In another aspect, it is disclosed a method of inhibiting primary tumor growth in a subject, said method comprising contacting said primary tumor with an IL-17 antagonist in an amount effective to inhibit primary tumor growth. In one embodiment, the mammalian cell is a cancer cell. In a particular embodiment, the mammalian cell is *in vivo* in a human subject. In another embodiment, the method further comprises measuring the amount of IL-17 in a sample from said subject; comparing the measured amount of IL-17 to a reference amount of IL-17 to determine if said subject is likely to respond to treatment with an anti-IL17 antagonist, wherein an amount of IL-17 that is greater than said reference amount indicates that the subject is likely to respond. In another embodiment, the method further comprises determining if AID expression is increased in a sample from said subject compared to a reference level of AID expression to identify if said subject is likely to respond to treatment with an IL-17 antagonist, wherein increased AID expression indicates that said subject is likely to respond. In another embodiment, the method further comprises determining if TWIST-1 expression is increased in a sample from said subject compared to a reference level of TWIST-1 expression to identify if said subject is likely to respond to treatment with an IL-17 antagonist, wherein increased TWIST-1 expression indicates that said subject is likely to respond

In one aspect, the present disclosure is directed to an IL-17 antagonist for the prevention or reduction of IL-17-induced DNA damage in a mammalian cell. In one embodiment, the IL-17-induced DNA damage is a result of upregulation of AID. In another embodiment, the IL-17-induced DNA damage is a result of upregulation of TWIST-1. In another embodiment, the IL-17-induced DNA damage is a result of inhibition of the p53 tumor suppressor pathway.

In another aspect, the present disclosure is directed to an IL-17 antagonist for the prevention or reversion of an epithelial to mesenchymal transition (EMT) of a cell. In one embodiment, said epithelial cell is a breast cell. In a more specific embodiment, the breast cell is a breast cancer cell. In another embodiment, the cell is a hepatocyte. In a specific embodiment, the hepatocyte is infected with a virus. In a more specific embodiment, the IL-17 antagonist prevents a virus-induced transformation of said hepatocyte. In an even more specific embodiment, the virus is selected from the group consisting of HBV and HCV. In a specific embodiment, the hepatocyte cell is a hepatocellular carcinoma cell. In another embodiment, the IL-17 antagonist further comprises an a BAFF antagonist.

### BRIEF DESCRIPTION OF THE DRAWINGS/FIGURES

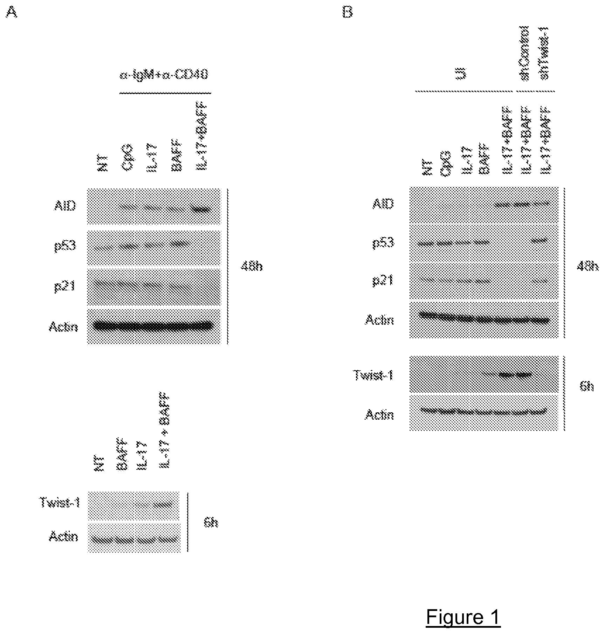

Figure 1: IL-17 and BAFF induce Activation Induced Deaminase (AID) and Twist-1 expression (an inhibitor of p53) in human B lymphocytes. (A) CD19+ peripheral human B lymphocytes were non-treated (NT) or stimulated with anti-IgM (a-IgM, 20 µg/mL) and anti-CD40 (α-CD40, 20 µg/mL) in the presence of CpG2006 (CpG, 2.5 µg/mL), IL-17 (1 ng/mL), BAFF (100 ng/mL) or a combination of IL-17 and BAFF as indicated. After 6 or 48h AID, p53, p21, Twist-1 and actin protein expression was assessed by immunoblotting. Actin was used as a loading control. (B) IL-17 and BAFF inhibit the p53 pathway via Twist-1 upregulation. B104 human B lymphoma cells were either uninfected (UI) or transduced using shRNA lentiviral constructs targeting the *LUCIFERASE* gene (shControl), or *TWIST1* (shTwist-1) mRNA. 48 h after infection, cells were placed in medium alone (NT) or in medium supplemented with CpG2006 (CpG, 2.5 µg/mL), IL-17 (1 ng/mL), BAFF (100 ng/mL) or a combination of IL-17 and BAFF for 6 or 48h, then AID, p53, p21, Twist-1 and actin protein expression was assessed by immunoblot. Actin was used as a loading control.

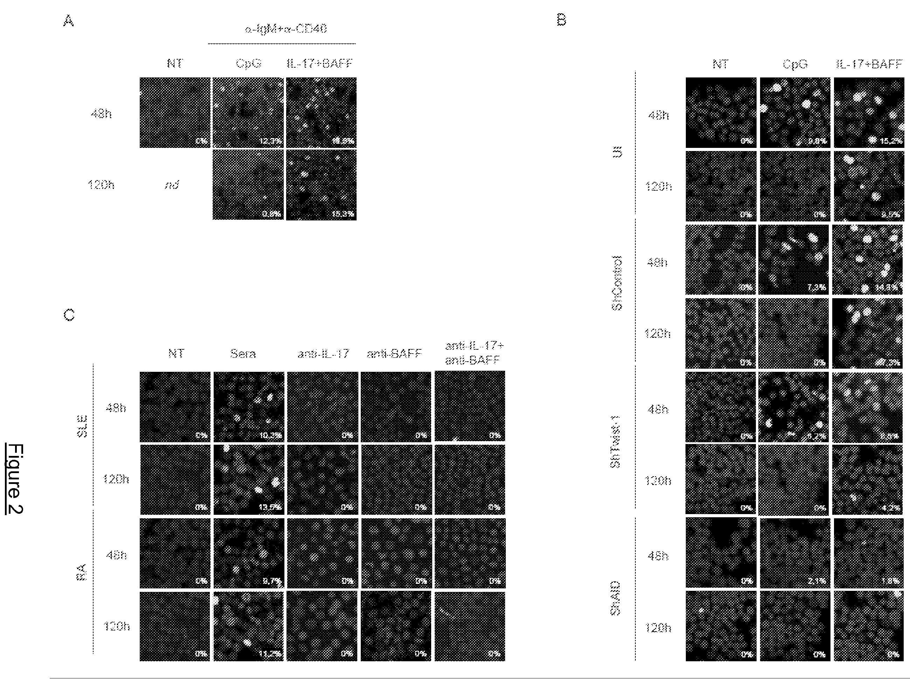

Figure 2: IL-17 and BAFF induce persistent DNA damage in human B lymphocytes. (A) CD19+ peripheral human B lymphocytes were non-treated (NT) or stimulated with anti-IgM (a-IgM, 20 µg/mL) and anti-CD40 (α-CD40, 20 µg/mL) in the presence of CpG2006 (CpG, 2.5 µg/mL), IL-17 (1 ng/mL), BAFF (100 ng/mL) or a combination of IL-17 and BAFF as indicated. (B) B104 human B lymphoma cells were either uninfected (UI) or transduced using shRNA lentiviral constructs targeting the *LUCIFERASE* gene (shControl), *TWIST1* (shTwist-1) or *AICDA* (shAID) mRNA. 48 h after infection, cells were placed in medium alone (NT) or media supplemented with CpG2006 (CpG, 2.5 µg/mL) or IL-17 (1 ng/mL) and BAFF (100 ng/mL) as indicated. (C) B104 human B lymphoma cells were cultured in the presence of medium alone (NT) or medium supplemented with 20% sera from Systemic Lupus Erythematosus (SLE) or Rheumatoid Arthritis (RA) patients in the presence or absence of BAFF-neutralizing antibodies (300 ng/mL) and/or IL-17-neutralizing antibodies (300 ng/mL) as indicated. (A, B and C). DNA alterations were measured at 48 and 120h by staining with Alexa 488 anti-phospho-yH2AX antibody and analyzed by confocal microscopy. Nuclei were counterstained with DRAQ5 (Cell Signalling).

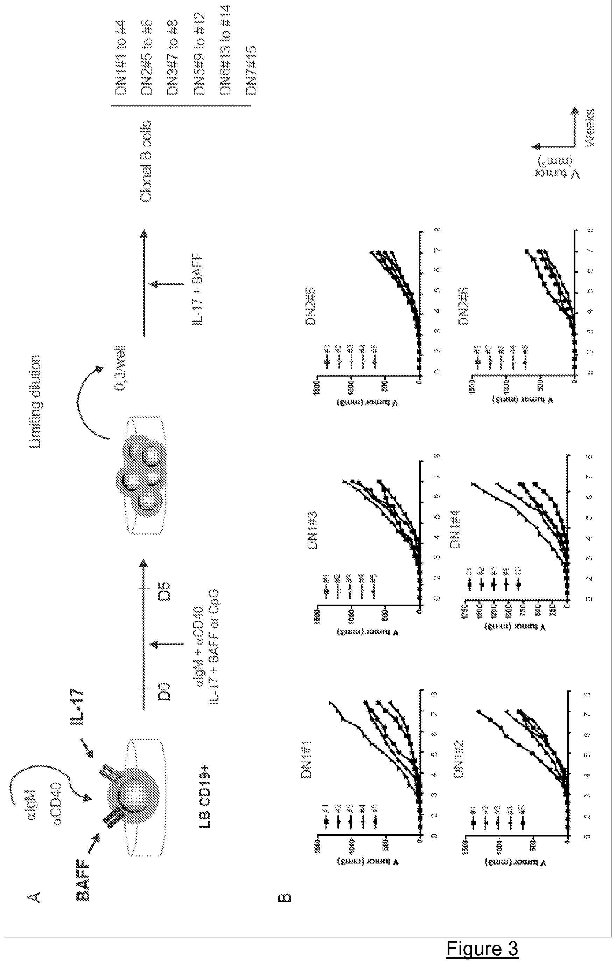

Figure 3: Stimulation of human B lymphocytes in the presence of IL-17 and BAFF allows immortalization of tumorigenic B cell clones. (A) CD19⁺ peripheral human B lymphocytes were stimulated with anti-IgM (αIgM, 20 µg/mL) and anti-CD40 (αCD40, 20 µg/mL) in the presence of CpG2006 (CpG, 2.5 µg/mL) or IL-17 (1 ng/mL) and BAFF (100 ng/mL) for 5 days as indicated, and then activated B cells were cloned by limiting dilution (0.3 cell/well) in medium supplemented with CpG2006 (CpG, 2.5 µg/mL) or IL-17 (1 ng/mL) and BAFF (100 ng/mL). Appearance of clones (hereafter referred as "B cell clones") was checked every week by phase-contrast microscopy. Once established, B cell clones were cultured in medium without cytokines. The experiment was performed with purified B lymphocytes from 7 different donors. Four B cell clones were obtained from donor 1 (DN1#1, DN1#2, DN1#3 and DN1#4), 2 B cell clones from donor 2 (DN2#5 and DN2#6), 2 B cell clones from donor 3 (DN3#7 and DN3# 8) 4 B cell clones from donor 5 (DN5#9, DN5#10, DN5#11 and DN5#12), 2 B cell clones from donor 6 (DN6#13 and DN6#14) and 1 B cell clone from donor 7 (DN7#15). (B) B cell clones DN1#1, DN1#2, DN1#3, DN1#4, DN2#5 and DN2#6 were subcutaneously grafted (10⁶ cells) in the flank of irradiated athymic Swiss nude mice (5 mice per group), tumor growth was monitored twice a week with calipers at the site of injection.

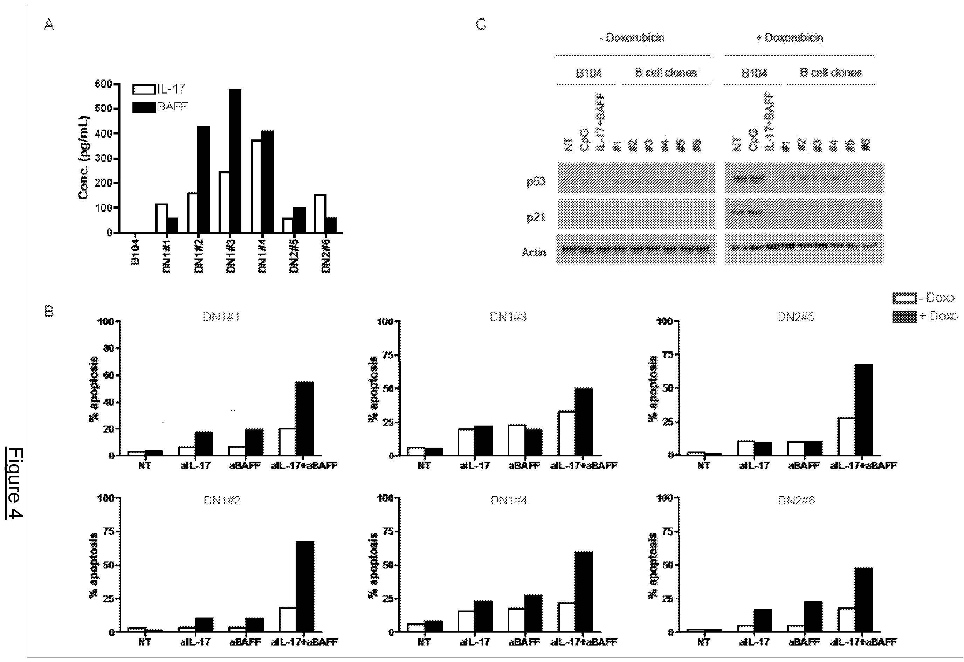

Figure 4: Autocrine production of IL-17 and BAFF protects B cell clones from doxorubicin-mediated cell death. (A) B cell clones secrete IL-17 and BAFF. B104 human B lymphoma cells and B cell clones DN1#1, DN1#2, DN1#3, DN1#4, DN2#5 and DN2#6 (see Figure 3, above) were seeded at 10⁵ cell/mL in medium alone and maintained in culture for 5 days. At day 5, IL-17 and BAFF concentration in cell culture supernatants was determined by ELISA. (B) IL-17 and BAFF neutralizing antibodies induce B cell clone apoptosis and increase doxorubicin-induced apoptosis. B cell clones DN1#1, DN1#2, DN1#3, DN1#4, DN2#5 and DN2#6 were cultured in medium alone or supplemented with IL-17- (aIL-17, 300 ng/mL) and/or BAFF- (aBAFF, 300 ng/mL) neutralizing antibodies for 24h and further treated (+ Doxo) or not (- Doxo) with doxorubicin (300 nM) for 96h as indicated. Viability was assessed by propridium iodide uptake and flow cytometry analysis. (C) Autocrine production of IL-17 and BAFF inhibits the p53 pathway in B cell clones. B104 human B lymphoma cell line, either non-treated (NT) or pre-treated for 24h in the presence of CpG2006 (CpG, 2.5 µg/mL) or IL-17 (1 ng/mL) and BAFF (100 ng/mL) as indicated, as well as clones DN1#1, DN1#2, DN1#3, DN1#4, DN2#5 and DN2#6 were treated or not with doxorubicin (300 nM) for 6h and then p53, p21 and actin protein expression was assessed by immunoblotting. Actin was used as a loading control.

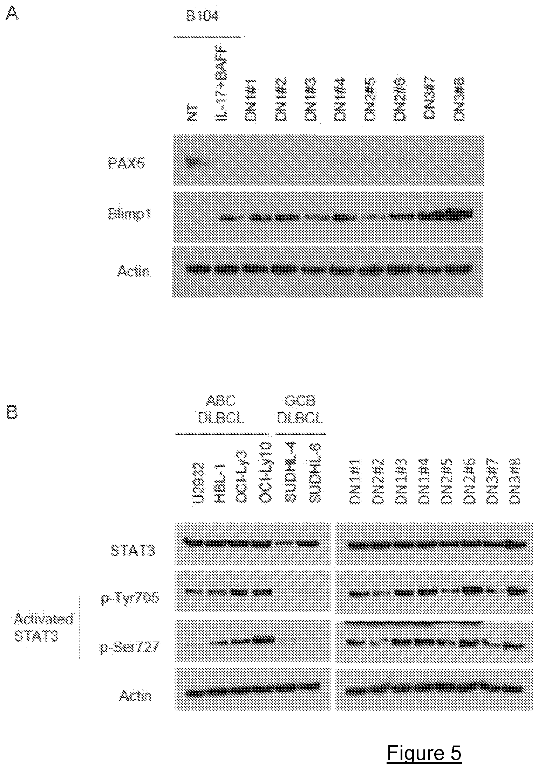

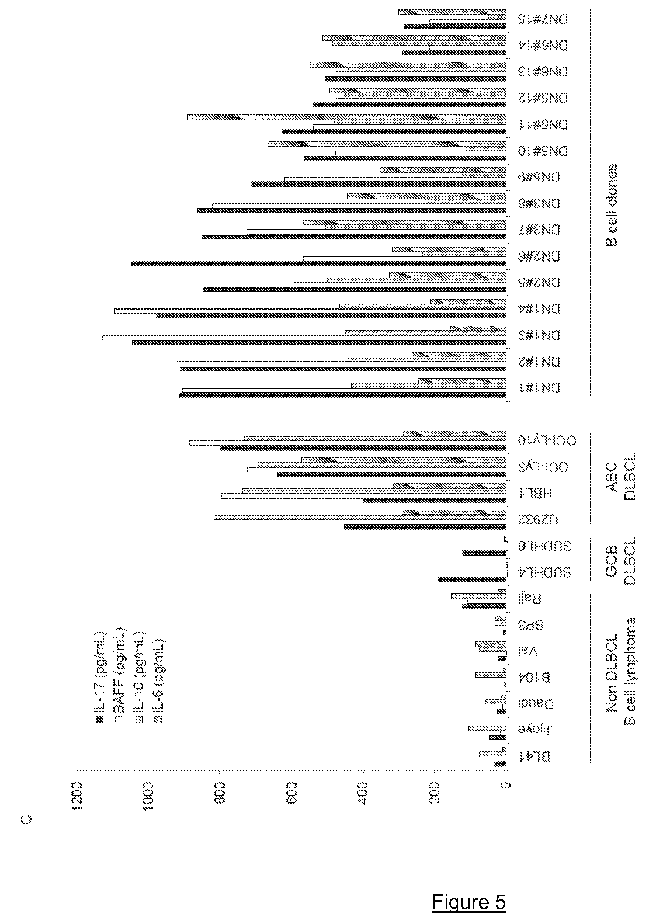

Figure 5: B cell clones generated by exposure to IL-17 and BAFF have a plamablast like phenotype and share special features of ABC-DLBCL. (A) B cell clones generated by exposure to IL-17 and BAFF have a plasmablast-like phenotype as they express BLIMP 1 but not PAX5. BLIMP 1, PAX5 and Actin protein expression from B cell clones DN1#1-4, DN2#4-6, DN3#7-8 was assed by immunoblotting. The B104 human B lymphoma cell line was used as a negative control (NT=untreated control), B104 cells stimulated with IL-17 and BAFF as a positive control (IL-17+BAFF). Actin was used as a loading control. (B) B cell clones generated by exposure to IL-17 and BAFF have and share special features of ABC-DLBCL. Like ABC-DLBCL cell lines, B cell clones DN1#1-4, DN2#4-6, DN3#7-8 generated by exposure to IL-17 and BAFF have an activated Stat3. Phosphorylation of Tyrosine 705 (p-Tyr705) and Serine 727 (p-Ser727) indicates activation of Stat3. Actin was used as a loading control. (C) Like ABC-DLBCL cell lines, B cell clones generated with IL-17 and BAFF secrete high levels of IL-17, BAFF, IL-6 and IL-10. B cell clones and various human lymphoma DLBCL and non DLBCL cell lines were seeded at 0.5 10⁶ cells per ml and concentrations of IL-6, IL-10, IL-17, and BAFF were measured in the cell culture supernatant of 5 day cultured cells. "ABC-DLBCL" refers to cell lines of "Activated B Cell like-Diffuse Large B Cell Lymphoma" subtype. "GCB-DLBCL» refers to cell lines of "Germinal Center B-like cell-Diffuse Large B Cell Lymphoma" subtype.

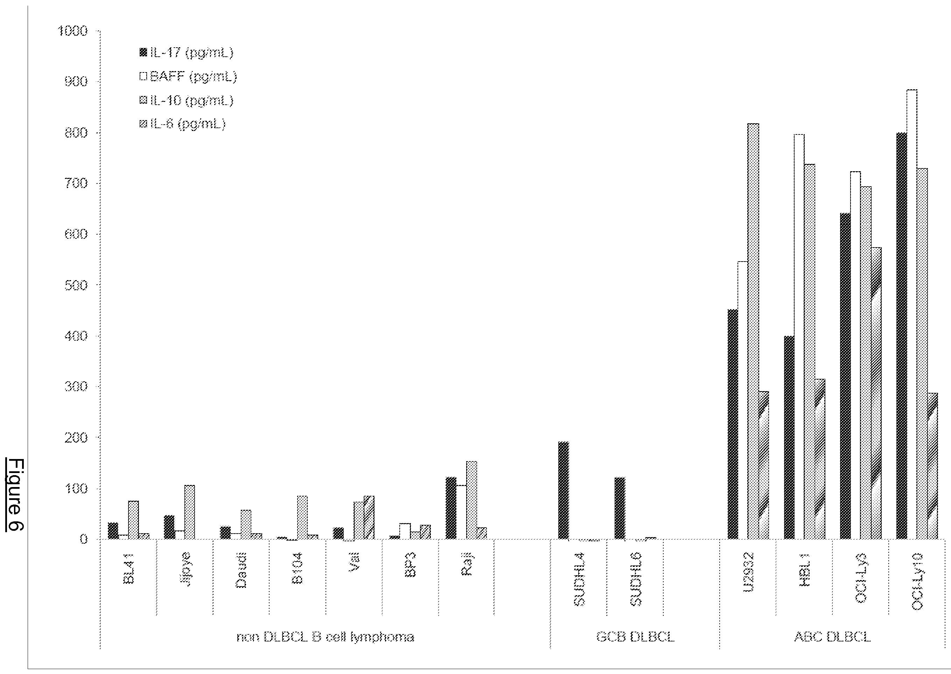

Figure 6: Human ABC-DLBCL lymphoma cell lines secrete IL-17, BAFF, IL-6 and IL-10. (A) Autocrine production of IL-17, BAFF, IL-6 and IL-10 is specific from human ABC-DLBCL lymphoma cell lines. Concentration of IL-17, BAFF, IL-6 and IL-10 were measured in the cell culture supernatant of 5 day cultures of various human Non-Hodgkin's Lymphoma cell lines. "ABC-DLBCL" refers to cell lines of "Activated B Cell like-Diffuse Large B Cell Lymphoma" subtype. "GCB-DLBCL» refers to cell lines of "Germinal Center B-like cell-Diffuse Large B Cell Lymphoma" subtype.

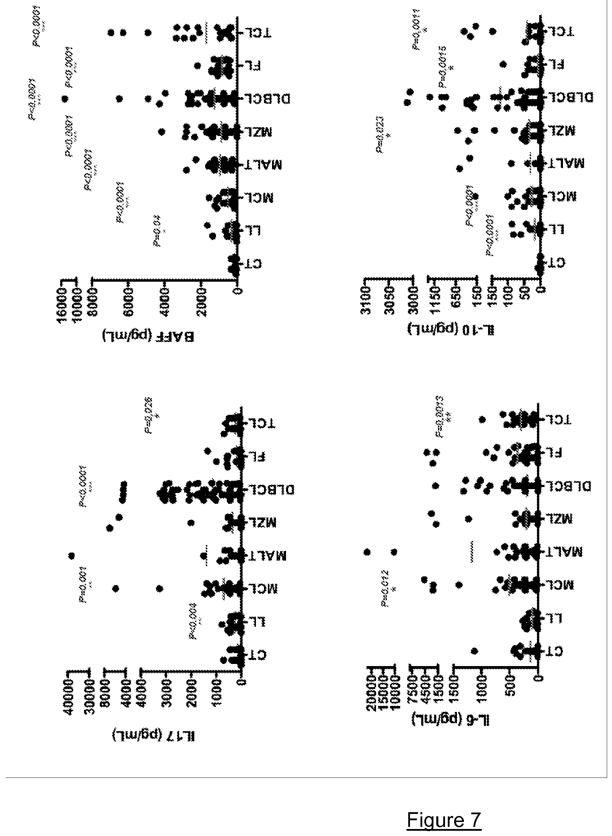

Figure 7: IL-17 BAFF as well as IL-6, IL-10 are increased in blood samples of DLBCL patients. Concentration of IL-17 BAFF, IL-6, IL-10 in blood samples of 40 healthy donors (CT, control) 31 LL, 33 MALT, 39 MCL, 64 MZL, 30 TCL, 49 FL and 112 DLBCL lymphoma patients. P values < 0.05 indicate statistically significant differences (unpaired student t test). LL=Lymphocytic leukemia; MALT=Mucosa associated lymphoid tissue; MCL=Mantle cell lymphoma; MZL=Marignal zone lymphoma; TCL=T cell lymphoma; FL=Follicular lymphoma; DLBCL=Diffuse large B cell lymphoma.

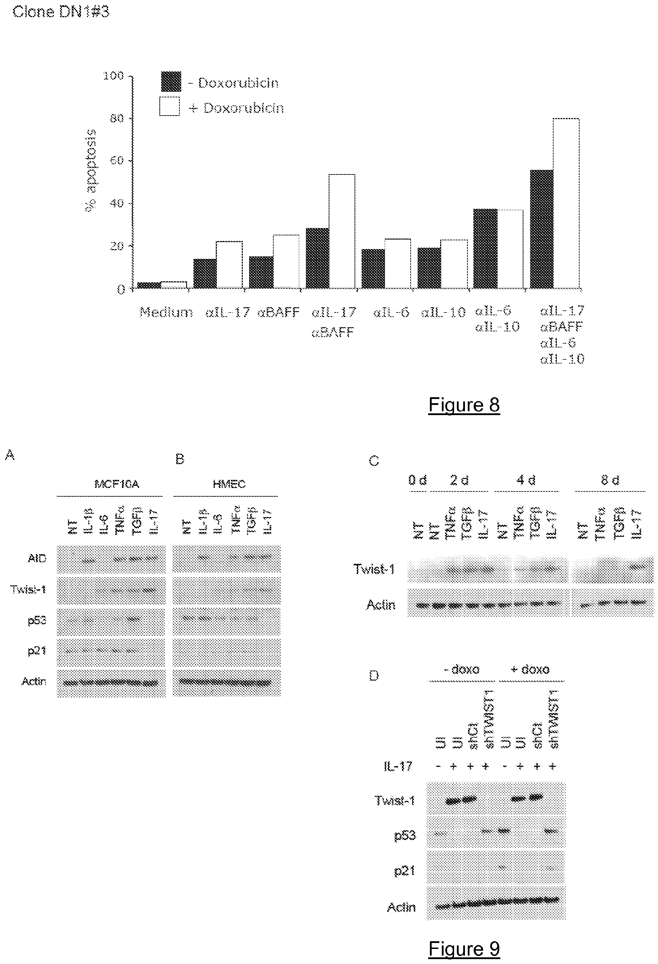

Figure 8: Neutralizing antibodies to IL-17, BAFF, IL-6, and IL-10 induce apoptosis and sensitize B cell clones to chemotherapy (doxorubicin). ABC-DLBCL like B cell clone DN1#3 was cultured in medium alone or supplemented with IL-17- (αIL-17, 1 µg/mL) and/or BAFF- (αBAFF, 1 µg/mL) and/or IL-6- (αIL-6, 1 µg/mL) and/or IL-10- (αIL-10, 1 µg/mL) neutralizing antibodies for 24 h and further treated or not with doxorubicin (300 nM) for 96 h as indicated. Viability was assessed by propridium iodide uptake and flow cytometry analysis.
Figure 9: IL-17 induces Activation Induced Deaminase (AID) and Twist-1 (a p53 inhibitor) expression in mammary epithelial cells. (A) Human mammary epithelial cells (HMEC) and (B) human immortalized mammary epithelial cells, MCF10A, were either non treated (NT) or stimulated for 48 h with IL-1β (50 ng/mL), IL-6 (50 ng/mL), TNFα (100 ng/mL), TGFβ (8 ng/mL) or IL-17 (10 ng/mL). Then AID, p53, p21, Twist-1 and actin protein expression was assessed by immunoblotting. Actin was used as a loading control. (C) Only IL-17 induces stable expression of Twist-1. MCF10A were either non treated (NT) or stimulated with IL-1β (50 ng/mL), IL-6 (50 ng/mL), TNFα (100 ng/mL), TGFβ (8 ng/mL) or IL-17 (10 ng/mL) for 0, 2, 4 or 8 days. Then Twist-1 and actin protein expression was assessed by immunoblotting. Actin was used as a loading control. (D) IL-17 inhibits the p53 pathway via Twist-1 upregulation. MCF10A were transduced or not (UI) using shRNA lentiviral constructs targeting the *LUCIFERASE* gene (shCt) or *TWIST1* gene (shTWIST1) mRNA. 48 h after infection, cells were treated or not for 48 h with IL-17 (10 ng/mL) and/or doxorubicin (+doxo, 300 nM) as indicated. Then Twist-1, p53, p21, and actin protein expression was assessed by immunoblotting. Actin was used as a loading control.

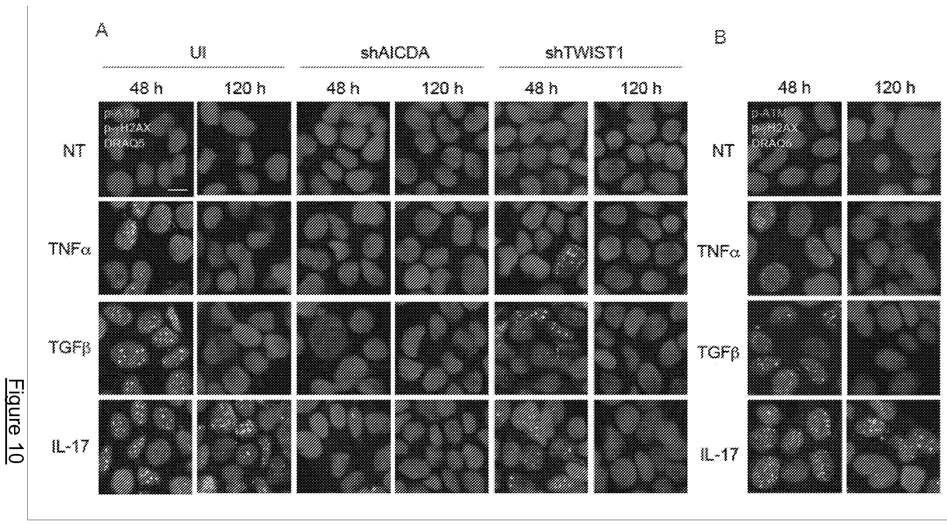

Figure 10: IL-17 induces persistent DNA damage in human mammary epithelial cells. (A) Human immortalized mammary epithelial cells (MCF10A) were either uninfected (UI) or transduced using shRNA lentiviral constructs targeting the *LUCIFERASE* gene (shControl), *AICDA* gene (shAID) or *TWIST1* gene (shTWIST1) mRNA. 48 h after infection, cells were placed in medium alone (NT) or media supplemented with TNFα (100 ng/mL), TGFβ (8 ng/mL) or IL-17 (10 ng/mL) as indicated. DNA alterations were measured at 48h and 120h by staining with Alexa 488 anti-phospho-yH2AX and anti-phospho ATM antibodies and analyzed by confocal microscopy. The nuclei were counterstained with DRAQ5 (Cell Signalling). Representative micrographs are shown. (B) Human mammary epithelial cells (HMEC) were placed in medium alone (NT) or media supplemented with TNFα (100 ng/mL), TGFβ (8 ng/mL) or IL-17 (10 ng/mL) as indicated. DNA alterations were measured at 48 h and 120 h by staining with Alexa 488 anti-phospho-yH2AX and anti-phospho ATM antibodies and analyzed by confocal microscopy. The nuclei were counterstained with DRAQ5 (Cell Signalling). Representative micrographs are shown. Scale bar = 20 µm.

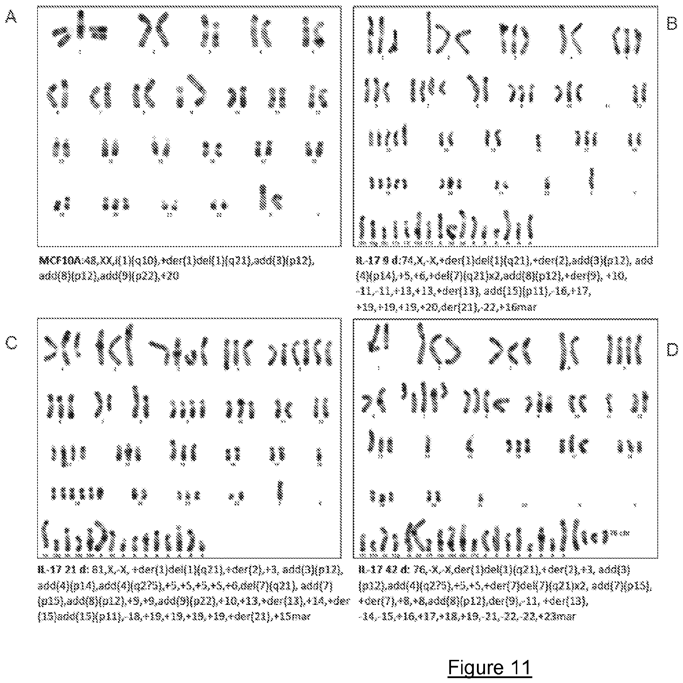

Figure 11: IL-17 induces genomic instability in mammary epithelial cells: chromosomal alteration assessed by karyotyping. Individual karyotypes of human immortalized mammary epithelial cells (MCF10A) showing chromosomal alterations after no exposure to IL-17 (A), 9 days (B), 21 days (C) and 42 days (D) after exposure to IL-17 (10 ng/mL). The karyotype formula is indicated.

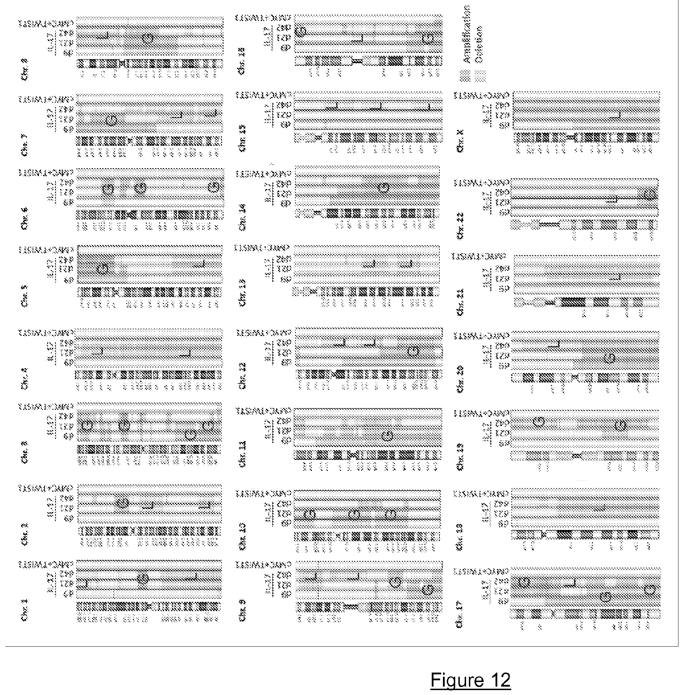

Figure 12: IL-17 induces genomic instability in mammary epithelial cells: chromosomal alteration assessed by CGH array. CGH profils of human immortalized mammary epithelial cells (MCF10A) MCF10A cells exposed to IL-17 (10 ng/mL) for 9 (d9), 21 (d21) and 42 (d42) days compared to parental MCF10A. Regions with DNA amplifications are represented by grey areas marked with "G" (gain). Regions with DNA losses are represented by grey areas marked with a "L" (loss). cMYC+TWIST1 is a combination of factors known to transform cells into cells able to generate tumors in nude mice. The same cells transformed by defined genetic event do not show genomic instability, demonstrating that genomic instability is indeed induced by IL-17.

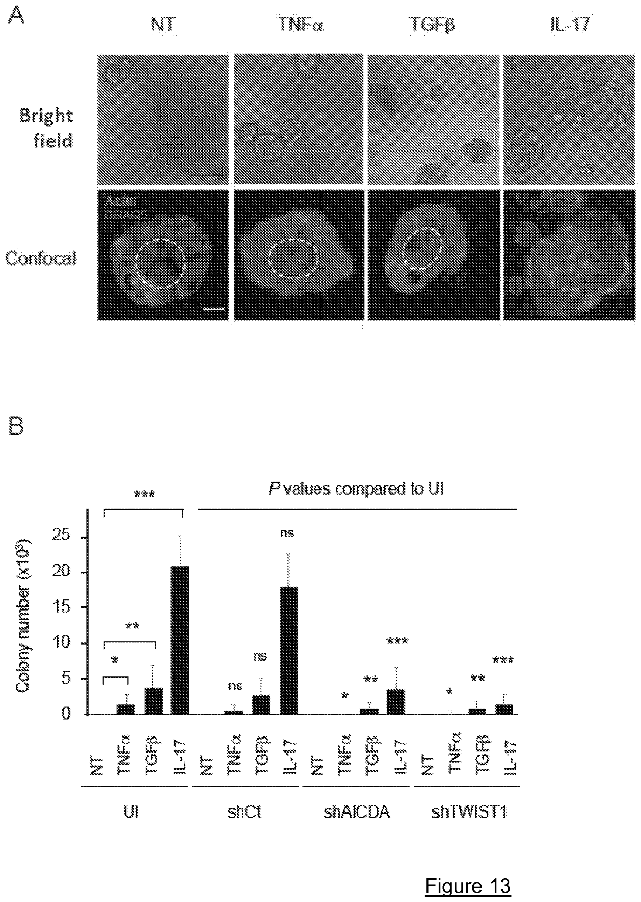

Figure 13: IL-17 disrupts mammary epithelial tissue homeostasis and induces transformation of mammary epithelial cells. (A) Human immortalized mammary epithelial cells (MCF10A) were left untreated (NT) or stimulated with TNFα (100 ng/mL), TGFβ (8 ng/mL) or IL-17 (10 ng/mL) for 9 days as indicated. Then they were cultured for 21 days in 3D in matrigel in order to assess their ability to form normal acinar structures visualized by bright field microscopy (upper panel) or immunofluorescence and confocal microscopy (lower panel). Representative micrographs of acinar structures are shown. Scale bar, 200 µm (upper panel) and 20 µm (lower panel). (B) MCF10A cells were either uninfected (UI) or transduced using shRNA lentiviral constructs targeting the *LUCIFERASE* gene (shCt), *AICDA* gene (shAICDA) or *TWIST1* gene (shTWIST1) mRNA. MCF10A were then left untreated (NT) or stimulated with TNFα (100 ng/mL), TGFβ (8 ng/mL) or IL-17 (10 ng/mL) for 21 days as indicated. Then they were cultured for 21 days in soft agar and the number of colonies was calculated. P values for student's t-test are indicated (*=P<0.05, **= P<0.01, ***=P<0.001). Both AID and Twist-1 are required for IL-17 induced transformation of mammary epithelial cells.

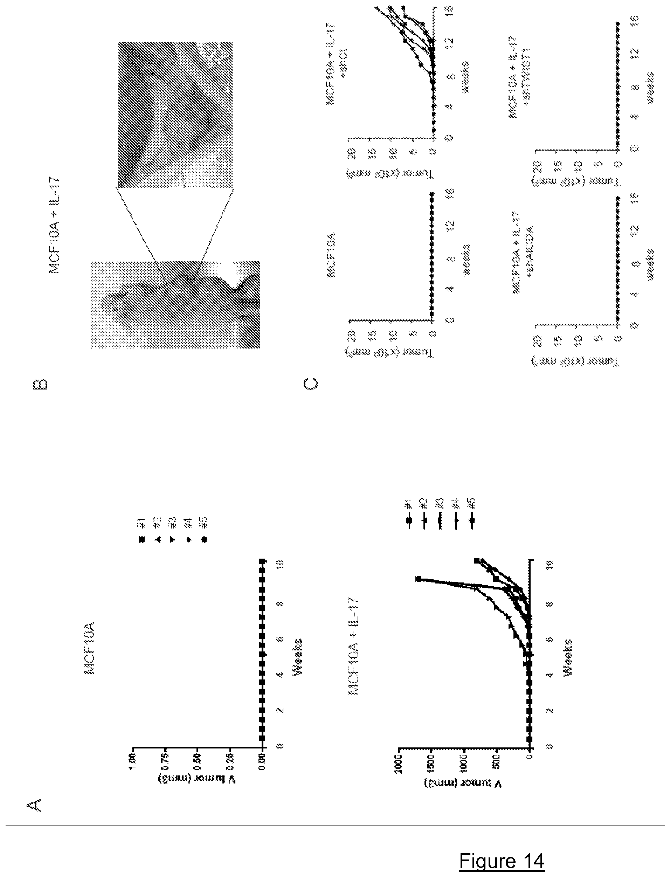

Figure 14: IL-17 induces *in vivo* tumorigenicity of mammary epithelial cells. (A) Human immortalized mammary epithelial cells (MCF10A) were either untreated (MCF10A) or stimulated *in vitro* with IL-17 (10 ng/mL, MCF10A + IL-17) for 21 days. 5.10⁶ cells were subcutaneously grafted in the flank of irradiated athymic Swiss nude mice (5 mice per group) and tumor growth was monitored twice a week with calipers at the site of injection. (B) Representative image of the tumors observed in mice engrafted with IL-17-stimulated MCF10A cells. (C) Growth curves of subcutaneously engrafted MCF10A cells that were uninfected (MCF10A) or transduced using shRNA lentiviral constructs targeting the *LUCIFERASE* gene (shCt), *AICDA* gene (shAICDA) or *TWIST1* gene (shTWIST1) mRNA and treated or not for 21 days with IL-17 (10 ng/ml), as indicated. Both AID and Twist-1 are required for IL-17 induced tumorigenicity.

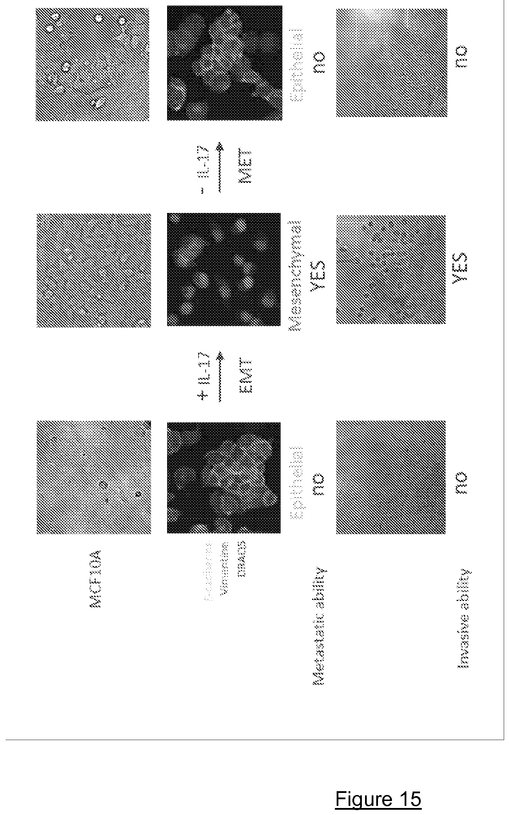

Figure 15: IL-17 induces Epithelial to Mesenchymal Transition (EMT) and invasive properties in mammary epithelial cells. Human immortalized mammary epithelial cells (MCF10A) were either untreated (1^{st} column), treated with IL-17 (+IL-17, 10 ng/mL) for 9 days (2^{nd} column) or treated with IL-17 (+IL-17, 10 ng/mL) for 9 days and then placed back in medium lacking IL-17 for 7 days (-IL-17, 3^{rd} column). IL-17 induces EMT in MC10A characterized by a switch from cobblestone-like to spindle like morphology (upper lane) visualized by bright field microscopy and by loss of the epithelial marker E-Cadherin (which, in color, fluoresces green) and gain of the mesenchymal marker Vimentin (which, in color, fluoresces red) as assessed by immunofluorescence (middle lane). IL-17 confers invasive ability to MCF10A cells as assessed by cluster assay (bottom lane). Removal of IL-17 can revert EMT (a process called mesenchymal to epithelial transition, MET) and invasiveness. Cell phenotype (epithelial or mesenchymal) as well as predicted metastatic and invasive ability are indicated.

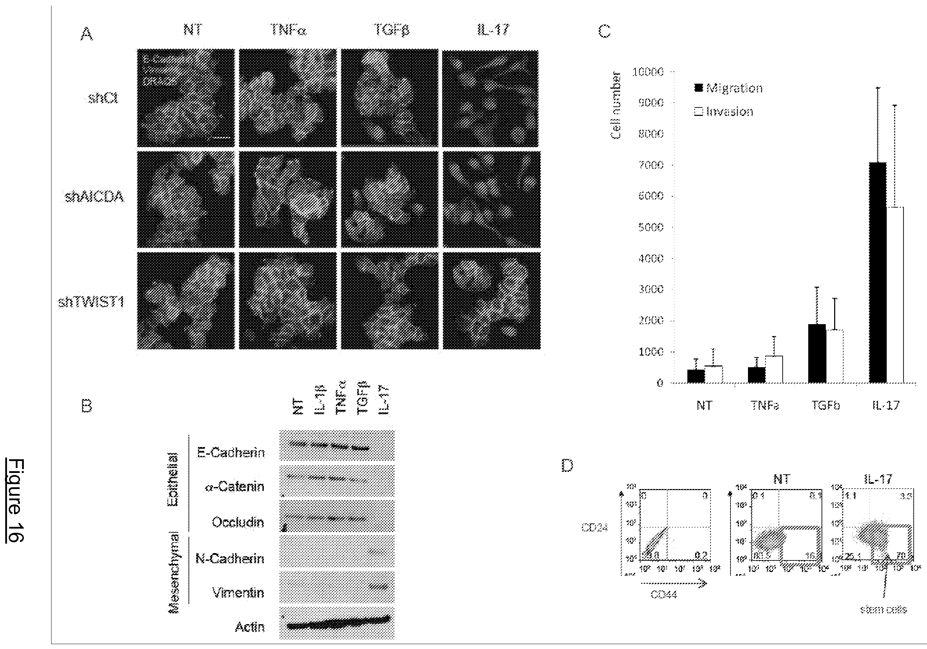

Figure 16: IL-17 induces Epithelial to Mesenchymal Transition (EMT) via Twist-1 upregulation in mammary epithelial cells which increases cell migration, invasion and stemness. Human immortalized mammary epithelial cells (MCF10A) cells were untreated (NT) or treated with TNFα (100 ng/mL), TGFβ (8 ng/mL) or IL-17 (10 ng/mL) for 9 days. (A) MCF10A cells were first transduced using shRNA lentiviral constructs targeting the *LUCIFERASE* gene (shCt), *AICDA* gene (shAICDA) or *TWIST1* gene (shTWIST1) mRNA and then treated with cytokines as indicated above. IL-17 (but no TNFα or TGFβ) induces EMT in ShCt and ShAICDA MCF10A characterized by loss of epithelial marker E-Cadherin (which, in color, fluoresces in green) and gain of mesenchymal marker Vimentin (which, in color, fluoresces in red) as assessed by immunofluorescence. However, IL-17 did not induce EMT in shTWIST1 MCF10A, demonstrating that Twist-1 is required for IL-17 induced EMT. (B) IL-17 (but no TNFα or TGFβ) induces EMT characterized by loss of epithelial markers E-Cadherin, α-Catenin and Occludin and gain of mesenchymal markers N-Cadherin and Vimentin as assessed by immunobloting. Actin was used as a loading control. (C) IL-17 enhances migratory (black bars) and invasive (white bars) abilities of MCF10A cells as assessed in Boyden chamber assays. (D) IL-17 increases the number of CD24^{low} CD44^{high} stem cells in MCF10A cells. Flow cytometry assay showing CD24 and CD44 expression in untreated (NT) and IL-17-treated MCF10A cells (IL-17). Decreased CD24 and increased CD44 expression is indicative of normal stem cells. Red box indicates the stem cell population.

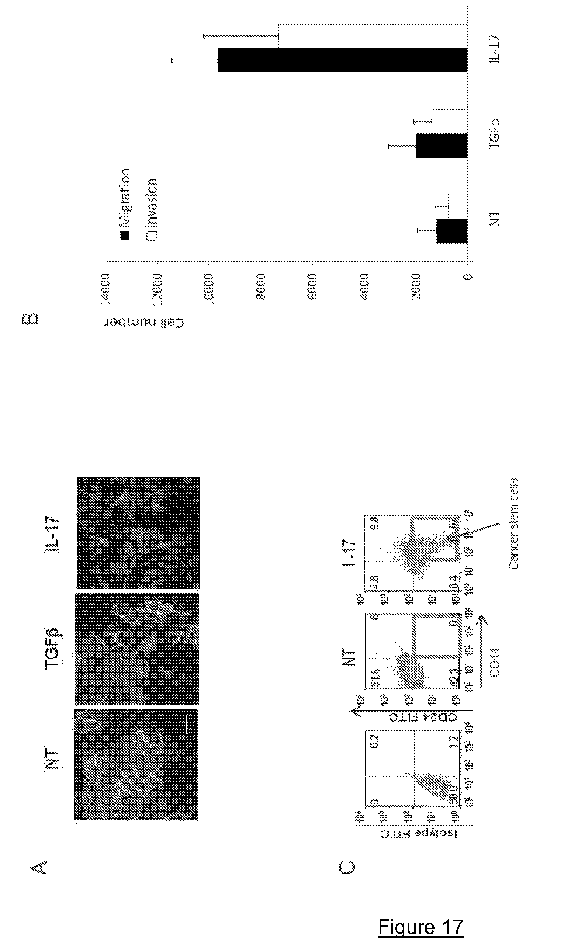

Figure 17: IL-17 induces Epithelial to Mesenchymal Transition (EMT) in well differentiated and non-metastatic MCF7 breast cancer cells which increases MCF7 cancer cell migration, invasion and stemness. MCF7 cells were untreated (NT) or treated TGFβ (8 ng/mL) or IL-17 (10 ng/mL) for 9 days. (A) IL-17 (but not TGFβ) induces EMT characterized by loss of epithelial marker E-Cadherin (which, in color, fluoresces in green) and gain of mesenchymal marker Vimentin (which, in color, fluoresces in red) as assessed by immunofluorescence. (B) IL-17 enhances migratory (black bars) and invasive (white bars) abilities of MCF7 cancer cells as assessed in Boyden chamber assays. (C) IL-17 increases the number of CD24^{low} CD44^{high} stem cells in MCF7 cancer cells. Flow cytometry assay showing CD24 and CD44 expression in untreated (NT) and IL-17-treated MCF7 cells (IL-17). Decreased CD24 and increased CD44 expression is indicative of cancer stem cells. Red box indicates the cancer stem cell population.

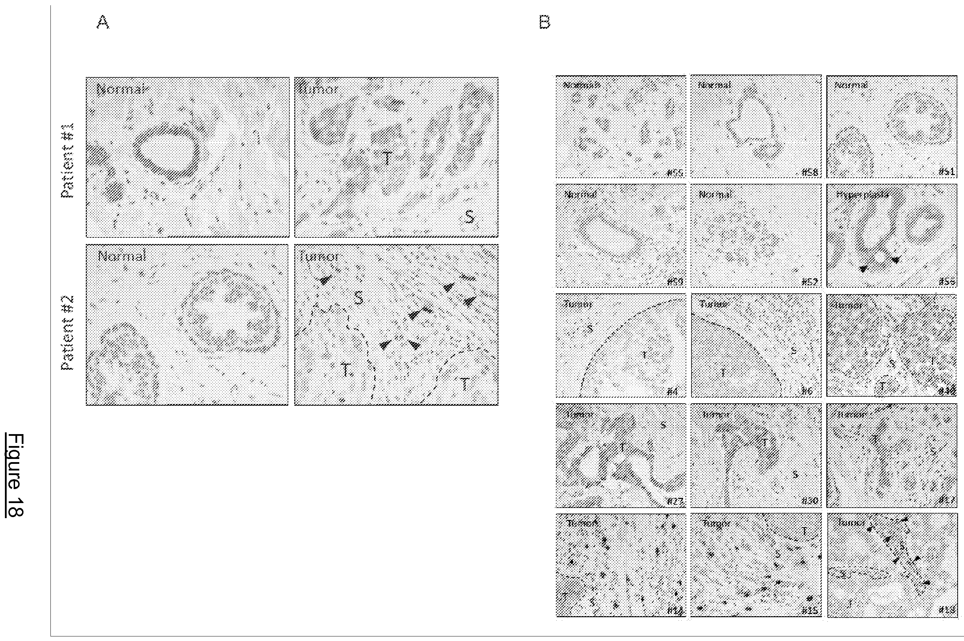

Figure 18: IL-17 expression is increased in breast cancers. (A) Representative immunohistochemistry staining of IL-17 stained sections of two IL-17 positive invasive ductal breast carcinomas. IL-17 is expressed by breast cancer cells but not normal mammary epithelial cells (patient #1, tumor cells are positive) or by immune cells infiltrating the tumor tissue (i.e., in the tumor stroma) but not the corresponding normal breast tissue (patient #2, tumor stroma is positive, arrows). (B) Representative micrographs of IL-17 stained sections of 5 negative normal breast tissue (#55, 58, 51, 59, 52), 1 positive breast tissue hyperplasia (#56), 2 negative invasive breast ductal carcinomas (#4, 6), 4 invasive breast ductal carcinomas with positive tumor cells (#40, 27, 30, 17), 3 invasive breast ductal carcinomas with positive stroma (#14, 15, 18). T=tumor; S=stroma.

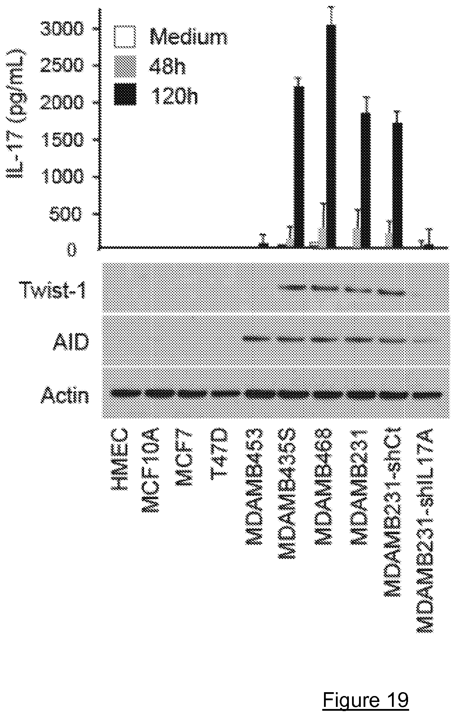

Figure 19: Autocrine production of IL-17 by human breast cancer cell lines parallels their tumorigenic and metastatic potential. (A) Graph showing IL-17 secretion by normal human breast cells (HMEC) or cell line (MCF10A) and various human breast cancer cell lines. IL-17 concentration was measured in the supernatant of cells cultured for 48 h and 120 h as indicated. In the MDAMB231 cell line, IL-17 inhibition by shRNA (shIL17A) decreases AID and Twist-1 expression, demonstrating that autocrine secretion of IL-17 controls AID and Twist-1 expression in these cells.

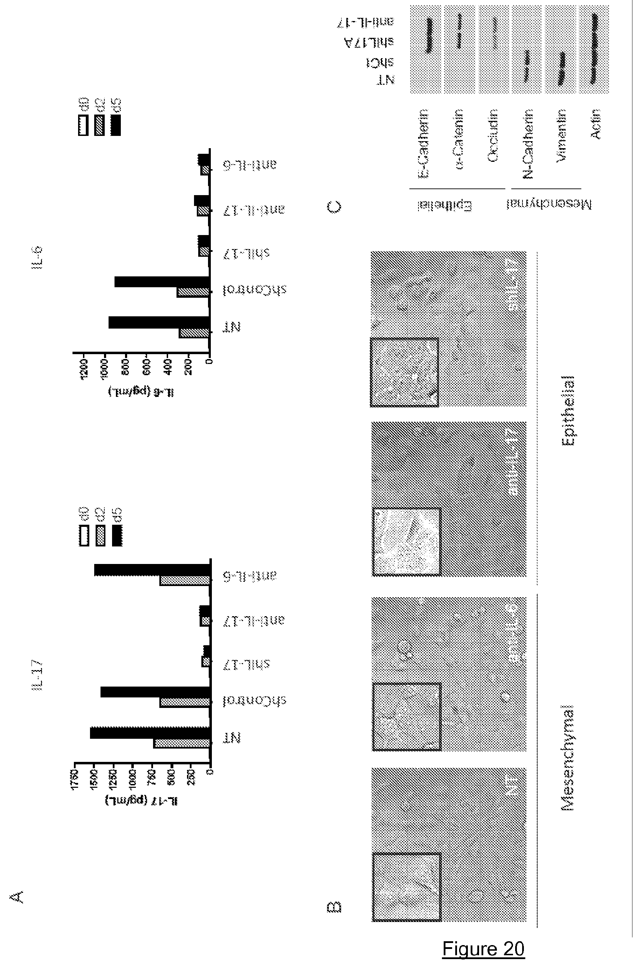

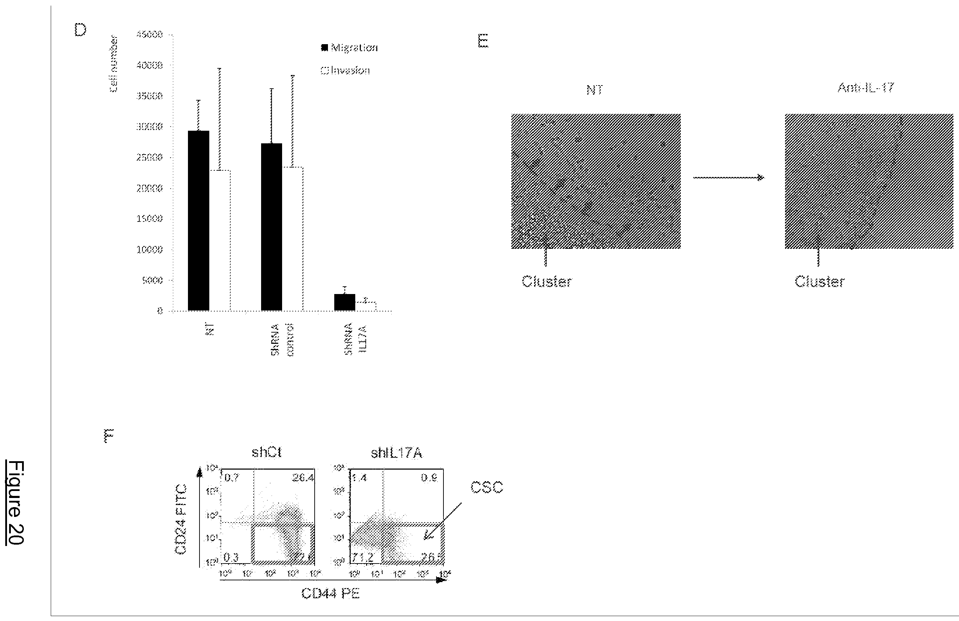

Figure 20: Autocrine production of IL-17 by metastatic MDAMB231 human breast cancer cells is essential to maintain their mesenchymal, migratory, invasive and stem cell phenotypes. (A) MDAMB231 non-treated (NT) or transduced with shRNA lentiviral constructs targeting the *LUCIFERASE* gene (shControl) or *IL17A* (shIL-17) cells were seeded at 3x10⁵ cell/mL in medium alone or in the presence of IL-17- (1 µg/mL) or IL-6- (1 µg/mL) neutralizing antibodies as indicated and maintained in culture for 5 days. IL-17 (left panel) and IL-6 (right panel) concentrations in cell culture supernatants were determined by ELISA at days 0 (d0), 2 (d2) and 5 (d5) as indicated. IL-6 secretion is under the control of IL-17 autocrine secretion. (B) Morphology of MDAMB231 cells untreated (NT), treated with IL-6- (1 µg/mL) or IL-17- (1 µg/mL) neutralizing antibodies or transduced with a lentiviral construct targeting the *IL17A* gene (shIL-17). The cell phenotype (epithelial or mesenchymal) according to the cell morphology is indicated below. IL-17 inhibition by shRNA (shIL17) or neutralization by neutralizing antibody (anti-IL-17, 1 µg/mL) induces a mesenchymal to epithelial transition (reversion of EMT) of MDAMB231 cells as shown by cell morphology (switch from spindle like to cobblestone-like morphology). (C) IL-17 inhibition by shRNA (shIL17A) or neutralization by neutralizing antibody (anti-IL-17, 1 µg/mL) induces a mesenchymal to epithelial transition (reversion of EMT) of MDAMB231 breast cancer cells as shown by upregualtion of epithelial markers and downregualtion of mesenchymal markers. MDAMB231 cells were either untreated (NT), transduced with a lentiviral construct targeting the *LUCIFERASE* gene (shCt) or the *IL17A* gene (shIL17A) or treated with anti IL-17 neutralizing antibody (1 µg/mL) for > 9 days. Then E-cadherin, α-Catenin, Occludin (epithelial markers), N-Cadherin, Vimentin (mesenchymal markers) and actin protein expression was assessed by immunoblotting. Actin was used as a loading control. (D) IL-17 inhibition reduces MDAMB231 breast cancer cell migration and invasion. MDAMB231 cells were untreated (NT), transduced with a lentiviral construct targeting the *LUCIFERASE* gene (shRNA Control) or the *IL17A* gene (shIL17A). MDAMB231 cell migration (black bars) and invasion (white bars) was assessed in Boyden chamber assays. (E) IL-17 neutralizing antibody decreases MDAMB231 breast cancer cell invasive properties. MDAMB231 cells were either untreated (left) or treated for 6 days with IL-17 neutralizing antibody (1 µg/mL) (right), and then tested for their invasive ability in cluster assay. (F) IL-17 inhibition decreases the number of CD24^{low} CD44^{high} cancer stem cells in MDAMB231 breast cancer cells. MDAMB231 cells were either untreated (NT), transduced with a lentiviral construct targeting the *LUCIFERASE* gene (shRNA Control) or the *IL17A* gene (shIL17A). Flow cytometry analysis of CD24 and CD44 expression after inhibition of IL-17 expression with shRNA shows a decrease in the number of cancer stem cells compared to cells infected with control shRNA. Red box indicates the cancer stem cell population.

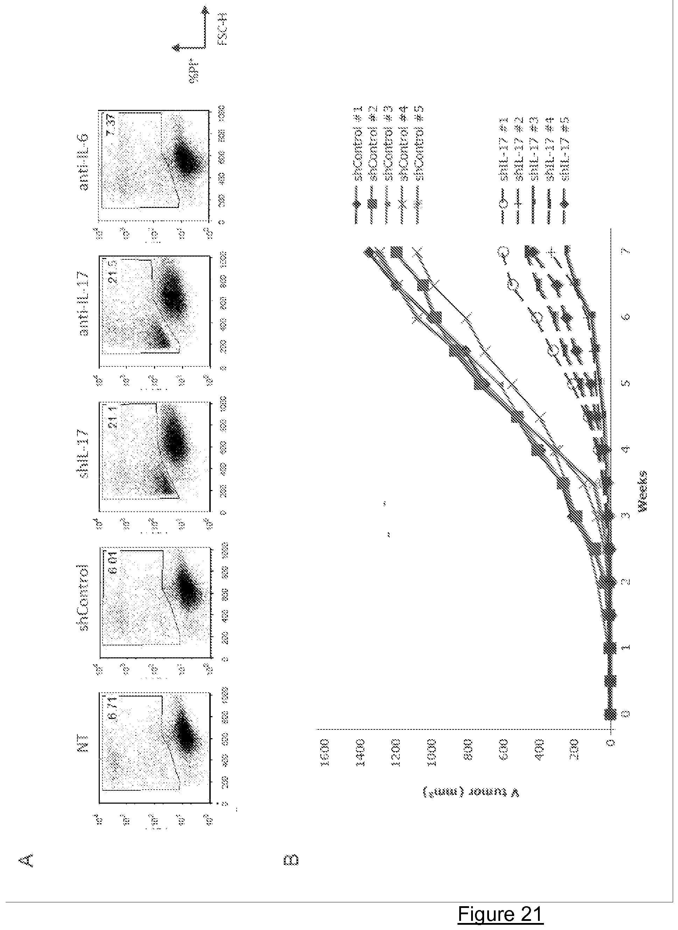

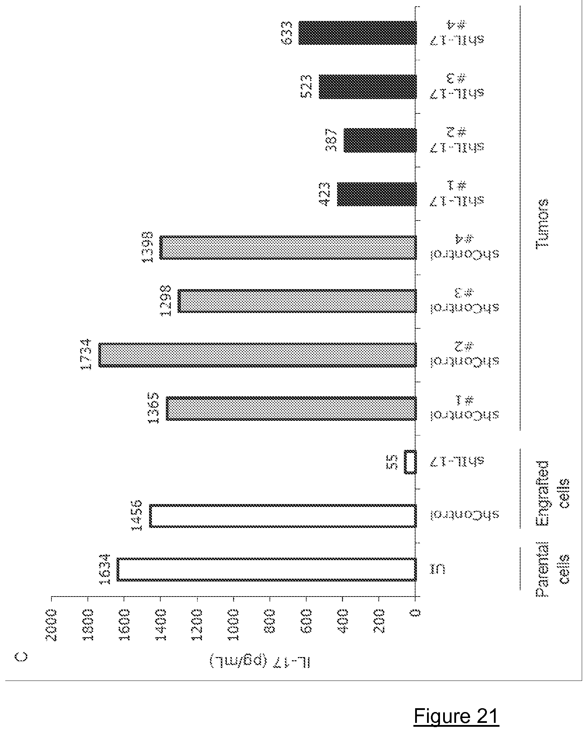

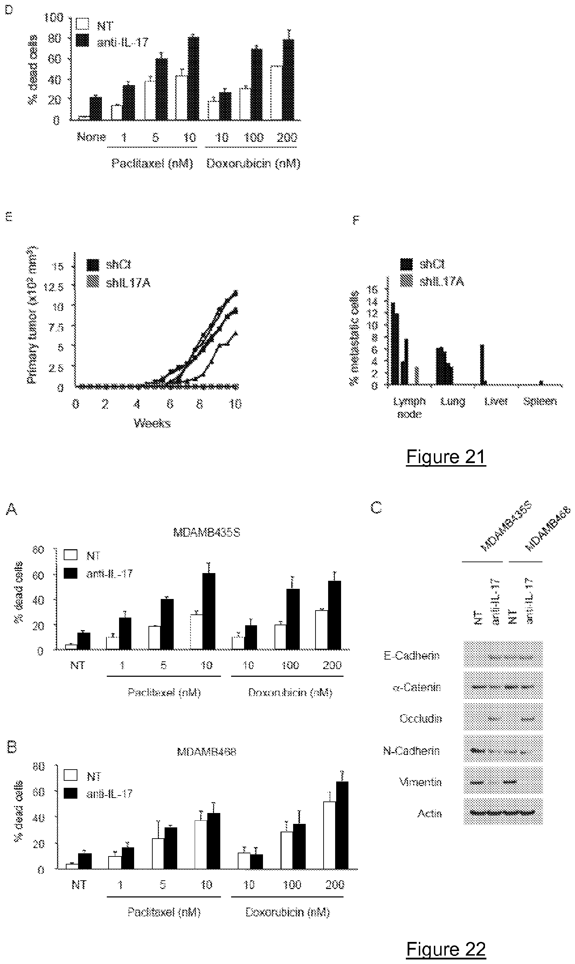

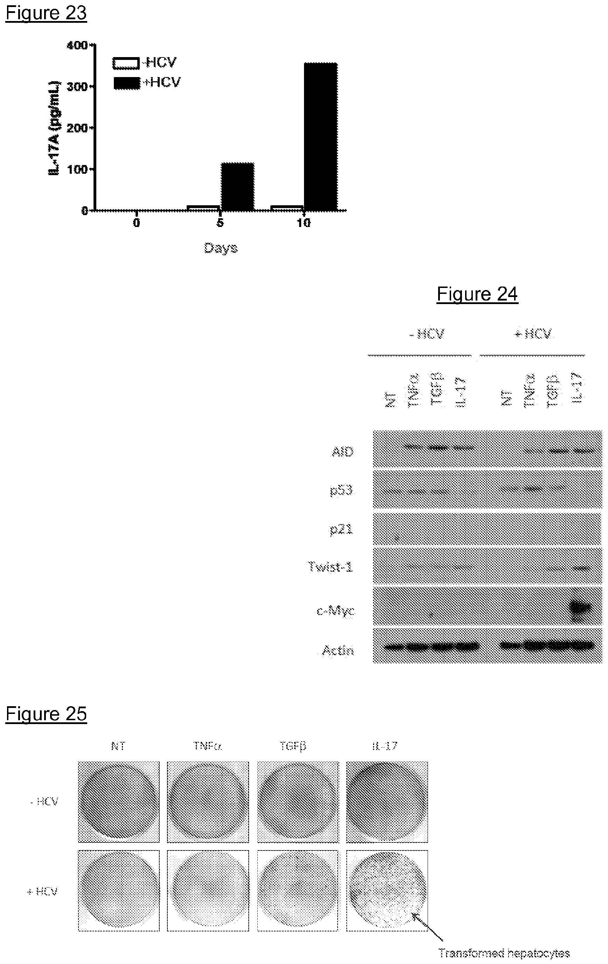

Figure 21: IL-17 inhibition and/or neutralization by neutralizing antibody induces tumor cell death and increases sensitivity to chemotherapy *in vitro* and abrogates tumor growth and metastasis *in vivo* of metastatic MDAMB231 human breast cancer cells. (A) IL-17 inhibition by shRNA or neutralization by neutralizing antibody induces tumor cell death of MDAMB231 cells. MDAMB231 cells were non-treated (NT) or transduced using shRNA lentiviral constructs targeting the *LUCIFERASE* gene (ShControl) or *IL17A* gene (shIL-17) or treated with IL-17- (1 µg/mL) or IL-6- (1 µg/mL) neutralizing antibodies and maintained in culture for 96 h. Cell viability was assessed at 96 h by propridium iodide uptake and flow cytometry analysis. (B) IL-17 inhibition dramatically reduces subcutaneous growth of MDAMB231 cells in nude mice. MDAMB231 cells were transduced with shRNA lentiviral constructs targeting the *LUCIFERASE* gene (shControl#1-5) or *IL17A* (shIL-17#1-5) and were then subcutaneously grafted (5.10⁶ cells) in the flank of irradiated athymic Swiss nude mice (5 mice per group) and tumor growth was monitored twice a week with calipers at the site of injection. (C) IL-17 concentration was measured in the supernatant of 5 day cultures of uninfected MDAMB231 cells (UI), of the MDAMB231 cell lines expressing IL-17 (shIL-17) or control (shControl) shRNA at the time of engraftment in mice and of the tumor cells obtained from surgical resection and dissociation of some primary tumors described in Figure 23B. Of note, tumors generated by sh-IL-17 MDAMB231 cells express much higher levels of IL-17 (ranging from 387 to 633 pg/ml) than the cells of origin (∼55 pg/ml). (D) IL-17 neutralization by neutralizing antibody induces MDAMB231 cell death and sensitizes MDAMB231 cells to chemotherapy. MDAMB231 were non-treated (NT) or treated in the presence of IL-17- (1 µg/mL) neutralizing antibody and paclitaxel or doxorubicin at indicated concentrations and maintained in culture for 96 h. Cell viability was assessed at 96 h by propridium iodide uptake and flow cytometry analysis. (E) IL-17 inhibition abrogates the growth of orthotopic xenografts of MDAMB231 cells in nude mice. 10⁵ MDAMB231 transduced either with shRNA lentiviral constructs targeting the *LUCIFERASE* gene (shCt) or *IL17A* gene (shIL17A) were grafted in the mammary fat pad of irradiated athymic Swiss nude mice (5 mice per group) and tumor growth was monitored twice a week with calipers at the site of injection. No tumors were detected in mice engrafted with MDAMB231 cells where IL-17 had been inhibited (shIL17A). (F) IL-17 inhibition abrogates metastatic dissemination to the lymph node, liver and lungs of orthotopic xenografts of MDAMB231 cells in nude mice. 10⁵ MDAMB231 transduced either with shRNA lentiviral constructs targeting the *LUCIFERASE* gene (shCt) or *IL17A* (shIL-17) were grafted in the mammary fat pad of irradiated athymic Swiss nude mice (5 mice per group). Metastases were detected by flow cytometry (detection of human cells expressing human IL-17R in the draining lymph node, liver, and the lungs of mice engrafted with MDAMB231 infected with control shRNA, whereas no metastases were observed with MDAMB231 infected with IL-17 targeting shRNA (with exception of one positive lymph node in one out of five mice).
Figure 22: IL-17 neutralization by neutralizing antibody induces tumor cell death, sensitization to chemotherapy and reversion to an epithelial phenotype in MDAMB435S and MDAMB468 human metastatic breast cancer cell lines. (A, B) IL-17 neutralization by neutralizing antibody induces MDAMB435S and MDAMB468 tumor cell death and sensitizes cells to chemotherapy. MDAMB435S (A) and MDAMB468 (B) were non-treated (NT) or cultured in the presence of IL-17- (anti-IL-17, 1 µg/mL) neutralizing antibody and paclitaxel or doxorubicin at indicated concentrations and maintained in culture for 96 h. Cell viability was assessed at 96 h by propridium iodide uptake and flow cytometry analysis. (C) IL-17 neutralization by neutralizing antibody restores an epithelial phenotype in metastatic MDAMB435S and MDAMB468 human breast cancer cell lines. MDAMB435S and MDAMB468 cells were either untreated (NT) or treated with anti IL-17 neutralizing antibody (1 µg/mL) for > 9 days. Then E-Cadherin, α-Catenin, Occludin (epithelial markers), N-Cadherin, Vimentin (mesenchymal markers) and actin protein expression was assessed by immunoblotting. Actin was used as a loading control. IL-17 neutralization by neutralizing antibody (anti-IL-17) restores an epithelial phenotype in MDAMB435S and MDAMB468 cells as shown by upregualtion of epithelial markers (E-Cadherin and/or α-Catenin and/or Occludin) and downregualtion of mesenchymal markers (N-Cadherin and/or Vimentin).
Figure 23: Autocrine production of IL-17 by hepatitis C virus (HCV)-infected human primary hepatocytes. Human primary hepatocytes either uninfected (-HCV) or infected with HCV viral particles (0.1 MOI, +HCV) were seeded at 3x10⁵ cell/mL and maintained in culture for 10 days. IL-17 concentrations in cell culture supernatants were determined by ELISA at days 5 and 10 as indicated.
Figure 24: IL-17 and HCV upregualte AID, Twist-1 (an inhibitor of p53) and c-Myc in human primary hepatocytes. Human primary hepatocytes infected with HCV (+HCV) or not (-HCV) were either non-treated (NT) or stimulated for 5 days with TNFα (100 ng/mL), TGFβ (8 ng/mL) or IL-17 (10 ng/mL). Then AID, p53, p21, Twist-1, c-Myc and actin protein expression was assessed by immunoblotting. Actin was used as a loading control. IL-17 and HCV induce AID and Twist-1 expression, resulting in the inhibition of p53 pathway. This is accompanied by the upregulation of oncogenes, e.g. c-Myc.
Figure 25: IL-17 cooperates with HCV for transformation of human primary hepatocytes. Human primary hepatocytes were infected with HCV (+HCV) or not (-HCV) and either untreated or exposed to TNFα (100 ng/mL), TGFβ (8 ng/mL) or IL-17 (10 ng/mL) for 21 days. Then, the transformed phenotype of the cells was assessed in foci formation assay. Cells exposed to IL-17 and HCV display a transformed phenotype.

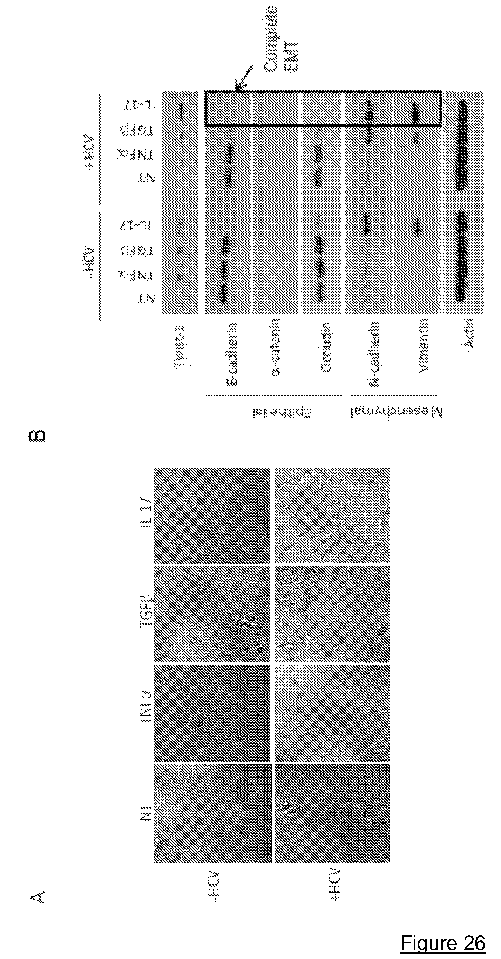

Figure 26: IL-17 induces Epithelial to Mesenchymal Transition (EMT) in human primary hepatocytes. Human primary hepatocytes were infected (+HCV) or not (-HCV) with HCV and either untreated or exposed to TNFα (100 ng/ml), TGFβ (8 ng/ml) or IL-17 (10 ng/ml) for 9 days. (A) IL-17 induces EMT characterized by a switch from cobblestone-like to spindle-like morphology. In the presence of HCV, some mesenchymal cells are seen in TGFβ-treated hepatocytes, suggesting partial EMT. (B) IL-17 (but no TNFα or TGFβ) induces EMT characterized by downregulation of epithelial markers (E-Cadherin, α-Catenin and Occludin) and gain of mesenchymal markers (N-Cadherin and Vimentin) as assessed by immunoblotting. HCV infection (+HCV) further increased IL-17 induced EMT. Twist-1 and actin protein expressions were also assessed by immunobloting. Actin was used as a loading control. Again, partial decrease in epithelial markers and partial increase in mesenchymal ones suggest partial EMT in TGFβ-treated hepatocytes that were infected with HCV.

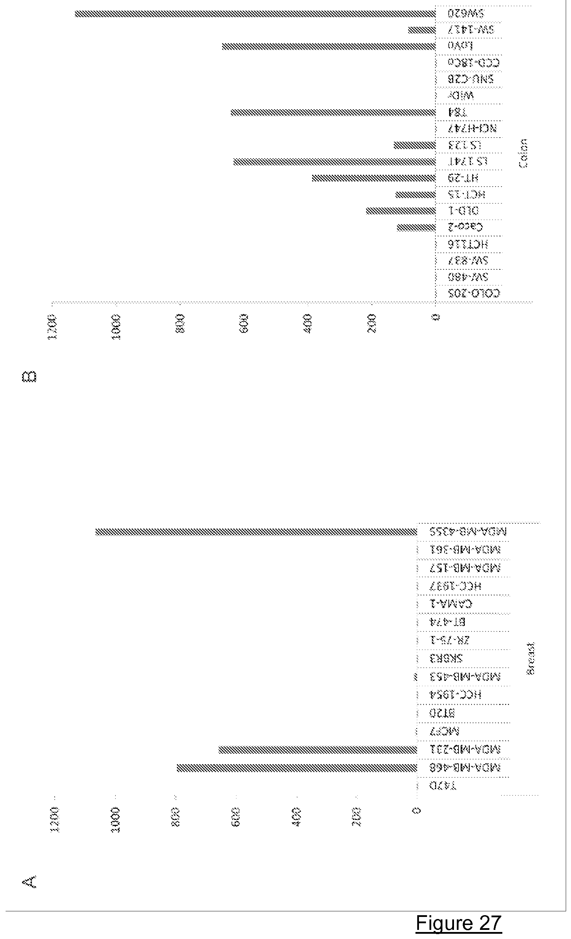

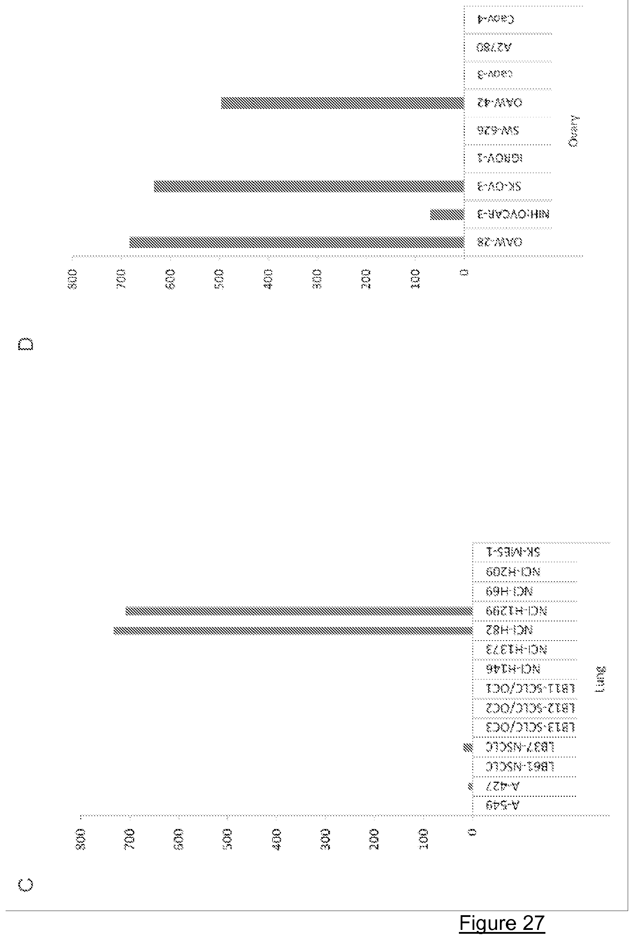

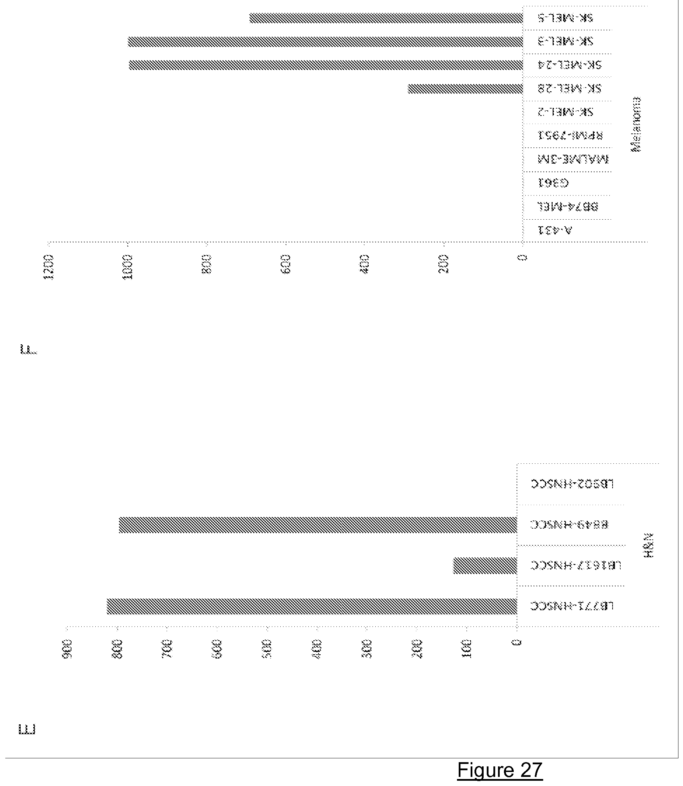

Figure 27: Autocrine production of IL-17 by various human cancer cell lines. Various human cancer cell lines were cultured *in vitro* and the concentration of IL-17 in the supernatant of 5 day cultures was measured by ELISA. Autocrine production of IL-17 is observed in some breast cancer cell lines (A), colon cancer cell lines (B), lung cancer cell lines (C), ovarian cancer cell lines (D), head and neck/esophageal cancer cell lines (E) and melanoma cell lines (F).

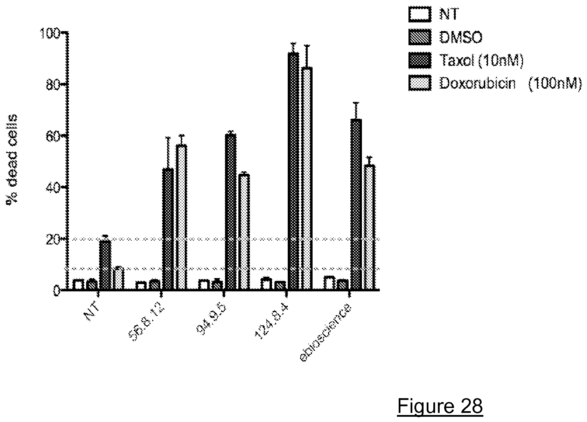

Figure 28: IL-17 neutralizing antibodies OREGA-56-8-12 (hybridoma deposited as CNCM-I-4476), OREGA-94-9-5 (hybridoma deposited as CNCM-I-4477) and OREGA-124-8-4 (hybridoma deposited as CNCM-I-4478) sensitize MDAMB231 breast cancer cells to chemotherapy. IL-17 antagonization by neutralizing antibody OREGA-56-8-12, OREGA-94-9-5 and OREGA-124-8-4 sensitizes MDAMB231 cell to chemotherapy. MDAMB231 cells were non-treated (NT) or treated in the presence of IL-17 neutralizing antibody (1 µg/mL) or treated in the presence of reference IL-17 neutralizing antibody (Ebioscience, 1 µg/mL) and treated or not with 10 nM paclitaxel (taxol) or 100 nM doxorubicin and maintained in culture for 72 h. Cell viability was assessed at 72 h by propridium iodide uptake and flow cytometry analysis.

### DETAILED DESCRIPTION OF THE INVENTION

### Definitions

Unless otherwise expressly defined, the terms used herein are to be understood according to their ordinary meaning in the art. Terms used in the singular or referred to as "a" or "an" also include the plural and vice versa, unless otherwise specified or indicated by context. Standard techniques and procedures are generally performed according to conventional methods in the art and various general references (*see* generally, Sambrook et al. Molecular Cloning: A Laboratory Manual, 2nd ed. (1989) Cold Spring Harbor Laboratory Press, Cold Spring Harbor, N.Y.), which are provided throughout this document

As used herein, the term "IL-17 antagonist" refers to an agent, substance, molecule, *etc.,* that disrupts, prevents, inhibits, or otherwise targets and/or interferes with the activity of IL-17 or the IL-17 pathway. Non-limiting examples of IL-17 antagonists include antibodies against IL-17 or its receptor (including, for example, neutralizing antibodies), small molecule antagonists, decoy receptors, protein antagonists (including fusion proteins and glycoproteins), and nucleic acid antagonists (e.g., gene silencers, short hairpin RNA, or "shRNA", siRNA, and antisense nucleic acid molecules).

As used herein, the term "BAFF antagonist" refers to an agent that disrupts, prevents, inhibits, or otherwise interferes with the activity of B-cell activating factor ("BAFF"). BAFF is also known as B-lymphocyte stimulator (BLys), TNFSF13B, TALL-1, zTNF4, and THANK. Non-limiting examples of BAFF antagonists include antibodies against BAFF or its receptor (including, for example, neutralizing antibodies), small molecule antagonists, decoy receptors, protein antagonists (including fusion proteins and glycoproteins), and nucleic acid antagonists (e.g., gene silencers, shRNA, siRNA, and antisense nucleic acid molecules).

As used herein, the term "IL-6 antagonist" refers to an agent, substance, molecule, *etc.,* that disrupts, prevents, inhibits, or otherwise targets and/or interferes with the activity of IL-6 or the IL-6 pathway. Non-limiting examples of IL-6 antagonists include antibodies against IL-6 or its receptor (including, for example, neutralizing antibodies), small molecule antagonists, decoy receptors, protein antagonists (including fusion proteins and glycoproteins), and nucleic acid antagonists (e.g., gene silencers, short hairpin RNA, or "shRNA", siRNA, and antisense nucleic acid molecules).

As used herein, the term "IL-10 antagonist" refers to an agent, substance, molecule, *etc.,* that disrupts, prevents, inhibits, or otherwise targets and/or interferes with the activity of IL-10 or the IL-10 pathway. Non-limiting examples of IL-10 antagonists include antibodies against IL-10 or its receptor (including, for example, neutralizing antibodies), small molecule antagonists, decoy receptors, protein antagonists (including fusion proteins and glycoproteins), and nucleic acid antagonists (*e.g.,* gene silencers, short hairpin RNA, or "shRNA", siRNA, and antisense nucleic acid molecules).

As used herein, the term "antibody" includes whole or "full" antibodies and any antigen binding fragment or single chains thereof. An "antibody" refers to a glycoprotein comprising at least two heavy (H) chains and two light (L) chains inter-connected by disulfide bonds, or an antigen binding portion thereof. A full heavy chain contains a heavy chain variable region (V_{H}) and a heavy chain constant region. A full heavy chain constant region has three domains, C_{H}1, C_{H}2 and C_{H}3. A full light chain contains a light chain variable region (V_{L}) and a light chain constant region that contains one domain, C_{L}. The V_{H} and V_{L} regions can be further subdivided into regions of hypervariability, termed complementarity determining regions (CDR), situated within the more conserved framework regions (FR). Each full V_{H} and V_{L} contains three CDRs and four FRs, arranged from amino-terminus to carboxy-terminus in the following order: FR1, CDR1, FR2, CDR2, FR3, CDR3, FR4. The V_{H} and V_{L} form a binding domain that interacts with an antigen. The constant regions of the antibodies may mediate the binding of the immunoglobulin to host tissues or factors, including various cells of the immune system (e.g., effector cells) and the first component (Clq) of the classical complement system.

As used herein, the term "antigen-binding fragment" refers to one or more fragments of an antibody that retain the ability to specifically bind to an antigen (e.g., IL-17 or BAFF). The antigen-binding function of an antibody can be performed by fragments of a full antibody. Examples of "antigen-binding fragments" include (i) an Fab fragment, a monovalent fragment consisting of the V_{L}, V_{H}, C_{L} and C_{H}1 domains; (ii) an F(ab)₂ fragment, a bivalent fragment comprising two Fab fragments linked by a disulfide bridge at the hinge region; (iii) an Fab' fragment, an Fab with part of the hinge region (*see,* FUNDAMENTAL IMMUNOLOGY, Paul ed., 3rd ed. 1993); (iv) an Fd fragment, consisting of the V_{H} and C_{H}1 domains; (v) an Fv fragment consisting of the V_{L} and V_{H} domains of a single arm of an antibody, (vi) a dAb fragment (Ward et al., (1989) Nature 341:544-546), which consists of a V_{H} domain; (vii) an isolated complementarity determining region (CDR); and (viii) a nanobody, a heavy chain variable region containing a single variable domain and two constant domains. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); *see e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

As used herein, the term "monoclonal antibody" refers to a preparation of antibody molecules of single molecular composition. A monoclonal antibody composition displays a single binding specificity and affinity for a particular epitope of an antigen (e.g., IL-17).

As used herein, the term "human antibody" is intended to include antibodies having variable regions in which both the framework and CDR regions are derived from human germline immunoglobulin sequences. If the antibody contains a constant region, the constant region also is derived from human germline immunoglobulin sequences. Human antibodies for use in the invention may include amino acid residues not encoded by human germline immunoglobulin sequences (e.g., mutations introduced by random or site-specific mutagenesis *in vitro* or by somatic mutation *in vivo*)*.*

As used herein, the term "humanized antibody" refers to antibodies in which CDR sequences derived from the germline of another mammalian species, such as a mouse, rat, or rabbit, have been grafted onto human framework sequences. Additional framework region modifications may be made within the human framework sequences.

As used herein, the term "chimeric antibody" refers to antibodies in which the variable region sequences are derived from one species and the constant region sequences are derived from another species, such as an antibody in which the variable region sequences are derived from a mouse, rat, or rabbit antibody and the constant region sequences are derived from a human antibody.

As used herein, the term "small molecule" includes, but is not limited to organic or inorganic compounds (including heterorganic and organometallic compounds) having a molecular weight of less than 1000 grams per mole, more specifically less than 750 grams per mole, and even more specifically, 500 grams per mole. Salts, esters, and other pharmaceutically acceptable forms of such compounds are also encompassed.

As used herein, the term "nucleic acid antagonist" is intended to include any gene silencing molecule or tool such as shRNA ("short hairpin RNA" or "small hairpin RNA") or interfering RNA ("RNAi" or small interfering RNA, "siRNA").

As used herein, the term "subject" or "patient" refers to a mammalian animal (including but not limited to non-primates such as cows, pigs, horses, sheep, cows, dogs, cats, rats, and mice), more specifically a primate (including but not limited to monkeys, apes, and humans), and even more specifically, a human.

As used herein, the term "reference amount" or "reference level" refers to an established amount or expression level of a substance or molecule, e.g., a protein, nucleic acid, enzyme, etc., to which an amount of the same substance or molecule measured from a subject sample is being compared. For example, a reference amount may be an amount of a protein found in a normal tissue sample (or an average amount from a population of tissue samples) to which the amount of a protein from a diseased (e.g., cancerous) tissue sample is compared.

As used herein, the term "expression" or "expressed" refers to the transcription of RNA (e.g., mRNA) from a nucleic acid template. "Expression" or "expressed" may also refer to translation of mRNA into a polypeptide. The term "expression" or "expressed" is also intended to include the production of a gene product or polypeptide that is released and/or secreted by a cell. In some instances, the term "produced" or "production" is used to indicate the expression of a gene product or polypeptide that is secreted or released by a cell (e.g., into the extracellular environment or, if the cell is in vitro, into the culture medium). The term "increased expression" is intended to include an alteration in gene expression at least at the level of increased mRNA production and/or at the level of polypeptide expression, generally resulting in an increased amount of a gene product or protein. In some instances, "increased expression" is used interchangeably with the term "overexpression" or "overexpressed."

As used herein, the term "amount effective" or "effective amount" (e.g., to treat, to prevent, to reduce, etc.) refers to an amount of a therapeutic agent, e.g., an IL-17 antagonist and/or a BAFF antagonist, that is sufficient to achieve the desired effect, such as, to alleviate one or more disease symptoms or effects in the treated subject or population, whether by inducing the regression of or inhibiting the progression of such symptom(s) or effects by any clinically measurable degree. The amount of a therapeutic agent that is effective to alleviate any particular disease symptom or effect (also referred to as the "therapeutically effective amount") or prevent an particular disease symptom or effect (also referred to as the "prophylactically effective amount") may vary according to factors such as the disease state, age, and weight of the patient, and the ability of the drug to elicit a desired response in the patient. Whether a disease symptom or effect has been alleviated can be assessed by any clinical measurement typically used (e.g., by healthcare providers or laboratory clinicians) to assess the severity or progression status of that symptom or effect.

As used herein, the term "likely to respond" means that a subject is more likely than not to receive a therapeutic benefit from the therapeutic agent, e.g., that the therapeutic agent is more likely than not to achieve the desired effect, such as, to alleviate one or more disease symptoms or effects. In one specific example, a subject who has a cell proliferation disorder in which levels of IL-17, AID, and/or TWIST-1 are increased compared to a reference amount of each factor is more likely to respond to treatment of the cell proliferation disorder with an IL-17 antagonist.

As used herein, the term "treat" or "treatment" refers to contact or administration of an exogenous pharmaceutical, therapeutic agent, diagnostic agent, or composition to the animal, human, subject, cell, tissue, organ, or biological fluid, and can refer, e.g., to therapeutic, pharmacokinetic, diagnostic, research, and experimental methods. Treatment of a cell encompasses contact of a reagent to the cell, as well as contact of a reagent to a fluid, where the fluid is in contact with the cell. Treatment also encompasses *in vitro* and *ex vivo* treatments, e.g. of a cell, by a reagent, diagnostic, binding compound, or by another cell. "Treatment," as it applies to a human, veterinary, or research subject, refers to therapeutic treatment, prophylactic or preventative measures, to research and diagnostic applications. "Treatment" as it applies to a human, veterinary, or research subject, or cell, tissue, or organ, encompasses contact of an IL-17 antagonist to a human or animal subject, a cell, tissue, physiological compartment, or physiological fluid.

As used herein, the term "prevent" or "prevention" or "prophylactic" or "prophylaxis" refers to the inhibition of the development, onset, or recurrence of a symptom, effect, disease, or disorder (e.g., a cell proliferation disorder).

As used herein, the term "sample" refers any biological fluid, cell, tissue, or other component from a subject. Samples include, without limitation, tissue fragments (diseased or non-diseased), organs, cells, cellular components, whole blood, serum, plasma, saliva, urine, synovial fluid, bone marrow, cerebrospinal fluid, vaginal mucus, cervical mucus, nasal secretions, sputum, semen, amniotic fluid, bronchoalveolar lavage fluid, cellular exudates, and tumor fragments.

As used herein, the term "targeting a cell" refers to administering a compound, e.g., an antagonist such as an IL-17 antagonist, to change the properties of the cell. Non-limiting examples of cell properties that may be affected by targeting of a cell include modifying the phenotype, altering the stem cell properties (e.g., of a cancer cell), and altering the proliferative ability of the cell (e.g., decreasing the ability of the cell to proliferate).

As used herein, the term B-cell is used interchangeably with the term "B lymphocyte".

### Overview

The present inventors have surprisingly found that IL-17 has a direct role in the onset and progression of cell proliferation disorders, such as cancer. More specifically, exposure of cells to IL-17 upregulates expression of AID and inhibits the p53 tumor suppressor pathway (e.g., by upregulating expression of TWIST-1).

In particular, the present inventors have surprisingly found that IL-17 can induce genomic instability and DNA damage in a mammalian cell through increased expression of activation-induced cytidine deaminase (AID). AID is a mutator enzyme that plays a key role in somatic hypermutation and class switch recombination, events which generate the diversity of the antibody repertoire from the set of immunoglobulin-encoding genes in B-cells. *See, e.g.,* Bransteitter et al., Proc. Natl. Acad. Sci. 100: 4102-4107 (2003); Smit et al., Cancer Res. 63: 3894-3898 (2003). However, AID can induce mutations outside the immunoglobulin (Ig) loci if mechanisms involved in the surveillance of DNA damage, such as the p53 guardian pathway, are turned off, *See, e.g.,* Ramiro A.R. et al., Nature, 440:105-9 (2006). Furthermore, whereas normal AID expression is limited to B cells, it can be aberrantly induced in mammary epithelial cells and breast cancer cells, *See, e.g.,* Pauklin S. et al., J Exp Med 206: 99-111 (2009) and in HCC *See, e.g.,* Kou T., et al., Int J Cancer, 120: 469-76 (2007).

As shown herein, in B-cells, IL-17 can act together with BAFF to increase expression of AID. As also shown herein, in other cell types, including breast (mammary) epithelial cells and liver (hepatocyte) epithelial cells, where AID expression is not normally detected, IL-17 can also act to increase AID expression. IL-17-induced expression of AID (e.g., increased expression in B-cells or expression in cells that don't normally express detectable levels of AID) causes damage and/or mutations to DNA, i.e., IL-17-induced DNA damage.

The present inventors have also found that IL-17 has an inhibitory effect on the p53 pathway. This inhibitory effect is mediated by IL-17-induced upregulation of TWIST-1. The p53 molecule is a tumor suppressor that normally functions by regulating the transcription of target genes involved in DNA replication and repair, cell cycle progression (*e.g.,* causing cell cycle arrest), and apoptosis, *See, e.g.,* Onishi et al, Anti-Cancer Agents in Medicinal Chemistry 8: 564-570 (2008). Activation of p53 would normally inhibit accumulation of cells with DNA damage. However, as shown in herein, expression of IL-17 causes an inhibition of p53 expression and function, thereby increasing the risk and/or likelihood that DNA damage (for example, damage caused by IL-17 induced expression of AID), will not be repaired and leading to transformation of cells into cancerous cells.

The present inventors have also found that IL-17 induces an increase in the expression of TWIST-1. TWIST-1 is an embryonic transcription factor that has been associated with the epithelial-to-mesenchymal transition of cancer cells, cancer metastases, and tumor progression. *See, e.g.,* Ansieau et al., Oncogene (2010) 1-12; Thierry et al., Cell 139:871-890 (2009); Yang et al., Cell 117: 927-939 (2004); Lee et al., Clin. Cancer Res. 12:5369-5376 (2006); Yang et al., Hepatology 50:1464-1474 (2009); Matsuo et al., BMC Cancer 9:240 (2009); and Choi and Diehl, Hepatology 50: 2007-2013 (2009). TWIST-1 expression has been detected in numerous cancers, including amelobalstoma, bladder, breast, cervical, choroids plexus, colon, endometrial, gastric, glioma, head and neck, hepatocellular carcinoma (HCC), kidney, melanoma, nasopharyngeal, esophageal squamous cell carcinoma, ovarian, pancreas, parathyroid, pheochromoytoma, prostate, neuroblastoma and sarcoma, and in many cases, is associated with poor prognosis and invasiveness. For a review, *see* Ansieau et al., Oncogene (2010) 1-12.

The present inventors have found, surprisingly, that cancer cells, for example, cancer cells of breast, colon, lung, ovary, head and neck, melanoma and lymphoma, can express IL-17 in an autocrine manner, as well as in the tumor microenvironment. This type of IL-17 expression correlates to the aggressiveness (e.g., cell survival, growth, invasion, and metastases) of e.g., breast tumors. Antagonists against IL-17 can induce cancer cell death and sensitize cells to therapeutic agents; abrogate primary tumor growth, and abrogate metastases. Hence, in one aspect, it is disclosed a method of treating or preventing a cell proliferation disorder associated with increased expression of IL-17 and/or AID by cancer cells or cells at risk for becoming cancerous, said method comprising administering to said cancer cells or cells at risk for becoming cancerous an IL-17 antagonist. In some embodiments, the method results in: targeting and/or killing the cancer cells or the cells at increased risk for becoming cancerous; increasing the effectiveness of a therapeutic agent, e.g. in treating or preventing a cell proliferation disorder; and/or preventing tumor metastasis.

Surprisingly, the present inventors have found that antagonists of IL-17 can prevent or reduce DNA damage caused by exposure to IL-17 or, in B cells, by exposure to a combination of IL-17 and BAFF. The present inventors have also found that antagonists of IL-17 can prevent transformation of a normal cell into a tumor cell. The present inventors have also found that antagonists of IL-17 can prevent or revert the epithelial to mesenchymal transition (EMT) of cells that leads to cancer invasion and metastases, as well as fibrosis in tissues such as liver. The present inventors have also found that antagonists of IL-17, used alone or in combination with chemotherapy, can induce tumor cell death. The present inventors have also found that antagonists of IL-17 can reduce tumor growth and metastases *in vivo.* Accordingly, in certain aspects, are disclosed methods of treating and/or preventing cell proliferation disorders or other IL-17-related disease with antagonists of IL-17 or a combination of IL-17 antagonists and BAFF-antagonists, as well as methods of identifying subjects at risk for such disorders or diseases and methods of identifying subjects who are likely to respond to treatment and/or prevention of the disorders or disease with these antagonists.

The present inventors have also surprisingly found that the diffuse large B cell lymphoma (DLBCL) type of lymphoma has a characteristic cytokine expression profile that distinguishes it from other types of lymphoma, in that DLBCL cells secrete IL-17, BAFF, IL-6, and IL-10. This profile is not seen with other lymphoma types. Combinations of antagonists against IL-17 and BAFF, also in combination with antagonists of IL-6 and/or IL-10, kill the lymphoma cells and sensitize them to therapeutic agents. Hence, in one aspect, it is disclosed a method of detecting and/or quantifying the expression of a panel of cytokines, particularly, IL-17, BAFF, IL-6, and IL-10 in cells from a patient (e.g., *in vivo, in vitro,* or *in situ*), to diagnose the type of lymphoma (e.g., DLBCL) and the subtype, (e.g., Activated B cell or "ABC" subtype) in the patient. In another aspect, it is disclosed a method of treating a lymphoma patient, more particularly a DLBCL patient, and even more particularly, an ABC-DLBCL patient by administering an IL-17 antagonist or a combination of an IL-17 antagonist with one or more of a BAFF antagonist, an IL-6 antagonist and/or an IL-10 antagonist.

### IL-17 and BAFF Sequences

IL-17, also known as IL-17A, interleukin 17A and cytotoxic T-lymphocyte-associated antigen 8 (CTLA-8). Human IL-17 has the following amino acid and mRNA sequences:

Recombinant human IL-17 (available, e.g., from PeproTech, Paris, France), is a 31.0 kDa homodimer of two 136 amino acid polypeptide chains, each with the following amino acid sequence:

B-cell activating factor (BAFF), also known as B-lymphcyte stimulator (BLys), CD257, Dendritic cell-derived TNF-like molecule (DTL), TALL1, TNF- and APO-related leukocyte expressed ligand 1(THANK), tumor necrosis factor ligand superfamily member 13B, TNFSF20, UNQ401/PRO738, and ZTNF4, has the following amino acid and mRNA sequences :
Isoform 1 amino acid sequence:
Isoform 1 mRNA nucleotide sequence:
Isoform 2 amino acid sequence:
Isoform 2 mRNA nucleotide sequence:

Recombinant human soluble BAFF (available, e.g., from PeproTech, Paris, France), is a 17 kDa, 153 amino acid polypeptide containing he TNF-like portion of the extracellular domain of BAFF with the following amino acid sequence:

### Identifying Subjects and Treatment and/or Prevention of Cell Proliferation Disorders

The increased expression of IL-17, as well as the IL-17-induced increase in expression of AID and/or TWIST-1, and the IL-17 induced inhibition of p53 activity can be used to identify subjects who have or are at increased risk for a cell proliferation disorder or other IL-17-related disease and/or who are likely to respond to treatment with IL-17 antagonists. Also, it has been shown that patients with an anomaly of the immune system, more specifically, autoimmune patients, e.g., patients with SLE or RA, have increased serum levels of IL-17 and BAFF. Patients with SLE and RA have an increased risk of developing, for example, non-Hodgkin's lymphoma. The present inventors have shown that IL-17 and BAFF can cooperate to induce the DNA mutating enzyme, AID, and to inhibit activity of the p53 tumor suppressor (e.g., by upregulating TWIST-1) thus leading to spontaneous B cell transformation into a cancer cell.

Accordingly, in one aspect, the present disclosure is directed to identifying a subject, *e.g.,* a human subject, who is at increased risk for a cell proliferation disorder or other IL-17 related disease. In one embodiment, the method comprises measuring the amount of IL-17 in a sample from a subject, for example, a patient who is suspected of or has certain characteristics that may give rise to a greater likelihood of developing a cell proliferation disorder or another IL-17-related disease, including but not limited to family history of disease, a disorder that leads to a compromised immune system, such as organ transplantation or autoimmune disease. The measured amount of IL-17 from the sample is compared to a reference amount of IL-17. In a particular embodiment, an amount of IL-17 that is increased compared to a reference amount indicates that the subject is at an increased risk for a cell proliferation disorder or other IL-17-related disease. In another embodiment, the method further comprises identifying a subject who is at increased risk for a cell proliferation disorder or other IL-17-related disease by measuring the amount of BAFF in a sample from the subject. In a particular embodiment, an amount of BAFF that is increased compared to a reference amount indicates that the subject is at an increased risk for a cell proliferation disorder or other IL-17-related disease (e.g., a disease, such as B-cell lymphoma, more particularly DLBCL, in which IL-17 and BAFF cooperate to cause pathogenesis and/or tumorigenesis). In another embodiment, the method further comprises measuring the amount of IL-6 and/or IL-10 in a sample from the subject. In a particular embodiment, an amount of IL-6 and/or IL-10 that is increased compared to a reference amount indicates that the subject is at an increased risk for a cell proliferation disorder such as B-cell lymphoma, more particularly DLBCL.

In a another embodiment, the method comprises determining if AID expression is increased in a sample from a subject compared to a reference amount of AID. In a particular embodiment, an amount of AID that is increased compared to a reference amount indicates that the subject is at an increased risk for a cell proliferation disorder or other IL-17-related disease (e.g., a disease in which IL-17 induces increased AID expression and results in pathogenesis and/or tumorigenesis). In a more specific embodiment, the method further comprises measuring the amount of IL-17 in a sample from the subject and comparing the measured amount to a reference amount of IL-17, wherein an increased amount of IL-17 compared to the reference amount and increased AID expression compared to the reference amount of AID expression indicates an increased risk for a cell proliferation disorder or other IL-17-related disease.

In another embodiment, the method comprises determining if TWIST-1 expression is increased in a sample from a subject compared to a reference amount of TWIST-1. In a particular embodiment, an amount of TWIST-1 that is increased compared to a reference amount indicates that the subject is at an increased risk for a cell proliferation disorder or other IL-17-related disease (e.g., a disease in which IL-17 induces increased TWIST-1 expression and results in pathogenesis and/or tumorigenesis, for example, by inhibiting p53 activity). In a more specific embodiment, the method further comprises measuring the amount of IL-17 in a sample from the subject and comparing the measured amount to a reference amount of IL-17, wherein an increased amount of IL-17 compared to the reference amount and increased TWIST-1 expression compared to the reference amount of TWIST-1 expression indicates an increased risk for a cell proliferation disorder or other IL-17-related disease. In another more specific embodiment, the method further comprises measuring IL-17 and/or TWIST-1 in a sample from the subject and comparing the measured amount to a reference amount and further determining if AID expression is increased in a sample from a subject compared to a reference amount of AID. In a particular embodiment, an amount of AID that is increased compared to a reference amount indicates that the subject is at an increased risk for a cell proliferation disorder or other IL-17-related disease.

In another aspect, the present disclosure is directed to identifying a subject *e.g.,* a human subject, who has or is at increased risk for a cell proliferation disorder or other IL-17-related disease who is likely to respond to treatment or prevention of the cell proliferation disorder or other IL-17-related disease with an IL-17 antagonist. In one embodiment, the method comprises measuring an amount of IL-17 in a sample from a subject. In a particular embodiment, an amount of IL-17 that is increased compared to a reference amount indicates that the subject is likely to respond to treatment or prevention of the cell proliferation disorder or another IL-17 related disease with an IL-17 antagonist. In another embodiment, the method further comprises identifying a subject who is likely to respond to treatment or prevention with an IL-17 antagonist or a BAFF antagonist, or a combination of both, by measuring the amount of BAFF in a sample from said subject. In a particular embodiment, an amount of BAFF that is increased compared to a reference amount indicates that the subject is likely to respond to treatment or prevention with an IL-17 antagonist, an anti-BAFF antagonist, or a combination of both. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

In an another embodiment, the method further comprises determining if AID expression is increased in a sample from a subject compared to a reference amount of AID. In a particular embodiment, an amount of AID that is increased compared to a reference amount indicates that the subject is likely to respond to treatment of a cell proliferation disorder or another IL-17-related disease (e.g., a disease in which IL-17 induces increased AID expression and resulting pathogenesis and/or tumorigenesis) with an IL-17 antagonist. In a more specific embodiment, the method further comprises measuring the amount of IL-17 in a sample from the subject and comparing the measured amount to a reference amount of IL-17, wherein an increased amount of IL-17 compared to the reference amount and increased AID expression compared to the reference amount of AID expression indicates that the subject is likely to respond to treatment or prevention of a cell proliferation disorder or another IL-17-related disease (e.g., a disease in which IL-17 induces increased AID expression and resulting pathogenesis and/or tumorigenesis) with an IL-17 antagonist. In a specific embodiment, the IL-17 antagonist is selected from the group consisting of a small molecule, an antigen binding molecule, a nucleic acid antagonist, and a protein antagonist.

In another embodiment, the method comprises determining if TWIST-1 expression is increased in a sample from a subject compared to a reference amount of TWIST-1. In a particular embodiment, an amount of TWIST-1 that is increased compared to a reference amount indicates that the subject is likely to respond to treatment or prevention of a cell proliferation disorder or another IL-17-related disease (e.g., a disease in which IL-17 induces increased TWIST-1 expression and resulting pathogenesis and/or tumorigenesis, for example, by inhibiting p53 activity) with an IL-17 antagonist. In a more specific embodiment, the method further comprises measuring the amount of IL-17 in a sample from the subject and comparing the measured amount to a reference amount of IL-17, wherein an increased amount of IL-17 compared to the reference amount and increased TWIST-1 expression compared to the reference amount of TWIST-1 expression indicates that the subject is likely to respond to treatment or prevention of a cell proliferation disorder or other IL-17-related disease with treatment of an IL-17 antagonist. In another more specific embodiment, the method further comprises measuring IL-17 and/or TWIST-1 in a sample from the subject and comparing the measured amount to a reference amount and further determining AID expression is increased in a sample from a subject compared to a reference amount of AID, wherein an amount of AID that is increased compared to a reference amount indicates that the subject is likely to respond to treatment or prevention of a cell proliferation disorder or other IL-17-related disease with treatment of an IL-17 antagonist. In a specific embodiment, the IL-17 antagonist is selected from the group consisting of a small molecule, an antigen binding molecule, a nucleic acid antagonist, and a protein antagonist.

In another aspect, the present disclosure is directed to a method of treating or preventing a cell proliferation disorder or another IL-17 related disease in a subject, e.g., a human subject, who has or is at increased risk for a cell proliferation disorder or other IL-17-related disease. In one embodiment, the method comprises: (a) measuring the amount of IL-17 in a sample from the subject; (b) comparing the measured amount of IL-17 to a reference amount of IL-17 to determine if the subject is likely to respond to treatment or prevention of the cell proliferation disorder with an anti-IL17 antagonist; and (c) administering to the subject an IL-17 antagonist in an amount effective to treat or prevent the cell proliferation disorder or other IL-17-related disease. In a particular embodiment, an amount of IL-17 that is greater than the reference amount indicates that the subject is likely to respond to treatment or prevention of the cell proliferation disorder or other IL-17-related disease with an IL-17 antagonist. In another embodiment, the method further comprises measuring the amount of BAFF in a sample from said subject. In a particular embodiment, an amount of BAFF that is increased compared to a reference amount indicates that the subject is likely to respond to treatment or prevention of a cell proliferation disorder or other IL-17-related disease with an IL-17 antagonist, an anti-BAFF antagonist, or a combination of both. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

In another embodiment, the method further comprises determining if AID expression is increased in a sample from the subject compared to a reference level of AID expression to determine if the subject is likely to respond to prevention or treatment of the cell proliferation disorder or other IL-17-related disease with an IL-17 antagonist. In a particular embodiment, increased AID expression indicates that the subject is likely to respond to treatment or prevention of the cell proliferation disorder or other IL-17-related disease with an IL-17 antagonist. In a specific embodiment, the IL-17 antagonist is selected from the group consisting of a small molecule, an antigen binding molecule, a nucleic acid antagonist, and a protein antagonist.

In one embodiment, the method comprises: (a) determining if AID expression is increased in a sample from the subject compared to a reference level of AID to identify if the subject is likely to respond to treatment or prevention of the cell proliferation disorder or other IL-17-related disease with an anti-IL17 antagonist; and (b) administering to the subject an IL-17 antagonist in an amount effective to treat or prevent the cell proliferation disorder or other IL-17-related disease. In a particular embodiment, increased AID expression indicates that the subject is likely to respond to treatment or prevention of the cell proliferation disorder or other IL-17-related disease with an anti-IL-17 antagonist. In a more specific embodiment, the method further comprises measuring the amount of IL-17 in a sample from the subject and comparing the measured amount of IL-17 to a reference amount of IL-17 to determine if the subject is likely to respond to treatment or prevention of the cell proliferation disorder or other IL-17-related disease with an anti-IL17 antagonist. In a particular embodiment, an amount of IL-17 that is greater than the reference amount indicates that the subject is likely to respond to treatment or prevention of the cell proliferation disorder or other IL-17-related disease with an IL-17 antagonist. In a more specific embodiment, the method further comprises determining if TWIST-1 expression is increased in a sample from the subject compared to a reference sample of TWIST-1. In a particular embodiment, an amount of TWIST-1 that is increased compared to a reference amount indicates that that the subject is likely to respond to treatment or prevention of the cell proliferation disorder or other IL-17-related disease with an IL-17 antagonist. In a specific embodiment, the IL-17 antagonist is selected from the group consisting of a small molecule, an antigen binding molecule, a nucleic acid antagonist, and a protein antagonist. In a particular embodiment, the measurement is made to determine whether IL-17 is produced by the cancer cells themselves (e.g., autocrine expression/production) or if cells in the tumor microenvironment express/produce IL-17. In some embodiments, measurements of other factors (e.g., cytokines such as BAFF, IL-6, or IL-17) are made to determine if cells (e.g., lymphoma cells) are expressing or producing the cytokines in an autocrine manner.As described in detail elsewhere herein, identification of a subject at increased risk for a cell proliferation disorder or other IL-17-related disease and/or identification of a subject likely to respond to treatment or prevention of a cell proliferation disorder or other IL-17-related disease with an IL-17 antagonist is, in some embodiments, accompanied by administration of an IL-17 antagonist and, optionally, another therapeutic agent and/or an antagonist of one or more of BAFF, IL-6 and/or IL-10.

Diffuse Large B Cell Lymphoma (DLBCL) is the main non-Hodgkin lymphoma in adults, representing about 31% of cases. For patients with DLBCL, the subtype has important clinical implications, since the ABC subtype is refractory to standard chemotherapy such as R-CHOP treatment (rituximab + cyclophosphamide + doxorubicin + vincristin) and has a worse prognosis compared to the GCB subtype. The original standard for DLBCL classification used gene expression profiling of frozen tissues (Alizadeh, AA, Nature, 2000; Wright, G, PNAS, 2003) which predicts patient outcome. However, such profiling is generally not feasible in the clinic. An immunohistochemistry (IHC) classification scheme based on 3 antibodies (Hans, CP et al., Blood, 2004) is used as a substitute for gene expression profiling classification. An improved IHC scheme based on 5 antibodies has also been proposed (Choi, WWL, Clin Cancer Research, 2009). However, recent clinical trials showed that IHC classification does not correlate well with gene expression profiling classification and does not predict outcome (Ott, G, Blood, 2010). Thus, accurate classification of ABC and GCB subtypes is still needed in order to predict patient prognosis and response to chemotherapy such as R-CHOP treatment. The present inventors have also surprisingly found that, in cell lines, ABC and GCB subtypes can be distinguished based on differential secretion of IL-17, BAFF, IL-6, and IL-10. Accordingly, in one aspect, it is disclosed a method of determining DLBCL subtype comprising measuring and/or quantifying the expression profile of IL-17, BAFF, IL-6 and IL-10 in a sample from a subject and comparing the expression profile to a reference profile for ABC subtype of DLBCL to determine if the expression profile indicates that the subject has the ABC subtype of DLBCL. In another embodiment, the method further comprises administering to the subject an amount of an IL-17 antagonist, or and IL-17 antagonist in combination with one or more of a BAFF antagonist, an IL-6 antagonist, and/or an IL-10 antagonist.

In a particular embodiment, the subject has an anomaly of the immune system. In a specific embodiment, the subject is immunocompromised. In another specific embodiment, the subject is the recipient of an organ or tissue transplant. In another specific embodiment, the subject has an autoimmune disorder, more specifically systemic lupus erythematosus (SLE) or rheumatoid arthritis (RA).

According to the methods disclosed therein, the reference amount of the molecule, factor or substance being used for comparison (e.g., the reference amount of IL-17, BAFF, AID, TWIST-1, *etc*.) is an average amount determined from a sample or population of samples of the same type (*e.g.,* same tissue or fluid type) that are determined to be normal and/or non-diseased, although the reference amount can be established by methods that are known and routinely used by those in the field.

Examples of cell proliferation disorders include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

In some particular embodiments, the cell proliferation disorder is a cancer of a tissue or organ selected from the group consisting of breast, bladder, liver, colon, ovary, lung, kidney, cervix, stomach, intestine, prostate, connective tissue, and skin. In more particular embodiments, the cell proliferation disorder is a cancer of the breast, colon, lung, ovary, esophagus, head and neck, or skin (e.g., melanoma). In another particular embodiment, the cell proliferation disorder is a lymphoproliferative disorder. In one embodiment, the lymphoproliferative disorder is a hematological malignancy. In a more particular embodiment, the hematological malignancy is selected from the group consisting of lymphoma, acute myeloid leukemia, chronic lymphcytic leukemia, acute lymphobalstic leukemia, chronic myelogenous leukemia, myeloma, and hairy cell leukemia. In a specific embodiment, the cell proliferation disorder in is lymphoma. In another specific embodiment, the cell proliferation disorder is breast cancer. In another specific embodiment, the cell proliferation disorder is hepatocellular carcinoma.

Examples of other IL-17-related diseases include neoplastic or pre-cancerous conditions and fibrosis, more specifically, liver fibrosis. Fibrosis may be induced by conditions such as alcohol abuse, genetic mutation, or viral infection. In a specific embodiment, fibrosis is caused by infection with a hepatitis virus. In a more specific embodiment, the hepatitis virus is selected from hepatitis C (HCV) and hepatitis B (HBV).

### Preventing or Reducing IL-17-induced DNA Damage

IL-17 induces increased expression of AID and inhibits the p53 DNA repair safeguard mechanisms (for example, by increasing expression of TWIST-1, a potent inhibitor of p53 activity), causing genomic instability and DNA damage that leads to carcinogenesis. In B-cells, IL-17 can also act in cooperation with BAFF to cause DNA damage and genomic instability. In hepatocytes, IL-17 can cooperate with hepatitis virus to generate genomic instability by upregulating AID and inhibiting p53 activity (e.g., through TWIST-1).

Accordingly, in one aspect, it is disclosed a method of preventing or reducing IL-17 induced DNA damage in a cell, more specifically, a mammalian cell, and even more specifically, a human cell, the method comprising contacting the cell with an IL-17 antagonist in an amount effective to prevent or reduce IL-17-induced DNA damage. In a particular embodiment, the IL-17-induced DNA damage is a result of increased expression of AID. In another particular embodiment, the IL-17-induced DNA damage is a result of increased expression of TWIST-1. In another particular embodiment, the IL-17-induced DNA damage is a result of inhibition of the p53 tumor suppressor pathway. In another embodiment, the IL-17-induced DNA damage is a result of a combination of one or more of the factors selected from the group consisting of increased expression of AID, increased expression of TWIST-1, and inhibition of the p53 tumor suppressor pathway. In a specific embodiment, the IL-17 antagonist is selected from the group consisting of a small molecule, an antigen binding molecule, a nucleic acid antagonist, and a protein antagonist.

In another aspect, it is disclosed a method of preventing or reducing IL-17-induced DNA damage in one or more cells of a human subject having or at risk of developing a cell proliferation disorder, said method comprising: (a) measuring the amount of IL-17 in a sample from the subject; (b) comparing the measured amount of IL-17 to a reference amount of IL-17 to determine if the subject is likely to respond to treatment or prevention of IL-17-induced damage with an anti-IL17 antagonist; and (c) administering to the subject an IL-17 antagonist in an amount effective to prevent or reduce the IL-17-induced DNA damage. In one embodiment, the method further comprises determining if AID expression is increased in a sample from the subject compared to a reference level of AID expression to identify if the subject is at increased risk for IL-17-induced DNA damage, wherein increased AID expression indicates that the subject at increased risk for IL-17-induced DNA damage. In one embodiment, the method further comprises determining if TWIST-1 expression is increased in a sample from the subject compared to a reference level of TWIST-1 expression to identify if the subject is at increased risk for IL-17-induced DNA damage, wherein increased TWIST-1 expression indicates that the subject at increased risk for IL-17-induced DNA damage. In another embodiment, both AID and TWIST-1 expression are measured and compared to reference amounts of AID and TWIST-1, respectively. In one embodiment, the method further comprises measuring the amount of BAFF in a sample from the subject and comparing the measured amount of BAFF to a reference amount of BAFF to determine if the subject is likely to respond to treatment with an anti-BAFF antagonist, wherein an amount of BAFF that is greater than the reference amount indicates that the subject is likely to respond; and administering to the subject an anti-BAFF antagonist in combination with the IL-17 antagonist in an amount effective to prevent or reduce IL-17-induced DNA damage. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

As disclosed therein, the mammalian cell may be *in vitro, in vivo,* or *ex vivo.* In a specific embodiment, the cell is *in vivo* in a human subject. In another specific embodiment, the mammalian cell is a human cell. In a particular embodiment, the mammalian cell is selected from the non-limiting group consisting of a mammary (breast) cell, a hepatocyte (liver cell), an ovarian cell, a B-cell, a cervical cell, an endothelial cell, a blood cell, a hematopoietic cell, a prostate cell, a skin cell, a stomach cell, an intestinal cell, an epithelial cell, a fibroblast, a pancreatic cell, a renal (kidney) cell, a colorectal cell, and a bone cell. Other non-limiting examples of cells, encompassed by the method disclosed therein are provided in other sections herein. In a specific embodiment, the mammalian cell is a mammary cell, more specifically a human mammary cell. In another specific embodiment, the mammalian cell is a hepatocyte, more specifically, a human hepatocyte. In a more specific embodiment, the hepatocyte is infected with a virus, more specifically, a hepatitis virus (e.g., HBV or HCV). In another specific embodiment, the mammalian cell is a B lymphocyte (B cell), more specifically, a human B lymphocyte. In one embodiment, the mammalian cell is a cancer cell. Non-limiting examples of types of cancer a provided elsewhere herein, and the cancerous cell could be derived from any of these cancers. In a specific embodiment, the cancer cell is selected from the group consisting of a lymphoma cell, a breast cancer cell, and a hepatocellullar carcinoma cell.

In one embodiment, it is disclosed a method of preventing or reducing IL-17-induced DNA damage in a hepatocyte, more specifically, a human hepatocyte that is infected with a virus, the method comprising contacting the cell with an IL-17 antagonist in an amount effective to prevent or reduce the IL-17-induced DNA damage. In a more specific embodiment, the virus is a hepatitis virus. In a more specific embodiment, the hepatitis virus is hepatitis C virus (HCV) or hepatitis B virus (HBV). In one embodiment, the hepatocyte is a hepatocellular carcinoma cell. In a particular embodiment, the hepatocyte is *in vivo* in a human subject.

In one embodiment, it is disclosed a method of preventing or reducing IL-17-induced DNA damage or damage induced by IL-17 and BAFF in a B-cell, the method comprising contacting the cell with an IL-17 antagonist in an amount effective to prevent or reduce the IL-17-induced DNA damage. In another embodiment, the method further comprises contacting the cell with a BAFF antagonist. In other embodiments, the method further comprises contacting the cell with an IL-6 antagonist and/or an IL-10 antagonist. In a specific embodiment, the B-cell is a cancerous B-cell. In a more specific embodiment, the B-cell is a lymphoma cell. In a more specific embodiment, the lymphoma cell is a non-Hodgkins lymphoma cell. In one embodiment, the cell is in a subject who is at increased risk for a B-cell cancer. In a more specific embodiment, the subject is immunocompromised. In a more specific embodiment, the subject has an autoimmune disease. In a more specific embodiment, the autoimmune disease is SLE or RA. In one embodiment, the BAFF antagonist is selected from the group consisting of a small molecule, a BAFF-specific antigen binding molecule, a nucleic acid antagonist, and a protein antagonist. In a more specific embodiment, the BAFF antagonist is an antigen binding molecule selected from the group consisting of a full antibody and an antigen binding fragment. In a specific embodiment, the IL-17 antagonist is selected from the group consisting of a small molecule, an antigen binding molecule, a nucleic acid antagonist, and a protein antagonist. In one embodiment, the IL-6 antagonist is selected from the group consisting of a small molecule, an IL-6-specific antigen binding molecule, a nucleic acid antagonist, and a protein antagonist. In one embodiment, the IL-10 antagonist is selected from the group consisting of a small molecule, an IL-10-specific antigen binding molecule, a nucleic acid antagonist, and a protein antagonist.

### Preventing or Reverting IL-17-induced Survival of Abnormal Cells, Maintaining and/or Improving Activity of a Chemotherapeutic Agent, and/or Inducing Cell Death

The present inventors have found that IL-17 production can inhibit the p53 tumor suppressor pathway. Inhibition of p53 by IL-17 can lead to the abnormal survival of cells that would otherwise undergo cell death (e.g., apoptosis) if the p53 pathway were functioning normally. Loss of p53--whether by mutation, decreased expression, or other inhibitions of its activity--is also associated with the development of resistance of cancers to chemotherapy or radiotherapy. *See, e.g.,* Gasco and Crook, Drug Resistance Updates 6: 323-328 (2003); Fojo, T., Drug Resistance Updates 5: 209-216 (2002); and Onishi et al, Anti-Cancer Agents in Medicinal Chemistry 8: 564-570 (2008). Also, an increase in the expression of the transcription factor, TWIST-1, has been associated with chemoresistance of certain cancer types to chemotherapeutic drugs. For example TWIST-1 is associated with chemoresistance of nasopharyngeal carcinoma to vincristin-paclitaxel; breast carcinoma to paclitaxel; fibroblasts in prostate carcinoma to danorubicin-cisplatin; lung adenocarcinoma to cisplatin; ovarian carcinoma to paclitaxel, and prostate carcinoma to paclitaxel. For a review, *see* Ansieau et al., Oncogene (2010) 1-12. As shown by the present inventors, administration of IL-17 antagonists can increase apoptosis of abnormal cells, prevent inhibition of the p53 pathway, and work in cooperation with chemotherapeutic agents to prevent or revert the survival of abnormal cells.

Accordingly, in one aspect, it is disclosed a method of preventing or reverting IL-17-induced abnormal survival of a mammalian cell, the method comprising contacting the cell with an IL-17 antagonist in an amount effective to prevent or revert IL-17-induced survival. In a specific embodiment, the abnormal cell is a precancerous cell. In another specific embodiment, the abnormal cell is a cancer cell (e.g., a cell that has already undergone transformation). In a specific embodiment IL-17 can be produced by pre-cancerous or a cancer cell microenvironment. In a specific embodiment, the abnormal mammalian cell is *in vivo* in a human subject. In a more specific embodiment, the method further comprises measuring the amount of IL-17, AID, and/or TWIST-1 in a sample from the human subject and comparing the measured amount of IL-17, AID, and/or TWIST-1, respectively, to a reference amount of IL-17, AID, and/or TWIST-1, to determine if the abnormal mammalian cell in the subject is likely to respond to prevention or reversion of IL-17-induced survival by treatment with an IL-17 antagonist, wherein an amount of IL-17, AID, and/or TWIST-1 that is greater than the reference amount indicates that the cell is likely to respond. In a specific embodiment, the IL-17 antagonist is selected from the group consisting of a small molecule, an antigen binding molecule, a nucleic acid antagonist, and a protein antagonist.

In another aspect, it is disclosed a method of inducing cell death of an IL-17-expressing cell, the method comprising contacting the cell with an IL-17 antagonist in an amount effective to induce cell death of the cell. In a particular embodiment, the cell is a cancer cell. In a specific embodiment, the IL-17-expressing cell is *in vivo* in a human subject. In a more specific embodiment, the method further comprises measuring the amount of IL-17, AID, and/or TWIST-1 in a sample from the human subject and comparing the measured amount of IL-17, AID, and/or TWIST-1, respectively, to a reference amount of IL-17, AID, and/or TWIST-1, to determine if the IL-17-expressing cell in the subject is likely to respond to induction of cell death by treatment with an IL-17 antagonist, wherein an amount of IL-17, AID, and/or TWIST-1 that is greater than the reference amount indicates that the cell is likely to respond. In a specific embodiment, the IL-17 antagonist is selected from the group consisting of a small molecule, an antigen binding molecule, a nucleic acid antagonist, and a protein antagonist. In another embodiment, the method further comprises contacting the cell with a chemotherapeutic agent.

In another aspect, the present disclosure is directed to increasing the effectiveness of a therapeutic agent for killing abnormally proliferating cells. In one embodiment, the method comprises administering to a subject with abnormally proliferating cells an amount of a therapeutic agent either before, simultaneous with, or after an IL-17 antagonist or a combination of an IL-17 antagonist with one or more of a BAFF antagonist, an IL-6 antagonist, and/or an IL-10 antagonist. In another embodiment, the method comprises: (a) measuring the amount of IL-17 in a sample from a subject, e.g., from a subject having a cell proliferation disorder; (b) comparing the amount of IL-17 in the sample to a reference amount of IL-17 to identify if the abnormally proliferating cells of the subject are likely to respond to treatment with an IL-17 antagonist.; and (c) administering an amount of an IL-17 antagonist effective to increase the effectiveness of the therapeutic agent at a time selected from the group consisting of before, during, or after administration of the therapeutic agent. In a particular embodiment, an amount of IL-17 that is greater than the reference amount indicates that the abnormally proliferating cells of the subject are likely to respond to treatment with the IL-17 antagonist. In another embodiment, the method further comprises measuring the amount of BAFF in a sample from the subject and comparing the measured amount of BAFF to a reference amount of BAFF to determine if the subject is likely to respond to treatment with an IL-17 antagonist, a BAFF antagonist, or a combination of both. In a particular embodiment, an amount of BAFF that is increased compared to a reference amount indicates that the subject is likely to respond to treatment or prevention with an IL-17 antagonist, a BAFF antagonist, or a combination of both. In another embodiment, the method further comprises detecting and/or measuring the expression of IL-6 and/or IL-10 in a sample from the subject and administering an amount of an IL-17 antagonist or an IL-17 antagonist with any combination of a BAFF antagonist, an IL-6 antagonist, and/or an IL-10 antagonist that is effective to increase the effectiveness of the therapeutic agent at a time selected from the group consisting of before, during, or after administration of the therapeutic agent. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

Exemplary therapeutic agents include, but are not limited to chemotherapeutic agents such as vinca alkaloids, epipodophyllotoxins, anthracycline antibiotics, actinomycin D, plicamycin, puromycin, gramicidin D, paclitaxel (Taxol™, Bristol Myers Squibb), colchicine, cytochalasin B, emetine, maytansine, and amsacrine (or "mAMSA"). The vinca alkaloid class is described in GOODMAN AND GILMAN'S THE PHARMACOLOGICAL BASIS OF THERAPEUTICS (7th ed.), (1985), pp. 1277-1280. Exemplary of vinca alkaloids are vincristine, vinblastine, and vindesine. The epipodophyllotoxin class is described, for example, in GOODMAN AND GILMAN'S THE PHARMACOLOGICAL BASIS OF THERAPEUTICS (7th ed.), (1985), pp. 1280-1281. Exemplary of epipodophyllotoxins are etoposide, etoposide orthoquinone, and teniposide. The anthracycline antibiotic class is described in GOODMAN AND GILMAN'S THE PHARMACOLOGICAL BASIS OF THERAPEUTICS (7th ed.), (1985), pp. 1283-1285. Exemplary of anthracycline antibiotics are daunorubicin, doxorubicin, mitoxantraone, and bisanthrene. Actinomycin D, also called Dactinomycin, is described, for example, in GOODMAN AND GILMAN'S THE PHARMACOLOGICAL BASIS OF THERAPEUTICS (7th ed.), (1985), pp. 1281-1283. Plicamycin, also called mithramycin, is described in Goodmand and Gilman's The Pharmacological Basis of Therapeutics (7th ed), (1985), pp.1287-1288. Additional chemotherapeutic agents include cisplatin (Platinol™, Bristol Myers Squibb), carboplatin (Paraplatin™, Bristol Myers Squibb), mitomycin (Mutamycin™., Bristol Myers Squibb), altretamine (Hexalen™, U.S. Bioscience, Inc.), cyclophosphamide (Cytoxan™, Bristol Myers Squibb), lomustine (CCNU) (CeeNU™ Bristol Myers Squibb), carmustine (BCNU) (BiCNU™, Bristol Myers Squibb).

Exemplary chemotherapeutic agents also include aclacinomycin A, aclarubicin, acronine, acronycine, adriamycin, aldesleukin (interleukin-2), altretamine (hexamiethylmelamine), aminoglutethimide, aminoglutethimide (cytadren), aminoimidazole carboxamide, amsacrine (m-AMSA; amsidine), anastrazole (arimidex), ancitabine, anthracyline, anthramycin, asparaginase (elspar), azacitdine, azacitidine (ladakamycin), azaguanine, azaserine, azauridine, 1,1',1" -phosphinothioylidynetris aziridine, azirino(2', 3':3,4)pyrrolo(1,2-a)indole-4,7-dione, BCG (theracys), BCNU, BCNU chloroethyl nitrosoureas, benzamide, 4-(bis(2-chloroethyl)amino)benzenebutanoic acid, bicalutamide, bischloroethyl nitrosourea, bleomycin (blenozane), bromodeoxyuridine, broxuridine, busulfan (myleran), carbamic acid ethyl ester, chlorambucil (leukeran), chloroethyl nitrosoureas, chorozotocin (DCNU), chromomycin A3, cis-retinoic acid, cladribine (2-chlorodeoxyadenosine; 2cda; leustatin), coformycin, cycloleucine, cyclophosphamide anhydrous, chlorambucil, cytarabine, cytarabine, cytarabine HCl (cytosar-u), 2-deoxy-2-(((methylnitrosoamino)carbonyl)amino)-D-glucose, dacarbazine, decarbazine, decarbazine (DTIC-dome), demecolcine, dexamethasone, dianhydrogalactitol, diazooxonorleucine, diethylstilbestrol, docetaxel (taxotere), eflomithine, estramustine, estramustine phosphate sodium (emcyt), ethiodized oil, ethoglucid, ethyl carbamate, ethyl methanesulfonate, fenretinide, floxuridine, floxuridine (fudr), fludarabine (fludara), fluorouracil (5-FU), fluoxymesterone (halotestin), flutamide, flutamide (eulexin), fluxuridine, gallium nitrate (granite), gemcitabine (gemzar), genistein, 2-deoxy-2-(3-methyl-3-nitrosoureido)-D-glucopyranose, goserelin (zoladex), hexestrol, hydroxyurea (hydra), idarubicin (idamycin), ifosfagemcitabine, ifosfamide (iflex), ifosfamide with mesna (MAID), interferon, interferon alfa, interferon alfa-2a, alfa-2b, alfa-n3, interleukin-2, iobenguane, iobenguane iobenguane, irinotecan (camptosar), isotretinoin (accutane), ketoconazole, 4-(bis(2-chloroethyl)amino)-L-phenylalanine, L-serine diazoacetate, lentinan, leucovorin, leuprolide acetate (LHRH-analog), levamisole (ergamisol), mannomustine, maytansine, mechlorethamine, mechlorethamine HCl (nitrogen mustard), medroxyprogesterone acetate (provera, depo provera), megestrol acetate (menace), melengestrol acetate, melphalan (alkeran), menogaril, mercaptopurin, mercaptopurine (purinethol), mercaptopurine anhydrous, MESNA, mesna (mesne), methanesulfonic acid, ethyl ester, methotrexate (mtx; methotrexate), methyl-ccnu, mimosine, misonidazole, mithramycin, mitoantrone, mitobronitol, mitoguazone, mitolactol, mitomycin (mutamycin), mitomycin C, mitotane (o,p'-DDD; lysodren), mitoxantrone HCl (novantrone), mopidamol, N,N-bis(2-chloroethyl)tetrahydro-2H-1,3,2-oxazaphosphorin-2-amine-2-oxide, N-(1-methylethyl)-4-((2-methylhydrazino)methyl)benzamide, N-methyl-bis(2-chloroethyl)amine, nicardipine, nilutamide (nilandron), nimustine, nitracrine, nitrogen mustard, nocodazole, nogalamycin, octreotide (sandostatin), pactamycin, pegaspargase (PEGx-1), pentostatin (2'-deoxycoformycin), peplomycin, peptichemio, photophoresis, picibanil, pipobroman, podofilox, podophyllotoxin, porfiromycin, prednisone, procarbazine, procarbazine HCl (matulane), prospidium, puromycin aminonucleoside, PUVA (psoralen+ultraviolet a), pyran copolymer, rapamycin, s-azacytidine, 2,4,6-tris(1-aziridinyl)-s-triazine, semustine, showdomycin, sirolimus, streptozocin (zanosar), suramin, tamoxifen citrate (nolvadex), taxon, tegafur, tenuazonic acid, TEPA, testolactone, thio-tepa, thioguanine, thiotepa (thioplex), tilorone, topotecan, tretinoin (vesanoid), triaziquone, trichodermin, triethylene glycol diglycidyl ether, triethylenemelamine, triethylenephosphoramide, triethylenethiophosphoramide, trimetrexate (neutrexin), tris(1-aziridinyl)phosphine oxide, tris(1-aziridinyl)phosphine sulfide, tris(aziridinyl)-p-benzoquinone, tris(aziridinyl)phosphine sulfide, uracil mustard, vidarabine, vidarabine phosphate, vinorelbine, vinorelbine tartrate (navelbine), (1)-mimosine, 1-(2-chloroethyl)-3-(4-methylcyclohexyl)-1-nitrosourea, (8S-cis)-10-((3-amino-2,3,6-trideoxy-alpha-L-lyxo-hexopyranosyl)oxy)-7,8,9,10-tetrahydro-6,8,11-trihydroxy-8-(hydroxyacetyl)-1-methoxy-5,12-naphthacenedione, 131-meta-iodobenzyl guanidine (I-131 MIBG), 5-(3,3-dimethyl-1-triazenyl)-1H-imidazole-4-carboxamide, 5-(bis(2-chloroethyl)amino)-2,4(1H,3H)-pyrimidinedione, 2,4,6-tris(1-aziridinyl)-s-thiazine, 2,3,5-tris(1-aziridinyl)-2,5-cyclohexadiene-1,4-dione, 2-chloro-N-(2-chloroethyl)-N-methylethanamine, N,N-bis(2-chloroethyl)tetrahydro-2H-1,3,2-oxazaphosphorin-2-amine-2-oxide, 3-deazauridine, 3-iodobenzylguanidine, 5,12-naphthacenedione, 5-azacytidine, 5-fluorouracil, (1aS,8S,8aR,8bS)-6-amino-8-(((aminocarbonyl)oxy)methyl)-1,1a, 2,8,8a,8b-hexahydro-8a-methoxy-5-methylazirino(2',3':3,4)pyrrolo(1,2-a)indole-4,7-dione, 6-azauridine, 6-mercaptopurine, 8-azaguanine, and combinations thereof.

In a particular embodiment, the chemotherapeutic agent used in the methods of the present invention is doxorubicin. In another particular embodiment, the chemotherapeutic agent used in the methods of the present invention is paclitaxel.

Exemplary therapeutic agents also include, but are not limited to, radiation therapies, tyrosine kinase inhibitors (e.g., azitinib, bosutinib, cediranib, crizotinib, dasatinib, erlotinib, gefitinib, imatinib, lapatinib, neratinib, nilotinib, ruxolitinib, semaxanib, vandentanib) and therapeutic antibodies (e.g., abagovomab, abciximab, adalimumab, adecatumumab, alemtuzumab, altizumab, belimumab, bevacizumab, cetuximab, gemtuzumab, ibritumomab, inflilximab, panitumumab, rituximab, tositumomab, trastuzumab).

Examples of cells that are encompassed by the present disclosure include, but are not limited to, a mammary (breast) cell, a hepatocyte (liver cell), an ovarian cell, a B-cell, a cervical cell, an endothelial cell, a blood cell, a hematopoietic cell, a prostate cell, a skin cell, a stomach cell, an intestinal cell, an epithelial cell, a pancreatic cell, a fibroblast, a renal (kidney) cell, a colorectal cell, and a bone cell. Other non-limiting examples of cells, encompassed by the method disclosed therein are provided in other sections herein. In a specific embodiment, the mammalian cell is a mammary cell, more specifically a human mammary cell. In another specific embodiment, the mammalian cell is a hepatocyte, more specifically, a human hepatocyte. In a more specific embodiment, the hepatocyte is infected with a virus. In another specific embodiment, the mammalian cell is a B lymphocyte (B cell), more specifically, a human B lymphocyte. In one embodiment, the mammalian cell is a cancer cell. Non-limiting examples of types of cancer are provided elsewhere herein, and the cancerous cell could be derived from any one of these cancers. In a specific embodiment, the cancer cell is selected from the group consisting of a lymphoma cell, a breast cancer cell, and a hepatocellullar carcinoma cell.

### Preventing or Reverting IL-17-induced Cell Transformation

As shown herein, IL-17 can immortalize cells and transform normal cells into tumor cells (referred to herein as "transformation"). IL-17 cooperates with BAFF to cause immortalization and transformation of normal B-lymphocytes into tumor cells. Transformed B cells can also express IL-17 and BAFF in an autocrine fashion. As shown herein, antagonists (e.g., antibodies) to IL-17 and BAFF can induce apoptosis in transformed B-cells. DLBCL cells also express IL-6 and IL-10 with IL-17 and BAFF in a characteristic fashion.

Also, as shown herein, IL-17 can cause oncogenic mutations and transform normal immortalized mammary epithelial cells into cancer cells. As also shown herein, HCV-infected primary hepatocytes were shown to produce IL-17. IL-17, but not TNF-α or TGF-β, can cooperate with the hepatitis C virus (HCV) to increase AID expression as well as Twist-1 expression which in turn inhibits the expression of the tumor suppressor gene p53 and leads to upregulation of oncogenes (e.g., c-Myc) and transformation of hepatocytes.

Accordingly, in one aspect, it is disclosed a method of preventing or reverting IL-17-induced transformation of a mammalian cell, the method comprising contacting the cell with an IL-17 antagonist in an amount effective to prevent or revert said IL-17-induced transformation. In a specific embodiment, the IL-17 antagonist is selected from the group consisting of a small molecule, an antigen binding molecule, a nucleic acid antagonist, and a protein antagonist. In another embodiment, the method further comprises contacting the cell with one or more of a BAFF antagonist, an IL-6 antagonist, and/or an IL-10 antaqonist.

In another aspect, it is disclosed a method of preventing or reverting IL-17-induced transformation of a mammalian cell of a human subject having or at risk of developing a cell proliferation disorder, the method comprising: (a) measuring the amount of IL-17 in a sample from the subject; (b) comparing the measured amount of IL-17 to a reference amount of IL-17 to determine if the subject is likely to respond to prevention or reversion of IL-17-induced cell transformation with an anti-IL17 antagonist; and (c) administering to the subject an IL-17 antagonist in an amount effective to prevent or reduce the IL-17-induced cell transformation. In one embodiment, the method further comprises determining if AID expression is increased in a sample from the subject compared to a reference level of AID expression to identify if the subject is at increased risk for IL-17-induced cell transformation, wherein increased AID expression indicates that the subject at increased risk for IL-17-induced cell transformation. In one embodiment, the method further comprises determining if TWIST-1 expression is increased in a sample from the subject compared to a reference level of TWIST-1 expression to identify if the subject is at increased risk for IL-17-induced cell transformation, wherein increased TWIST-1 expression indicates that the subject at increased risk for IL-17-induced cell transformation. In another embodiment, both AID and TWIST-1 expression are measured and compared to reference amounts of AID and TWIST-1, respectively. In one embodiment, the method further comprises measuring the amount of BAFF in a sample from the subject and comparing the measured amount of BAFF to a reference amount of BAFF to determine if the subject is likely to respond to treatment or reversion of IL-17-induced cell transformation with an anti-BAFF antagonist, wherein an amount of BAFF that is greater than the reference amount indicates that the subject is likely to respond; and administering to the subject an anti-BAFF antagonist in combination with the IL-17 antagonist in an amount effective to prevent or reduce IL-17-induced cell transformation. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

According to the present disclosure, the mammalian cell may be *in vitro, in vivo, in situ,* or *ex vivo.* In a specific embodiment, the cell is *in vivo* in a human subject. In another specific embodiment, the mammalian cell is a human cell. In a particular embodiment, the mammalian cell is selected from the non-limiting group consisting of a mammary (breast) cell, a hepatocyte (liver cell), an ovarian cell, a B-cell, a cervical cell, an endothelial cell, a blood cell, a hematopoietic cell, a prostate cell, a skin cell, a stomach cell, an intestinal cell, an epithelial cell, a pancreatic cell, a renal (kidney) cell, a fibroblast, a colorectal cell, and a bone cell. Other non-limiting examples of cells, encompassed by the method disclosed therein are provided in other sections herein. In a specific embodiment, the mammalian cell is a mammary cell, more specifically a human mammary cell. In another specific embodiment, the mammalian cell is a hepatocyte, more specifically, a human hepatocyte. In a more specific embodiment, the hepatocyte is infected with a virus, more specifically, a hepatitis virus (e.g., HBV or HCV). In another specific embodiment, the mammalian cell is B cell, more specifically, a human B lymphocyte. In one embodiment, the mammalian cell is a cancer cell. Non-limiting examples of types of cancer a provided elsewhere herein, and the cancerous cell could be derived from any of these cancers. In a specific embodiment, the cancer cell is selected from the group consisting of a lymphoma cell, a breast cancer cell, a colon cancer cell, a lung cancer cell, an ovarian cancer cell, an esophageal cell, a head and neck cancer cell, a melanoma cell, and a hepatocellullar carcinoma cell.

In a specific embodiment, the method comprises preventing or reverting IL-17 induced transformation of a hepatocyte, more specifically, a human hepatocyte, the method comprising contacting the hepatocyte with an IL-17 antagonist in an amount effective to prevent or revert IL-17-induced transformation of the hepatocyte. In a specific embodiment, the hepatocyte is infected with a virus. In a more specific embodiment, the virus is a hepatitis virus. In a more specific embodiment, the hepatitis virus is selected from the group consisting of HBV and HCV. In one embodiment, the hepatocyte is a hepatocelluar carcinoma cell. In a particular embodiment, the hepatocyte is *in vivo* in a human subject. In a particular embodiment, transformation is induced by IL-17 and a virus, more specifically, a hepatitis virus, and even more specifically HCV or HBV. In a specific embodiment, the IL-17 antagonist is selected from the group consisting of a small molecule, an antigen binding molecule, a nucleic acid antagonist, and a protein antagonist.

In one embodiment, the method comprises preventing or reverting IL-17-induced transformation of a mammary cell, more specifically, a human mammary cell, the method comprising contacting the mammary cell with an IL-17 antagonist in an amount effective to prevent or revert IL-17-induced transformation of the mammary cell. In a particular embodiment, the mammary is *in vivo* in a human subject. In a specific embodiment, the IL-17 antagonist is selected from the group consisting of a small molecule, an antigen binding molecule, a nucleic acid antagonist, and a protein antagonist.

In one embodiment, the method comprises preventing or reverting IL-17-induced transformation of a B cell, more specifically, a human B cell, the method comprising contacting the B cell with an IL-17 antagonist in an amount effective to prevent or revert IL-17-induced transformation of the B cell. In another embodiment, the method further comprises contacting the B-cell with a BAFF antagonist in an amount effective to prevent or revert transformation of the B cell induced by the activity of BAFF and IL-17. In another embodiment, the method further comprises contacting the B-cell with an IL-6 antagonist and/or an IL-10 antagonist in an amount effective to prevent or revert transformation of the B cell induced by the activity of BAFF and IL-17. In a particular embodiment, the B cell is *in vivo* in a human subject. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist. In a specific embodiment, the IL-6 antagonist is selected from the group consisting of a small molecule, an antigen binding molecule, a nucleic acid antagonist, and a protein antagonist. In a specific embodiment, the IL-10 antagonist is selected from the group consisting of a small molecule, an antigen binding molecule, a nucleic acid antagonist, and a protein antagonist.

### Preventing or Reverting IL-17-induced Inhibition of the p53 Suppressor Pathway

The present inventors have found that IL-17 production can inhibit the p53 tumor suppressor pathway. IL-17 induces an upregulation of the transcription factor, TWIST-1, which is a potent inhibitor of p53 activity. Inhibition of p53 by IL-17 can lead to the survival of cells that would otherwise undergo cell death (e.g., apoptosis) if the p53 pathway were functioning normally. As shown by the present inventors, administration of IL-17 antagonists can prevent or revert inhibition of the p53 pathway.

Accordingly, in one aspect, it is disclosed a method of preventing or reverting IL-17-induced inhibition of the p53 suppressor pathway in one or more cells of a human subject having or at risk of developing a cell proliferation disorder, the method comprising: (a) measuring the amount of IL-17 in a sample from the subject; (b) comparing the measured amount of IL-17 to a reference amount of IL-17 to determine if the subject is likely to respond to prevention or reversion of IL-17 induced inhibition of p53 with an anti-IL17 antagonist; and (c) administering to the subject an IL-17 antagonist in an amount effective to prevent or revert said IL-17-induced inhibition of the p53 suppressor pathway. In a particular embodiment, an amount of IL-17 that is higher than a reference amount indicates that the subject is likely to respond to prevention or reversion of IL-17 induced inhibition of p53 with an anti-IL17 antagonist. In another embodiment, the method further comprises determining if TWIST-1 expression is increased in a sample from the subject compared to a reference level of TWIST-1 expression to identify if the subject is at increased risk for IL-17-induced inhibition of the p53 suppressor pathway and/or if the subject is likely to respond to prevention or reversion of IL-17-induced inhibition of the p53 suppressor pathway with an IL-17 antagonist. In a particular embodiment, TWIST-1 expression indicates that the subject is at increased risk for IL-17 induced inhibition of the p53 tumor suppressor pathway and/or is likely to respond to prevention or reversion of IL-17-induced inhibition of the p53 suppressor pathway with an IL-17 antagonist. In a specific embodiment, the IL-17 antagonist is selected from the group consisting of a small molecule, an antigen binding molecule, a nucleic acid antagonist, and a protein antagonist.

In another embodiment, the method further comprises measuring the amount of BAFF in a sample from the subject to determine if subject is at increased risk for inhibition of the p53 suppressor pathway induced by IL-17 and/or a combination of IL-17 and BAFF, and to determine if the subject is likely to respond to prevention or reversion of IL-17- or IL-17/BAFF-induced inhibition of the p53 suppressor pathway with an IL-17 antagonist or a combination of an IL-17 antagonist and a BAFF antagonist. In a particular embodiment, an amount of BAFF that is increased compared to a reference amount indicates that the subject is likely to respond to prevention or reversion of IL-17- or IL-17/BAFF-induced inhibition of the p53 suppressor pathway with an IL-17 antagonist, an anti-BAFF antagonist, or a combination of both. In a specific embodiment, the IL-17 antagonist is selected from the group consisting of a small molecule, an antigen binding molecule, a nucleic acid antagonist, and a protein antagonist. In a specific embodiment, the BAFF antagonist is selected from the group consisting of a small molecule, an antigen binding molecule, a nucleic acid antagonist, and a protein antagonist.

According to the present disclosure, the cell may be *in vitro, in vivo, in situ,* or *ex vivo.* In a specific embodiment, the cell is *in vivo* in a human subject. In a particular embodiment, the cell is selected from the non-limiting group consisting of a mammary (breast) cell, a hepatocyte (liver cell), an ovarian cell, a B-cell, a cervical cell, an endothelial cell, a blood cell, a hematopoietic cell, a prostate cell, a skin cell, a stomach cell, an intestinal cell, an epithelial cell, a pancreatic cell, a renal (kidney) cell, a fibroblast, a colorectal cell, and a bone cell. In a more particular embodiment, the cell is selected from the group consisting of a lymphoma cell, a breast cancer cell, a colon cancer cell, a lung cancer cell, an ovarian cancer cell, an esophageal cell, a head and neck cancer cell, and a melanoma cell. Other non-limiting examples of cells, encompassed by the method disclosed therein are provided in other sections herein. In a specific embodiment, the mammalian cell is a mammary cell, more specifically a human mammary cell. In another specific embodiment, the mammalian cell is a hepatocyte, more specifically, a human hepatocyte. In a more specific embodiment, the hepatocyte is infected with a virus, more specifically, a hepatitis virus (e.g., HBV or HCV). In another specific embodiment, the mammalian cell is a B lymphocyte (B cell), more specifically, a human B lymphocyte. In one embodiment, the mammalian cell is a cancer cell. Non-limiting examples of types of cancer a provided elsewhere herein, and the cancerous cell could be derived from any of these cancers. In a specific embodiment, the cancer cell is selected from the group consisting of a lymphoma cell, a breast cancer cell, and a hepatocellullar carcinoma cell.

### Inhibiting Primary Tumor Growth

The present inventors have shown that IL-17 can promote primary tumor growth and that treatment with antagonists of IL-17 inhibits growth of primary tumors.

Accordingly, in one aspect, it is disclosed a method of inhibiting primary tumor growth in a subject, the method comprising contacting the primary tumor with an IL-17 antagonist in an amount effective to inhibit primary tumor growth. In one embedment, the method is directed to treating, preventing, or inhibiting primary tumor growth in a subject, wherein the tumor growth is associated with increased expression of IL-17 and/or AID by cancer cells or cells at increased risk for becoming cancerous (e.g., cells that express IL-17 and/or AID in an autocrine manner), the method comprising administering to the cells an effective amount of an IL-17 antagonist. In one embodiment, the method further comprises measuring the amount of IL-17 in a sample from the subject; comparing the measured amount of IL-17 to a reference amount of IL-17 to determine if the subject is likely to respond to inhibition of primary tumor growth with an anti-IL17 antagonist. In a particular embodiment, an amount of IL-17 that is greater than the reference amount indicates that the subject is likely to respond. In another embodiment, the method further comprises measuring the amount of AID and/or TWIST-1 in a sample from the subject and comparing the measured amount of AID and/or TWIST-1, respectively, to a reference amount of AID and/or TWIST-1 to determine if the the subject is likely to respond to inhibition of primary tumor growth with an IL-17 antagonist, wherein an amount of AID,and/or TWIST-1 that is greater than the reference amount indicates that the subject is likely to respond. In a specific embodiment, the IL-17 antagonist is selected from the group consisting of a small molecule, an antigen binding molecule, a nucleic acid antagonist, and a protein antagonist.

In a particular embodiment, the primary tumor is from a tissue or organ selected from the group consisting of breast, bladder, liver, colon, ovary, lung, esophageal, head and neck, B lymphocyte, and skin. In a more specific embodiment, the tumor is a breast tumor. In another specific embodiment, the tumor is a liver tumor. In another specific embodiment, the tumor is a B cell lymphoma. Non-limiting examples of types of cancer a provided elsewhere herein, and the tumor could be derived from any of these cancers.

### Preventing Tumor Metastases and Preventing and/or Reverting Epithelial to Mesenchymal Transition

The present inventors have found that IL-17 can induce an epithelial-to-mesenchymal transition of cells that leads to aggressiveness and invasiveness associated with cancer metastases. In hepatocytes, IL-17 can also cooperate with HCV to induce this transition. Accordingly, in one aspect, it is disclosed a method for treating, preventing, or inhibiting metastases associated with increased expression of IL-17 and/or AID by cancer cells, cells at increased risk for becoming cancerous, or cancer cells at increased risk for metastasizing, the method comprising administering to the cells an effective amount of an IL-17 antagonist. In one embodiment, the cells are cancer stem cells.

Accordingly, in one aspect, it is disclosed a method of preventing tumor metastases in a subject having a cell proliferation disorder, the method comprising: (a) measuring the amount of IL-17 in a sample from the subject; (b) comparing the amount of IL-17 in the sample to a reference amount of IL-17 to identify if the subject is likely to respond to prevention of the tumor metastases with an IL-17 antagonist; and (c) administering an amount of an IL-17 antagonist effective to prevent tumor metastases in said subject. In a particular embodiment, an amount of IL-17 that is greater than said reference amount indicates that the subject is likely to respond. In another embodiment, the method further comprises determining if TWIST-1 expression is increased in a sample from the subject compared to a reference level of TWIST-1 expression to identify if the subject is at increased risk for tumor metastases. In a particular embodiment, TWIST-1 expression indicates an increased risk for tumor metastases in the subject. In another embodiment, the method further comprises determining if the sample comprises cells with the characteristics of mesenchymal cells. This determination can be made by methods described elsewhere herein and know in the art. In one non-limiting example, mesenchymal and epithelial cell characteristics can be determined by detecting expression of certain proteins such as E-cadherin, α-catenin, and occludin (epithelial cell markers), and/or N-cadherin and vimentin (mesenchymal cell markers). In a specific embodiment, the IL-17 antagonist is selected from the group consisting of a small molecule, an antigen binding molecule, a nucleic acid antagonist, and a protein antagonist.

In a particular embodiment, the metastases originate from a primary tumor that is from a tissue or organ selected from the group consisting of breast, bladder, liver, colon, ovary, lung, kidney, cervix, stomach, intestine, prostate, esophageal, head and neck, connective tissue, and skin. In a more particular embodiment, the metastases originate from a primary tumor that is from a tissue or organ selected from the group consisting of breast, colon, lung, ovary, esophagus, head and neck, or skin (e.g., melanoma). In a more specific embodiment, the mestastasis is from a breast tumor. In another specific embodiment, the mestastasis is from a liver tumor. Non-limiting examples of types of cancer a provided elsewhere herein, and metastases could be derived from any of these cancers that has metastatic potential.

In another aspect, it is disclosed a method of preventing or reverting an epithelial to mesenchymal transition (EMT) of one or more cells of a subject, the method comprising contacting the one or more cells with an IL-17 antagonist in an amount effective to prevent or revert the EMT. In one embodiment, the cells express and/or produce IL-17 in an autocrine manner. In one embodiment, the method further comprises: measuring the amount of IL-17 in a sample from the subject and comparing the measured amount of IL-17 to a reference amount of IL-17 to determine if the one or more cells is likely to respond to prevention or reversion of an EMT with an anti-IL17 antagonist. In a particular embodiment, an amount of IL-17 that is greater than the reference amount indicates that the subject is likely to respond. In another embodiment, the method further comprises measuring the amount of AID and/or TWIST-1 in a sample from the subject and comparing the measured amount of AID and/or TWIST-1, respectively, to a reference amount of AID and/or TWIST-1 to determine if the one or more cells is likely to respond to prevention or reversion of an EMT with an IL-17 antagonist, wherein an amount of AID,and/or TWIST-1 that is greater than the reference amount indicates that the one or more cells is likely to respond. In a specific embodiment, the IL-17 antagonist is selected from the group consisting of a small molecule, an antigen binding molecule, a nucleic acid antagonist, and a protein antagonist.

According to the present disclosure, the mammalian cell may be *in vitro, in vivo, in situ,* or *ex vivo.* In a specific embodiment, the cell is *in vivo* in a human subject. In another specific embodiment, the mammalian cell is a human cell. In a particular embodiment, the mammalian cell is selected from the non-limiting group consisting of a mammary (breast) cell, a hepatocyte (liver cell), an ovarian cell, a cervical cell, an endothelial cell, a blood cell (for example a B lymphocyte), a hematopoietic cell, a prostate cell, a skin cell, a stomach cell, an intestinal cell, an epithelial cell, a pancreatic cell, a renal (kidney) cell, a fibroblast, a colorectal cell, and a bone cell. Other non-limiting examples of cells, encompassed by the method disclosed therein are provided in other sections herein. In a specific embodiment, the mammalian cell is a mammary cell, more specifically a human mammary cell. In another specific embodiment, the mammalian cell is a hepatocyte, more specifically, a human hepatocyte. In a more specific embodiment, the hepatocyte is infected with a virus, more specifically, a hepatitis virus (e.g., HBV or HCV). In one embodiment, the mammalian cell is a cancer cell. Non-limiting examples of types of cancer a provided elsewhere herein, and the cancerous cell could be derived from any of these cancers. In a specific embodiment, the cancer cell is selected a breast cancer cell or a hepatocellullar carcinoma cell.

In a specific embodiment, the method comprises preventing or reverting an IL-17 induced EMT of a hepatocyte, more specifically, a human hepatocyte, the method comprising contacting the hepatocyte with an IL-17 antagonist in an amount effective to prevent or revert the IL-17-induced EMT of the hepatocyte. In a specific embodiment, the hepatocyte is infected with a virus. In a more specific embodiment, the virus is a hepatitis virus. In a more specific embodiment, the hepatitis virus is selected from the group consisting of HBV and HCV. In one embodiment, the hepatocyte is a hepatocelluar carcinoma cell. In a particular embodiment, the hepatocyte is *in vivo* in a human subject. In a specific embodiment, the IL-17 antagonist is selected from the group consisting of a small molecule, an antigen binding molecule, a nucleic acid antagonist, and a protein antagonist.

In one embodiment, the method comprises preventing or reverting an IL-17-induced EMT of a mammary cell, more specifically, a human mammary cell, the method comprising contacting the mammary cell with an IL-17 antagonist in an amount effective to prevent or revert the IL-17-induced EMT of the mammary cell. In a particular embodiment, the mammary is *in vivo* in a human subject. In a specific embodiment, the IL-17 antagonist is selected from the group consisting of a small molecule, an antigen binding molecule, a nucleic acid antagonist, and a protein antagonist.

EMT in hepatocytes is also associated with liver fibrosis. IL-17 can induce EMT and therefore lead to fibrosis of the liver. HCV can cooperate with IL-17 to cause EMT in hepatocytes and lead to fibrosis. Accordingly, in another aspect, it is disclosed a method of treating and/or preventing fibrosis of the liver of a subject, the method comprising administering to the subject an IL-17 antagonist in an amount sufficient to treat or prevent fibrosis of the liver. In one embodiment, the method further comprises: measuring the amount of IL-17 in a sample from the subject and comparing the measured amount of IL-17 to a reference amount of IL-17 to determine if the one or more cells is likely to respond to prevention or treatment of liver fibrosis with an anti-IL17 antagonist. In a particular embodiment, an amount of IL-17 that is greater than the reference amount indicates that the subject is likely to respond. In another embodiment, the method further comprises measuring the amount of TWIST-1 in a sample from the subject and comparing the measured amount to a reference amount of TWIST-1 to determine if the subject is likely to respond to prevention or treatment of liver fibrosis with an IL-17 antagonist, wherein an amount of TWIST-1 that is greater than the reference amount indicates that the subject is likely to respond. In a specific embodiment, the IL-17 antagonist is selected from the group consisting of a small molecule, an antigen binding molecule, a nucleic acid antagonist, and a protein antagonist.

### IL-17 Antagonists, BAFF Antagonists, IL-6 Antagonists, and IL-10 Antagonists

IL-17 antagonists as disclosed therein include any agent, molecule, or substance that inhibits activity of IL-17. For example, IL-17 antagonists include agents, molecules, or substances that block or inhibit binding of IL-17 to its receptor, or that block or inhibit homodimer or heterodimer formation of IL-17 molecules with, e.g., other IL-17 molecules or other IL-17 forms such as IL-17F. Non-limiting examples of IL-17 antagonists include antibodies against IL-17 or its receptor (including neutralizing antibodies), small molecules that inhibit IL-17 activity, decoy receptors, protein antagonists (including fusion proteins and glycoproteins), and nucleic acid inhibitors of IL-17.

BAFF antagonists as disclosed therein include any agent, molecule, or substance that inhibits activity of BAFF. For example, BAFF antagonists include agents, molecules, or substances that block or inhibit binding BAFF to its receptor or other molecules involved in BAFF signaling and/or activity. Non-limiting examples of BAFF antagonists include antibodies against BAFF or its receptor (including neutralizing antibodies), small molecules that inhibit BAFF activity, decoy receptors, protein antagonists (including fusion proteins and glycoproteins), and nucleic acid inhibitors of BAFF.

IL-6 antagonists as disclosed therein include any agent, molecule, or substance that inhibits activity of IL-6. For example, IL-6 antagonists include agents, molecules, or substances that block or inhibit binding of IL-6 to its receptor or other molecules involved in IL-6 signaling and/or activity. Non-limiting examples of IL-6 antagonists include antibodies against IL-6 or its receptor (including neutralizing antibodies), small molecules that inhibit IL-6 activity, decoy receptors, protein antagonists (including fusion proteins and glycoproteins), and nucleic acid inhibitors of IL-6.

IL-10 antagonists as disclosed therein include any agent, molecule, or substance that inhibits activity of IL-10. For example, IL-10 antagonists include agents, molecules, or substances that block or inhibit binding of IL-10 to its receptor or other molecules involved in IL-6 signaling and/or activity. Non-limiting examples of IL-6 antagonists include antibodies against IL-10 or its receptor (including neutralizing antibodies), small molecules that inhibit IL-10 activity, decoy receptors, protein antagonists (including fusion proteins and glycoproteins), and nucleic acid inhibitors of IL-10.

In a particular embodiment, the IL-17 antagonist and/or the BAFF antagonist and/or the IL-6 antagonist and/or the IL-10 antagonist is an antibody. Antibodies useful in the invention include, but are not limited to, polyclonal, monoclonal, multispecific, human, humanized or chimeric antibodies, single chain antibodies, scFv fragments, Fab fragments, F(ab')2 fragments, fragments produced by an Fab expression library, domain-deleted antibodies (including, e.g., CH2 domain-deleted antibodies), anti-idiotypic (anti-Id) antibodies (including, e.g., anti-Id antibodies to antibodies of the invention), and antigen binding fragments of any of the above. Antibodies useful in the invention also include, but are not limited to, engineered forms of antibodies and antibody fragments such as diabodies, triabodies, tetrabodies, and higher multimers of scFvs, as well as minibodies, such as two scFv fragments joined by two constant (C) domains. See, *e.g.,* Hudson, P.J. and Couriau, C., Nature Med. 9: 129-134 (2003); U.S. Publication No. 20030148409; U.S. Patent No. 5,837,242.

Antibodies useful in the invention may be from any animal origin including birds and mammals. Preferably, the antibodies are human, murine (*e.g.,* mouse and/or rat), donkey, ship rabbit, goat, guinea pig, camel, horse, or chicken.

The antibodies useful in the invention may be monoclonal antibodies. Monoclonal antibodies can be prepared using a wide variety of techniques known in the art including the use of hybridoma, recombinant, and phage display technologies, or a combination thereof. For example, monoclonal antibodies useful in the invention can be prepared using hybridoma methods, such as those described by Kohler and Milstein, Nature, 256:495, 1975; Harlow et al., ANTIBODIES: A LABORATORY MANUAL, (Cold Spring Harbor Laboratory Press, 2nd ed. 1988); Hammerling, et al., in: MONOCLONAL ANTIBODIES AND T-CELL HYBRIDOMAS 563-681 (Elsevier, N.Y., 1981), or other methods known in the art. In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro* by known methods.

Monoclonal antibodies may also be made by recombinant DNA methods, such as those described in U.S. Pat. No. 4,816,567 (Cabilly et al.). DNA encoding the monoclonal antibodies of the invention can be readily isolated and sequenced using conventional procedures (*e.g.,* by using oligonucleotide probes that are capable of binding specifically to genes encoding the heavy and light chains of murine antibodies). Libraries of antibodies or active antibody fragments can also be generated and screened using phage display techniques, *e.g.,* as described in U.S. Pat. No. 5,804,440 to Burton et al. and U.S. Pat. No. 6,096,441 to Barbas et al.*.*

*In vitro* methods can also be used for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994 and U.S. Pat. No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, *etc.* In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr Opin Biotechnol 3:348-354, 1992). Examples of techniques that can be used to produce single-chain Fvs and antibodies, as well as diabodies, triabodies, and tetrabodies, include those described in U.S. Pat. Nos. 4,946,778 and 5,258,498; Huston et al., Methods in Enzymology 203:46-88 (1991); Shu et al., PNAS 90:7995-7999 (1993); Skerra et al., Science 240:1038-1040 (1988); U.S. Application Publication No. 20020018749 and U.S. Pat. No. 5,837,242.

Antibodies useful in the invention can, in turn, be used to generate anti-idiotype antibodies that "mimic" polypeptides of the invention using techniques well known to those skilled in the art. (*See, e.g.,* Greenspan & Bona, FASEB J. 7(5):437-444; (1989) and Nissinoff, J. Immunol. 147(8):2429-2438 (1991)). For example, antibodies that bind to and competitively inhibit polypeptide multimerization and/or binding of a polypeptide of the invention to a ligand can be used to generate anti-idiotypes that "mimic" the polypeptide multimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide of the invention and/or to bind its ligands/receptors, and thereby block its biological activity.

Antibodies useful in the invention also include a human antibody and/or a humanized antibody. Many non-human antibodies (*e.g.,* those derived from mice, rats, or rabbits) are naturally antigenic in humans, and thus can give rise to undesirable immune responses when administered to humans. Therefore, the use of human or humanized antibodies in the methods of the invention serves to lessen the chance that an antibody administered to a human will evoke an undesirable immune response.

The human antibodies useful in the invention can be prepared using any technique. Examples of techniques for human monoclonal antibody production include those described by Cole et al. (Monoclonal Antibodies and Cancer Therapy, Alan R., Ed. Liss, p. 77, 1985) and by Boerner et al. (J Immunol, 147(1):86-95, 1991). Human antibodies of the invention (and fragments thereof) can also be produced using phage display libraries (Hoogenboom et al., J Mol Biol, 227:381, 1991; Marks et al., J Mol Biol, 222:581, 1991).

For example, the antibodies useful in the invention can also be generated using various phage display methods known in the art. In phage display methods, functional antibody domains are displayed on the surface of phage particles which carry the polynucleotide sequences encoding them. In a particular embodiment, such phage can be utilized to display antigen binding domains expressed from a repertoire or combinatorial antibody library. Phage expressing an antigen binding domain that binds the antigen of interest can be selected or identified with antigen, *e.g.,* using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods that can be used to make the antibodies usefule in the invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.,* as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); Sawai et al., AJRI 34:26-34 (1995); and Better et al., Science 240:1041-1043 (1988).

Human antibodies useful in the invention can also be obtained from transgenic animals. For example, transgenic, mutant mice that are capable of producing a full repertoire of human antibodies, in response to immunization, have been described (see, *e.g.,* Jakobovits et al., Proc. Natl. Acad. Sci. USA, 90:2551-255, 1993; Jakobovits et al., Nature, 362:255-258, 1993; Bruggermann et al., Year in Immunol. 7:33, 1993). Specifically, the homozygous deletion of the antibody heavy chain joining region (J*(H)*) gene in these chimeric and germ-line mutant mice results in complete inhibition of endogenous antibody production, and the successful transfer of the human germ-line antibody gene array into such germ-line mutant mice results in the production of human antibodies upon antigen challenge. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, *e.g.,* all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, Int. Rev. Immunol. 13:65-93 (1995). For a detailed discussion of this technology for producing human antibodies and human monoclonal antibodies and protocols for producing such antibodies, *see, e.g.,* PCT publications WO 98/24893; WO 92/01047; WO 96/34096; WO 96/33735; European Patent No. 0 598 877; U.S. Pat. Nos. 5,413,923; 5,625,126; 5,633,425; 5,569,825; 5,661,016; 5,545,806; 5,814,318; 5,885,793; 5,916,771; and 5,939,598. In addition, companies such as Abgenix, Inc. (Freemont, Calif.) and Genpharm (San Jose, Calif.) can be engaged to provide human antibodies directed against a selected antigen using technology similar to that described above.

Completely human antibodies that recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12:899-903 (1988)).

In another embodiment, the antibody antagonist may be a humanized antibody. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fc, Fv, Fab, Fab', or other antigen-binding portion of an antibody) which contains a portion of an antigen binding site from a non-human (donor) antibody integrated into the framework of a human (recipient) antibody.

To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (*e.g.,* a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525, 1986, Reichmann et al., Nature, 332:323-327, 1988, and Presta, Curr Opin Struct Biol, 2:593-596, 1992). However, fragments that do not contain a portion of the constant region are also envisioned for use in the invention.

Methods for humanizing non-human antibodies are well known in the art. For example, humanized antibodies can be generated according to the methods of Winter and co-workers (Jones et al., Nature, 321:522-525, 1986, Riechmann et al., Nature, 332:323-327, 1988, Verhoeyen et al., Science, 239:1534-1536, 1988), by substituting rodent CDRs or CDR sequences for the corresponding sequences of a human antibody. Methods that can be used to produce humanized antibodies are also described in U.S. Pat. No. 4,816,567 (Cabilly et al.), U.S. Pat. No. 5,565,332 (Hoogenboom et al.), U.S. Pat. No. 5,721,367 (Kay et al.), U.S. Pat. No. 5,837,243 (Deo et al.), U.S. Pat. No. 5, 939,598 (Kucherlapati et al.), U.S. Pat. No. 6,130,364 (Jakobovits et al.), and U.S. Pat. No. 6,180,377 (Morgan et al.).

Various IL-17 antibodies have been developed and used specifically for autoimmune disorders. Such antibodies include a human anti-IL-17 monoclonal antibody, AIN 457 (Novartis, Basel, Switzerland). This antibody is currently in or will be in clinical trials for rheumatoid arthritis, Crohn's disease, psoriasis, uveitis, ankylosing spondylarthopathy, multiple sclerosis, and ozone-induced airway neutrophilia. AIN 457 was shown to be safe and effective in 46% of antibody-treated patients with Rheumatoid Arthritis compared to 27% of patients who received placebo. Durez et al., Communication EULAR 2009-RA. Also, a humanized monoclonal antibody against IL-17A, LY2439821 (Eli Lilly, Indianapolis, IN, USA), was tested in clinical trials for safety, tolerability and evidence of efficacy of intravenous LY2439821 in patients with rheumatoid arthritis. Efficacy results showed that certain doses were statistically significantly more effective compared to placebo in treating RA patients. *See* Genoevse et al., Communication EULAR 2009-RA; Genoevse et al., Arthritis & Rheumatism, 62: 929-39, (2010). Other anti-IL17 antibodies include eBio64CAP17 mouse anti-human neutralizing antibody (eBioscience) or derivatives thereof. Other examples of anti-IL-17 antibodies are set forth in US2009/0175881A1, US2008/0269467A1, WO2008/001063A1, and WO2007/117749A1. Such antibodies are contemplated for use in the invention, as well as IL-17 antibodies that can be made by methods described herein and known in the art.

Likewise, anti-BAFF antibodies have been developed for autoimmune disorders. For example, the humanized anti-BAFF antibody, belimumab (Glaxo-SimithKline, United Kingdom), is currently in or will be in clinical trials for systemic lupus erythematosus (SLE), rheumatoid arthritis, kidney transplant, and Sjogren syndrome. Belimumab was found to be biologically active and well tolerated in SLE patients. *See* Wallace et al., Arthritis & Rheumatism 61:1168-1178 (2009); *see also,* Jacobi et al., Arthritis & Rheumatism 62: 201-210 (2010). Other BAFF antibodies include mouse anti-human BAFF (also known as BLys, CD257) 1D6 monoclonal antibody (eBiosience) or derivatives thereof. Such antibodies are contemplated for use in the invention, as well as BAFF antibodies that can be made by methods described herein and known in the art.

Another antagonist (inhibitor) of BAFF is a fusion protein known as Atacicept (Merck Serono), which contains the extracellular BAFF/APRIL-binding domain of the TACI. This BAFF inhibitor is part of a clinical trial program for SLE, multiple sclerosis, RA, and optic neuritis. Atacicept was found to inhibit the bioactivity of BAFF, APRIL and BAFF/APRIL heterotrimers, Dillon et al., Communication (EULAR 2009), and was found to be generally well tolerated systemically and locally in SLE patients and displayed biological activity in reducing B-cells and Ig levels, Pena-Rossi et al., Lupus 18:547-555 (2009). Another protein antagonist of BAFF is the Briobacept BR3-Fc BAFF inhibitor (Biogen, Genentech). Briobacept is a recombinant glycoprotein with two BAFF receptors linked to the Fc domain of human IgG1. It was shown to be safe and well-tolerated in patients with RA. Shaw et al., Communication EULAR 2009. Such protein antagonists are contemplated for use in the present disclosure.

Nucleic acid antagonists and/or gene silencers such as siRNA and shRNA, can be developed and administered by methods known in the art. For example lentiviral vectors comprising nucleic acid sequences that target IL-17A or BAFF are contemplated for use in the present disclosure. Protocols for transduction with shRNA lentiviral particles are known in the art (*see, e.g.,* sample protocol from Santa Cruz Biotechnology, Inc., at *scbt.com*/*protocol_shrna_lentiviralparticles_transduction.html*).

### Detection Methods and Assays

The methods as disclosed therein provide for detecting and/or measuring amounts of a factor, molecule, etc., e.g., IL-17, AID, BAFF, TWIST-1, and p53. Such detections and/or measurements may be performed by methods well known in the art, for example, by nucleic acid detection methods, ELISA, Western blotting, immunohistochemistry, immunocytochemistry, immunoprecipitation, affinity chromatography, or cell based assays.

To determine the (increased or decreased) expression levels of genes in the practice of the present disclosure, any method known in the art may be utilized. In one preferred embodiment, expression based on detection of RNA which hybridizes to the genes identified and disclosed herein is used. This is readily performed by any RNA detection or amplification+detection method known or recognized as equivalent in the art such as, but not limited to, reverse transcription-PCR, and methods to detect the presence, or absence, of RNA stabilizing or destabilizing sequences.

Alternatively, expression based on detection of DNA status may be used. Detection of the DNA of an identified gene as methylated or deleted may be used for genes that have decreased expression. This may be readily performed by PCR based methods known in the art, including, but not limited to, Q-PCR. Conversely, detection of the DNA of an identified gene as amplified may be used for genes that have increased expression. This may be readily performed by PCR based, fluorescent in situ hybridization (FISH) and chromosome in situ hybridization (CISH) methods known in the art.

Expression based on detection of a presence, increase, or decrease in protein levels or activity may also be used. Detection may be performed by any immunohistochemistry- (IHC) based, blood-based (especially for secreted proteins), antibody- (including autoantibodies against the protein) based, exfoliate cell (from the cancer) based, mass spectroscopy-based, and image- (including used of labeled ligand) based method known in the art and recognized as appropriate for the detection of the protein. Antibody- and image- based methods are additionally useful for the localization of tumors after determination of cancer by use of cells obtained by a non-invasive procedure (such as ductal lavage or fine needle aspiration), where the source of the cancerous cells is not known. A labeled antibody or ligand may be used to localize the carcinoma(s) within a patient.

Antibodies can be used to assay levels of polypeptides encoded by polynucleotides as disclosed thereinin a biological sample using classical immunohistological methods known to those of skill in the art (*e.g., see* Jalkanen, et al., J. Cell. Biol. 101:976-985 (1985); Jalkanen, et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (¹³¹I, ¹²⁵I, ¹²³I, ¹²¹I, carbon (¹⁴C), sulfur (³⁵S), tritium (³H), indium (¹¹⁵mIn, ¹¹³In, ¹¹²In, ¹¹¹In), and technetium (⁹⁹Tc, ⁹⁹mTc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ⁸⁸Re, ¹⁴²Pr, ¹⁰⁵Rh, ⁹⁷Ru; luminescent labels, such as luminol; and fluorescent labels, such as fluorescein and rhodamine, and biotin.

In addition to assaying levels of polypeptide as disclosed therein in a biological sample, proteins can also be detected *in vivo* by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, ¹³¹I, 1¹²In, ⁹⁹mTc, (¹³¹I, ¹²⁵I, ¹²³I, ¹²¹I), carbon (¹⁴C), sulfur (35S), tritium (³H), indium (¹¹⁵mIn, ¹¹³mIn, ¹¹²In, ¹¹¹In), and technetium (⁹⁹Tc, ⁹⁹mTc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F, ¹⁵³Sm, ¹⁷⁷Lu, ⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru), a radio-opaque substance, or a material detectable by nuclear magnetic resonance, is introduced (for example, parenterally, subcutaneously or intraperitoneally) into the mammal to be examined for immune system disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody or antibody fragment will then preferentially accumulate at the location of cells which express the polypeptide encoded by a polynucleotide as disclosed therein. In vivo tumor imaging is described in S.W. Burchiel et al., "Immunopharmacokinetics of Radiolabeled Antibodies and Their Fragments" (Chapter 13 in TUMOR IMAGING: THE RADIOCHEMICAL DETECTION OF CANCER, S. W. Burchiel and B. A. Rhodes, eds., Masson Publishing Inc. (1982)).

Techniques known in the art may be applied to label polypeptides as disclosed therein(including antibodies). Such techniques include, but are not limited to, the use of bifunctional conjugating agents (*see e.g.,* U.S. Pat. Nos. 5,756,065; 5,714,631; 5,696,239; 5,652,361; 5,505,931; 5,489,425; 5,435,990; 5,428,139; 5,342,604; 5,274,119; 4,994,560; and 5,808,003).

### Kits and Articles of Manufacture

The materials and methods of the present disclosure are ideally suited for preparation of kits produced in accordance with well known procedures. The present application thus discloses kits comprising agents (like the antagonists, e.g., polynucleotides and/or antibodies, described herein as non-limiting examples) for the detection of expression of IL-17, AID, BAFF, TWIST, p53, p21, IL-6, IL-10, and other relevant factors. Such kits, optionally comprising the agent with an identifying description or label or instructions relating to their use in the methods as disclosed therein, are provided. Such a kit may comprise containers, each with one or more of the various reagents (typically in concentrated form) used in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more primer complexes as disclosed therein (e.g., appropriate length poly(T) or random primers linked to a promoter reactive with the RNA polymerase), antibodies, protein detection reagents, or other labeled detection and/or quantification reagents. A set of instructions will also typically be included.

The present application also discloses a pharmaceutical pack or kit comprising one or more containers filled with one or more of the ingredients of the pharmaceutical compositions as disclosed therein. For example, the pharmaceutical pack or kit may contain a preparation comprising an IL-17 antagonist and a therapeutic agent (e.g., a chemotherapeutic agent) and/or a BAFF antagonist. In some embodiments, the kit may further comprise an IL-6 antagonist and/or an IL-10 antagonist. In some embodiments, the compounds are in the same container. In other embodiments, the compounds are in separate containers. In some embodiments, the therapeutic agent is in the same container as the IL-17 antagonist preparation. In other embodiments, the therapeutic agent is in a separate container. Optionally associated with such container(s) can be a notice in the form prescribed by a governmental agency regulating the manufacture, use or sale of pharmaceuticals or biological products, which notice reflects approval by the agency of manufacture, use or sale for human administration.

### Pharmaceutical Compositions and Methods of Administration

In accordance with the invention, antagonists and compositions provided may be administered to a subject, e.g., a human patient. The actual amount administered, and rate and time-course of administration, will depend on the nature and severity of what is being treated.

Methods of preparing and administering one or more of the antagonistsas disclosed therein, or antigen-binding fragments, variants, or derivatives thereof to a cell or to a subject in need thereof are well known to or are readily determined by those skilled in the art. The route of administration of the antagonists may be, for example, oral, parenteral, by inhalation or topical. The term parenteral as used herein includes, *e.g.,* intravenous, intraarterial, intraperitoneal, intramuscular, subcutaneous, rectal or vaginal administration. While all these forms of administration are clearly contemplated as being within the scope of the present disclosure, a form for administration would be a solution for injection, in particular for intravenous or intraarterial injection or drip. Usually, a suitable pharmaceutical composition for injection may comprise a buffer (*e.g.* acetate, phosphate or citrate buffer), a surfactant (*e.g.* polysorbate), optionally a stabilizer agent (*e.g.* human albumin), etc. However, in other methods compatible with the teachings herein, the antagonists as disclosed therein can be delivered directly to the site of the adverse cellular population (e.g., the abnormal cells, precancerous cells, cancer cells, etc.), thereby increasing the exposure of the diseased tissue to the therapeutic agent.

The present application also discloses pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

In a particular embodiment, the composition is formulated in accordance with routine procedures as a pharmaceutical composition adapted for intravenous administration to humans. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In one aspect, the present disclosure is directed to an IL-17 antagonist for the prevention or reduction of IL-17-induced DNA damage in a mammalian cell. In a specific embodiment, the IL-17 antagonist is selected from the group consisting of a small molecule, an IL-17-specific antigen binding molecule a nucleic acid antagonist and a protein antagonist. In a more specific embodiment, the antigen binding molecule is a selected from the group consisting of an antibody and an antigen binding antibody fragment. In one embodiment, the IL-17-induced DNA damage is a result of upregulation of AID. In another embodiment, the IL-17-induced DNA damage is a result of upregulation of TWIST-1. In another embodiment, the IL-17-induced DNA damage is a result of inhibition of the p53 tumor suppressor pathway. In one embodiment, the cell is a human cell, more specifically, a mammary cell, a hepatocyte, or a B cell.

In another aspect, the present disclosure is directed to an IL-17 antagonist for the prevention or reversion of an epithelial to mesenchymal transition (EMT) of a cell. In one embodiment, the epithelial cell is a breast cell, more specifically, a breast cancer cell. In another embodiment, the epithelial cell is a hepatocyte, more specifically, a hepatocellular carcinoma cell. In a more specific embodiment, the hepatocyte is infected with a virus. In one embodiment, the IL-17 antagonist prevents a virus-induced transformation of the hepatocyte. In a specific embodiment, the virus is a hepatitis virus, more specifically, HBV and HCV. In another embodiment, the IL-17 antagonist further comprising a BAFF antagonist. In a specific embodiment, the BAFF antagonist is selected from the group consisting of a small molecule, a BAFF-specific antigen binding molecule, a nucleic acid antagonist, and a protein antagonist. In one embodiment, the cell is a B-cell, more specifically, a cancerous B-cell, and even more specifically, a non-Hodgkins lymphoma cell. In one embodiment, the B-cell is *in vivo* in a human patient. In a more specific embodiment, the patient is at increased risk for a B-cell cancer. In an even more specific embodiment, the patient suffers from an autoimmune disorder, more specifically, systemic lupus erythematosus or rheumatoid arthritis.

The compounds of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment, inhibition and prevention of a disease or disorder associated with IL-17 can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from *in vitro* or animal model test systems.

For antagonists that are antibodies, the dosage administered to a patient is typically about 0.1 mg/kg to about 100 mg/kg of the patient's body weight. Preferably, the dosage administered to a patient is between about 0.1 mg/kg and about 20 mg/kg of the patient's body weight, more preferably about 1 mg/kg to about 10 mg/kg of the patient's body weight. In some embodiments the antibodies are administered at a total dose of about 10 mg/kg to about 50mg/kg of the patient's body weight. In another embodiment the antibodies are administered at a total dose of about 20 mg/kg to about 40 mg/kg. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration of the antibodies by modifications such as, for example, lipidation.

IL-17 antagonists and, optionally additional agents such as anti-BAFF antagonists anti-IL6 antagonists, anti-IL-10 antagonists, and/or a therapeutic agent, may be administered in a pharmaceutically effective amount for the *in vivo* treatment of a cell proliferation disorder or other IL-17-related disease. In this regard, it will be appreciated that the IL-17 antagonists and any additional agents will be formulated so as to facilitate administration and promote stability of the active agent(s). Preferably, pharmaceutical compositions in accordance with the present invention comprise a pharmaceutically acceptable, non-toxic, sterile carrier such as physiological saline, non-toxic buffers, preservatives and the like.

In one embodiment, the entire IL-17 antagonist dose is provided in a single bolus. Alternatively, the dose can be provided by multiple administrations, such as an extended infusion method or by repeated injections administered over a span of hours or days, for example, a span of about 2 to about 4 days. Likewise, additional agents can be administered in the a single dose or by mutiple administrations.

In some embodiments, the IL-17 antagonist and the BAFF antagonist and/or the IL-6 antagonist, the IL-10 antagonist, and/or the therapeutic agent are administered together in the same pharmaceutical preparation. In other embodiments the compounds are administered as separate pharmaceutical preparations, either concurrently or sequentially.

Various delivery systems are known and can be used to administer a compound according to the invention, e.g., encapsulation in liposomes, microparticles, microcapsules, recombinant cells capable of expressing the compound, receptor-mediated endocytosis (*see, e.g.,* Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the invention, care must be taken to use materials to which the protein does not absorb.

In another embodiment, the compound or composition can be delivered in a vesicle, in particular a liposome (*see* Langer, Science 249:1527-1533 (1990); Treat et al., in LIPOSOMES IN THE THERAPY OF INFECTIOUS DISEASE AND CANCER, Lopez-Berestein and Fidler (eds.), Liss, New York, pp. 353-365 (1989); Lopez-Berestein, ibid., pp. 317-327; *see generally* ibid.)

In yet another embodiment, the compound or composition can be delivered in a controlled release system. In one embodiment, a pump may be used (*see* Langer, *supra;* Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (*see* MEDICAL APPLICATIONS OF CONTROLLED RELEASE, Langer and Wise (eds.), CRC Pres., Boca Raton, Florida (1974); CONTROLLED DRUG BIOAVAILABILITY, DRUG PRODUCT DESIGN AND PERFORMANCE, Smolen and Ball (eds.), Wiley, New York (1984); Ranger and Peppas, J., Macromol. Sci. Rev. Macromol. Chem. 23:61 (1983); *see also* Levy et al., Science 228:190 (1985); During et al., Ann. Neurol. 25:351 (1989); Howard et al., J.Neurosurg. 71:105 (1989)). In yet another embodiment, a controlled release system can be placed in proximity of the therapeutic target, i.e., the brain, thus requiring only a fraction of the systemic dose (*see, e.g.,* Goodson, in MEDICAL APPLICATIONS OF CONTROLLED RELEASE, supra, vol. 2, pp. 115-138 (1984)). Other controlled release systems are discussed in the review by Langer (Science 249:1527-1533 (1990)).

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis et al. U.S. Pat. No: 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Mayer and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Maryland (1989).

General principles of antibody engineering are set forth in Antibody Engineering, 2nd edition, C.A.K. Borrebaeck, Ed., Oxford Univ. Press (1995). General principles of protein engineering are set forth in Protein Engineering, A Practical Approach, Rickwood, D., et al., Eds., IRL Press at Oxford Univ. Press, Oxford, Eng. (1995). General principles of antibodies and antibody-hapten binding are set forth in: Nisonoff, A., Molecular Immunology, 2nd ed., Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (eds), Basic and Clinical -Immunology (8th ed.), Appleton & Lange, Norwalk, CT (1994) and Mishell and Shiigi (eds), Selected Methods in Cellular Immunology, W.H. Freeman and Co., New York (1980).

Standard reference works setting forth general principles of immunology include Current Protocols in Immunology, John Wiley & Sons, New York; Klein, J., Immunology: The Science of Self-Nonself Discrimination, John Wiley & Sons, New York (1982); Kennett, R., et al., eds., Monoclonal Antibodies, Hybridoma: A New Dimension in Biological Analyses, Plenum Press, New York (1980); Campbell, A., "Monoclonal Antibody Technology" in Burden, R., et al., eds., Laboratory Techniques in Biochemistry and Molecular Biology, Vol. 13, Elsevere, Amsterdam (1984), Kuby Immunnology 4th ed. Ed. Richard A. Goldsby, Thomas J. Kindt and Barbara A. Osborne, H. Freemand & Co. (2000); Roitt, I., Brostoff, J. and Male D., Immunology 6th ed. London: Mosby (2001); Abbas A., Abul, A. and Lichtman, A., Cellular and Molecular Immunology Ed. 5, Elsevier Health Sciences Division (2005); Kontermann and Dubel, Antibody Engineering, Springer Verlan (2001); Sambrook and Russell, Molecular Cloning: A Laboratory Manual. Cold Spring Harbor Press (2001); Lewin, Genes VIII, Prentice Hall (2003); Harlow and Lane, Antibodies: A Laboratory Manual, Cold Spring Harbor Press (1988); Dieffenbach and Dveksler, PCR Primer Cold Spring Harbor Press (2003).

Having now generally described the invention, the same will be more readily understood through reference to the following examples which are provided by way of illustration, and are not intended to be limiting of the present invention, unless specified.

### EXAMPLES

### EXAMPLE 1: Experimental Procedures

The following materials and methods were used in the Examples described herein.

### Cell Culture and Reagents

Blood samples from healthy adult donors were obtained from the Etablissement Français du Sang (EFS, Lyon, France). Peripheral blood lymphocytes were prepared on FicollPAQUE gradient. Total B lymphocytes were further isolated by positive selection of CD19⁺ cells using magnetic anti-CD19 microbeads (StemCell Technology) after depletion of glycophorin A⁺, CD14⁺, CD56⁺, CD16⁺ and CD3⁺ cells (Miltenyi Biotech). All antibodies were from Beckman Coulter. Purified CD19⁺ B cells, BL41, Jijoye, Daudi, B104, Val, BP3, Raji, U2932, OCI-Ly3 and HBL1 lymphoma B cell lines and B cell clones were cultured in RPMI-1640 medium supplemented with 10% (vol/vol) fetal calf serum (FCS), antibiotics (penicillin and streptomycin; Gibco), Hepes (Gibco) and Gentamycin (Invitrogen). SUDHL4, SUDHL6 cell lines were cultured in RPMI-1640 medium supplemented with 20% (vol/vol) fetal calf serum (FCS), antibiotics (penicillin and streptomycin; Gibco), Hepes (Gibco) and Gentamycin (Invitrogen). OCI-Ly10 cell line was cultured in IMDM medium supplemented with 20% (vol/vol) fetal calf serum (FCS), antibiotics (penicillin and streptomycin; Gibco), Hepes (Gibco) and Gentamycin (Invitrogen). Human Mammary Epithelial Cells (HMEC) were obtained from Lonza and cultured in Mammary Epithelial Growth Medium (TEBU, 815-500). Immortalized human mammary epithelial cells (MCF10A), MCF7, T47D, MDAMB435S, MDAMB453, MDAMB231 and MDAMB468 cell lines were obtained from the American Type Culture Collection. MCF10A cells were cultured in DMEM-F12 medium (Invitrogen) supplemented with 10% FCS, insulin (10 µg/mL) hydrocortisone (0.5 µg/mL), EGF (2.5 µg/mL) and cholera toxin (2.5 µg/mL). T47D cell line was cultured in RPMI medium (Invitrogen) supplemented with 10% FCS, 0.01 mg/mL insulin and 1 mM sodium pyruvate. MCF7 cell line was cultured in DMEM medium (Invitrogen) supplemented with 10% FCS and 0.01 mg/mL insulin. MDAMB231 cell line was cultured in DMEM medium (Invitrogen) supplemented with 10% FCS. MDAMB435S cell line was cultured in L15 medium (Invitrogen) supplemented with 10% FCS, 0.01 mg/mL insulin and 0.01 mg/mL glutathione. MDAMB453 and MDAMB468 cell lines were cultured in L15 medium (Invitrogen) supplemented with 10% FCS. Primary human hepatocytes were obtained from Cancéropole Auvergne Lyon Rhone-Alpes platform. Hepatocytes were prepared from liver lobectomy segments taken from a donor liver that had not been used for transplantation. Hepatocytes were isolated according to the previously published procedure (Pichard L. et al., Molecular Pharmacology, 1992). For each culture, hepatocytes were seeded at confluence (1.4.10⁵ cells/cm²) in 6-well plates or 25cm² culture dishes precoated with collagen I (Corning) in a total volume of 3 ml of William's medium E (Invitrogen). For the first 4 h, 5% FCS was present in the medium to favor cell attachment. The serum-rich medium was then removed and cells were maintained in serum-free medium at 37 °C and 5% CO₂. The use of human hepatic specimens for the present study has been approved by the French National Ethics Committee.

Recombinant cytokines: For B cells and cell lines: recombinant BAFF (PeproTech, ref 310-13) and recombinant IL-17 (PeproTech, ref 200-17) were used at 100 ng/mL and 1 ng/mL, respectively, for B cell experiments. Anti-CD40 (mAb89 Santa Cruz) was used at 20 µg/mL, anti-IgM (eBioscience, SA-DA4) was used at 20 µg/mL and CpG2006 (InvivoGen) at 2.5 µg/mL. For epithelial cells, recombinant IL-17 (PeproTech, ref 200-17) was used at 10 ng/mL, recombinant IL-6 (PeproTech, 200-06) was used at 50 ng/mL, recombinant TNFα (PeproTech, 300-01A) was used at 50 or 100 ng/mL, recombinant IL-1β (eBioscience, 14-8018-62) was used at 50 ng/mL and recombinant TGFβ (PeproTech, 100-21) was used at either 8 or 10 ng/mL.

Neutralizing antibodies: For B cells and cell lines: IL-17- (eBioscience, 16-7178), BAFF- (eBioscience, 14-9017), IL-10- (anti-IL-10, R&D Systems, MAB2171, Clone 25209) and IL-6- (R&D Systems, Mab 206, clone 6708) specific neutralizing antibodies were used at the concentration of 300 ng/mL. For epithelial cells: IL-17- (eBioscience, 16-7178) and IL-6- (R&D Systems, Mab 206, clone 6708) specific neutralizing antibody were used at 1µg/mL With the exception of those noted in figure 28, the IL-17 neutralizing antibody used in all experiments was the reference neutralizing antibody from eBioscience (16-7178).

Chemotherapy: Paclitaxel (Sigma) and doxorubicin (Sigma) were used at 1-10 nM and 10-300 nM respectively, as indicated.

### Lentiviral Infections

Infections were performed at an optimized multiplicity of infection (MOI) of 14 or 16 PFU per cell in complete adequate medium leading to a percentage of infected cells systematically greater than 95%. PWPIR lentiviral vector was used to express ectopic Twist-1 and c-MYC. Lentiviral short hairpin RNA (pLVTHM) targeting the *LUCIFERASE* (Control) or *TWIST1* human genes were described previously (Ansieau et al., Cancer Cell, 14: 79-89, 2008). Lentiviral ShRNA particles targeting human *IL17A* gene (sc-39649-V) and human *AICDA,* the gene coding for AID (sc-42729-V) were purchased at Santa Cruz Biotechnologies.

### Immunoblotting

Cells were lyzed in ice-cold lysis buffer (10 mM Tris-HCl pH 7.8, 1% Nonidet P-40, 150 mM NaCl that include protease inhibitors). Cell lysates were electrophoresed through 4-12% acrylamide gels (Invitrogen) and transferred to nitrocellulose membranes (Invitrogen). Membranes were probed with antibodies against Twist-1 (obtained from Roberta Maestro, CRO IRCCS, National Cancer Institute; Aviano), p53 (Santa Cruz Biotechnologies, sc-47698), p21CIP1/WAF1 (Santa Cruz Biotechnologies, sc-53870), c-Myc (Santa Cruz Biotechnologies, SC-40), AID (18783-78480, Genway), E-Cadherin (610181, BD Biosciences), Vimentin (Dako, V9), α-Catenin (BD Biosciences, 610194), Occludin (BD Biosciences, 611090), N-Cadherin (BD Biosciences, 610920) and β-Actin (Sigma, AC-15), followed by Horse Radish Peroxydase-conjugated anti-mouse or rabbit Ig antiserum (GE healthcare). The membranes were developed using ECL (Pierce).

### DNA Damage Analysis

Cells were grown in adequate medium (GIBCO) and subjected to cytokine treatment for 48 to 120 h. Cells were then fixed on Coverslips with 10% formaldehyde for 1 h and permeabilized with 0.5% Triton-X-100 for 30 min, blocked with 3% BSA for 3 h, and then incubated with anti-p-yH2AX (Abcam, ab2893) and anti-p-ATM (Abcam, ab36810) antibodies for 2 h and Alexa-488 or Alexa-568 conjugated secondary antibodies for 1 h. Nuclei were counterstained with 2 mg/mL DRAQ5 (Cell Signaling). Coverslips were then mounted with Fluoromount-G.

### Subcutaneous mouse Xenografts

Animal maintenance and experiments were carried out in accordance with the animal care guidelines of the European Union and with French laws and were validated by the Comité Régional d'Ethique Animale CNRS Rhône-Alpes. Four week- old female athymic Swiss nude mice (Charles River Laboratories) were irradiated (4 gy) and subcutaneously grafted in the left flank with 10⁶ cells. Tumor growth was monitored twice a week with calipers at the site of injection. Animals were allowed to form tumors up to 17 mm in diameter, at which point they were euthanized.

### Quantification of cytokines by ELISA

Amounts of IL-17, IL-6 were measured with commercial ELISA kits (PeproTech), amounts of BAFF and IL-10 was determined with the fluorokine commercial ELISA kit Quantikine (R&D Systems) following instructions of the manufacturer. For blood samples, quantification of cytokines was performed in the serum. For cell lines, quantification of cytokines was performed in the cell culture supernatant at various timepoints.

For quantification of IL-17 secretion by tumor cells from nude mice experiments, tumors were surgically removed and were dissociated with 100 µm cell culture filters. Cells were then cultured in adequate medium and quantification of IL-17 was performed in the cell culture supernatant of 5 day cultures.

### Cell Viability

Cell viability was assessed by propidium iodide (Sigma) uptake. Data were acquired on a FACSCalibur (BD Biosciences) and were analyzed with FlowJo software (TreeStar).

### Cohort of blood samples from healthy donors and lymphoma patients

Sera from 40 healthy adult donors were prepared from blood samples from healthy adult donors obtained from the Etablissement Français du Sang (EFS). Sera from lymphoma patients were obtained from the Centre de Biologie Lyon Sud. Approval was obtained according to local ethic committees of the Hospices Civils de Lyon. Informed consent was provided to each subject.

### Standard R-banding karyotyping

Standard R-banding was carried out on metaphasic cells by routine cytogenetic techniques (Biomnis) by a cytogenetician following the recommendations of the International System for Human Cytogenetic Nomenclature (ISCN 2009).

### Array-Comparative Genomic Hybridization (a-CGH)

a-CGH was performed by IMAXIO (France) on Agilent's 4 x 180 k human aCGH array.

### 3D Cell Culture (Acinus formation assay)

Culture slides (BD Bioscience) containing 100 µl of Matrigel (BD Bioscience) were incubated 10 min at 4°C followed by 15 min at 37°C. 5.10³ cells were resuspended in complete medium with 2% of Matrigel, placed on top of the culture slides, and then cultured for 3 weeks, supernatant being replaced with complete medium containing 2% Matrigel twice a week. Each single cell gives rise to one acinus-like structure.
For immunofluorescence, the supernatant of the culture slides was removed and cells were washed with PBS. Cells were then fixed with 4% paraformaldehyde for 20 min, washed in 1% PBS, permeabilized with 0.5% triton/PBS. The actin (cytoskeleton) was then marked using Phalloidin-TRITC (5 µg/mL, Sigma) and nuclei were counterstained with 2 mg/mL DRAQ5 (Cell Signaling). Culture slides were then mounted with Fluoromount-G (Southern Biotech) and analyzed using a confocal microscope

### Soft Agar Assays

Cells were cultured in 6-well plates prefilled with 0.75% agar (SeaPlaque agarose, Cambrex) complete medium. Cells (2.10⁴) were placed on top of the prefilled wells in 0.45% agar complete medium. Isolated cells are thus allowed to grow in suspension. Development of clones from a single cell demonstrates the ability to have anchorage independent growth, a characteristic of transformed cells. Each condition was performed in triplicates. For quantification of colonies, three randomly selected fields for each well were counted (that is, 9 fields per condition), and the number of colonies was then calculated.

### EMT analysis by immunofluorescence

Cells on coverslips were fixed with 4% formaldehyde for 10 min, permeabilized with 0.5% Triton X-100 for 25 min at room temperature, saturated for 30 min with 10% FCS in PBS, washed in PBS, incubated 2 h at room temperature with monoclonal anti-E-Cadherin (610181, BD Biosciences) or anti-Vimentin (V9, Dako) antibodies, and then incubated for 1 h with FITC- or TRITC-conjugated rabbit anti-mouse antibodies (Dako). Nuclei were counterstained with 2 mg/mL DRAQ5 (Cell Signaling). Coverslips were then mounted with Fluoromount-G.

### Cluster Assays

Matrigel (BD Biosciences) was added to the wells of an eight-well Labtek chamber (BD Biosciences) in a volume of 300 µl/well. A Matrigel plug of about 1 mm diameter was removed. The hole was successively filed with 10⁵ cells and 100 µl of Matrigel. Appropriate growth medium was added on top. Cultures were analyzed for up to 4 days. Areas of migration were visualized using an Olympus IX50 (NA 0.075). Samples were performed in duplicate.

### In vitro migration and invasion assays.

For transwell migration assay, 5.10⁴ cells were plated in the top chamber with the non-coated membrane (24-well insert; pore size, 8 mm; BD Biosciences). For invasion assay, 5.10⁴ cells were plated in the top chamber with Matrigel-coated membrane (24-well insert; pore size, 8 mm; BD Biosciences). In both assays, cells were plated in 1% serum medium, and 10% serum medium was used as a chemo-attractant in the lower chamber. The cells were incubated for 24 h. Cells that reached the lower chamber were stained with Giemsa and counted. Total number of migrating/invading cell was calculated by analyzing 5 fields per well in two independent experiments, that is 10 fields per condition.

### Detection of IL-17R, CD24 and CD44 expression by flow cytometry.

Expression of human IL-17R (FAB177P, R&D system), CD24 (BD Pharmingen) and CD44 (BD Pharmingen) was determined by flow cytometry on a FACSCalibur flow cytometer (BD Biosciences) and analyzed using FlowJo software (Tree star). The antibodies were used at 1/25, 1/100 and 1/100 respectively.

### Tissue microarrays

IL-17 staining on tissues arrays (SUPER BIO CHIPS, slide CBA3) was performed by an anatomopathologist using anti-IL-17 antibody (polyclonal goat antiserum, 5 mg/mL, R&D Systems) according to a protocol adapted from Coury F et al. (2008), "Langerhans cell histiocytosis reveals a new IL-17A-dependent pathway of dendritic cell fusion." Nat Med 14: 81-87, and validated on renal allograft paraffin sections from patient with chronic active rejection.

### Orthotopic mouse Xenografts

Animal maintenance and experiments were carried out in accordance with the animal care guidelines of the European Union and with French laws and were validated by the Comité Régional d'Ethique Animale CNRS Rhône-Alpes. Four week- old female athymic Swiss nude mice (Charles River Laboratories) were irradiated (4 gy) and grafted in the mammary fat pad with 10⁵ cells. Tumor growth was monitored twice a week with calipers at the site of injection. Animals were allowed to form tumors up to 17 mm in diameter, at which point they were euthanized.

### Metastases detection

The draining lymph node, the lungs, the liver and the spleen were surgically removed, dissociated with 100 µm cell culture filters and washed twice with 1 mM EDTA-PBS buffer. Dissociated cells were analyzed by flow cytometry using anti human IL-17R antibody (FAB177P, R&D system) to detect human MDAMB231 cancer cells. Of important note, the anti-human IL-17R antibody did not cross-react with murine cells.

### Human Primary Hepatocyte Infection by HCV

Human primary hepatocytes seeded in 25 cm² culture dishes were incubated with HCV genotype 3A inoculum (0.1 MOI) in 3 ml serum free William's medium E for 36 h. Cells were washed three times with Williams' E medium and then maintained in William's medium E supplemented with 10% FCS. The medium was changed every 3 days until harvest.

### Foci Formation Assay

Human primary hepatocytes (10³) were infected or not with HCV (genotype 3A). Three days following infection cells were left untreated (NT) or stimulated with of TNFα (100 ng/mL), TGFβ (8 ng/mL) and IL-17 (10 ng/mL) for 9 days. Then cells were trypsinized and co-cultured with 10⁵ human primary hepatocytes. Cells were grown at confluence in 6-well plates. Untransformed cells stop proliferating when they reach confluency. On the other hand, transformed hepatocytes have lost contact inhibition and form foci that were visualized by fixation (paraformaldehyde 4%) and Giemsa staining.

### EXAMPLE 2: IL-17 and IL-17/BAFF and Lymphoma

It has previously been shown that patients with Systemic Lupus Erythematosus (SLE) or Rheumatoid Arthritis (RA) have an increased risk of developing non-Hodgkin's lymphoma. The present inventors have shown that patients with SLE or RA have increased serum levels of IL-17 and BAFF. The present inventors have also demonstrated that IL-17 and BAFF sustain the expression of AID in activated B lymphocytes. AID enzyme promotes the deamination of cytidine residues into uracils, creating DNA mismatches that are processed into mutations or double-strand break intermediates leading, under physiological conditions, to both somatic hypermutation and class switch recombination (Muramatsu M. et al., Cell, 102(5):553-563 2000). These two processes, which occur within germinal centers, reshape the primary antibody repertoire after antigen encounter. AID is therefore essential to antibody diversity and efficient humoral immunity. Although AID preferentially targets immunoglobulin (Ig) loci, it has been clearly demonstrated in mice that AID acts more widely than previously thought and that numerous mutations in non-Ig loci, target genes that are linked to B cell tumorogenesis (Liu M. et al., Trends Immunol., 30: 173-181 2009). Genetic instability and the appearance of oncogeneic mutations resulting from AID-induced DNA alterations in non-Ig genes is revealed when mechanisms involved in the surveillance of DNA damage, such as the p53 pathway, are inhibited (Ramiro A.R. et al., Nature, 440: 105-109, 2006).

The present inventors showed that exposure of human B lymphocytes (either activated CD19⁺ peripheral B lymphocytes purified from healthy donors or the B104 human B lymphoma cell line) to IL-17 and BAFF causes an overexpression of AID and the concomitant upregulation of the transcription factor Twist-1, which, in turn, inhibits the expression of the p53 tumor suppressor, as well as its direct target p21 (Figure 1A, B). Inhibition of the p53 tumor suppressor is indeed mediated by Twist-1 as B104 cells expressing Twist-1 shRNA (shTwist-1) do not show inhibition of p53 and p21 in response to IL-17 and BAFF stimulation (Figure 1B).

The present inventors also showed that IL-17 and BAFF induce genomic instability in B lymphocytes. Exposure of activated human CD19⁺ peripheral B lymphocytes (Figure 2A) or B104 human B lymphoma cell line (Figure 2B) to IL-17 and BAFF induce DNA damage at 48 h that persisted at 120 h, as revealed by staining with antibodies directed against phosphorylated histone H2AX (y-H2AX). Thus, IL-17 and BAFF induce genomic instability in B lymphocytes. Stimulation of CD19⁺ peripheral B lymphocytes or B104 cells in the presence of CpG also induced detectable DNA damages at 48 h, but DNA damage was transient and did not persist over time. Inhibition of Twist-1 expression (shTwist-1) in B104 cells reduced DNA damage observed at 48 h and prevented the persistence of damage in response to IL-17 and BAFF stimulation (Figure 2B). Inhibition of the expression of the DNA mutator AID (shAID) in B104 cells prevented the generation of DNA damage in response to both types of stimulation (Figure 2B).

The present inventors also showed that sera from both SLE patients and RA patients induced genomic instability in B lymphocytes. Figure 2C, top two rows, shows exposure of B104 cells to serum from SLE patients. The bottom two rows of Figure 2C show exposure of B104 cells to serum from RA patients. Untreated B104 cells (NT) showed no DNA damage. With serum treatment alone, numerous cells showed signs of DNA damage at 48 h and 120 h, as revealed by staining with antibodies directed against phosphorylated histone H2AX (y-H2AX). However, importantly, when serum-exposed cells were also treated with IL-17 neutralizing antibody, BAFF neutralizing antibody or a combination of both IL-17 and BAFF neutralizing antibodies, cells did not display any DNA damage in response to treatment with SLE or RA sera. (Figure 2C).

The present inventors also demonstrated that stimulation of human CD19⁺ peripheral B lymphocytes (purified from healthy donors) with IL-17 and BAFF is sufficient for long-term survival and immortalization of normal human B cells and that immortalized B cell clones are tumorogenic *in vivo.* Human CD19⁺ peripheral B lymphocytes purified from 7 different healthy donors were stimulated with BCR, CD40 stimulatory antibodies and CpG or IL-17 and BAFF combination for five days and then cloned by limiting dilution in medium supplemented with CpG or IL-17 and BAFF combination (Figure 3A). The long-term culture of activated CD19⁺ peripheral B lymphocytes in the presence of IL-17 and BAFF, but not in the presence of CpG, allowed the emergence of immortal B cell clones (hereafter referred as "B cell clones"). The spontaneous immortalization and transformation rate of CD19⁺ peripheral B lymphocytes was about 0.3%. Figure 3B shows that B cell clones obtained following long-term culture in the presence of IL-17 and BAFF were tumorigenic *in vivo.* Subcutaneous engraftment of B cell clones (10⁶ cells) in irradiated nude mice led to a rapid tumor engraftment of the 6 clones tested so far (Figure 3B).

The present inventors showed that the B cell clones transformed by exposure to IL-17 and BAFF have acquired capacity to secrete, in an autocrine manner, IL-17 and BAFF (Figure 4). Compared to the B104 B cell line, all the clones that were tested spontaneously produced variable levels of IL-17 and BAFF (Figure 4A). Figure 4B shows that the administration of IL-17 and BAFF neutralizing antibodies (alone or in combination) induced from ∼20 to 35% of cell death in B cell clones (white bars). Moreover, B cell clones were remarkably resistant to doxorubicin-induced apoptosis (Figure 4B). Resistance of B cell clones to doxorubicin-induced apoptosis was associated with an absence of induction of p53 and its target p21 in response to doxorubicin treatment (Figure 4C). Importantly, the results showed that blockade of secreted IL-17 and BAFF with specific neutralizing antibodies sensitized B cell clones to doxorubicin-induced apoptosis (from ∼50 to 78% of cell death, black bars) (Figure 4B).

The present inventors showed that immortalization and transformation of human CD19⁺ peripheral B lymphocytes (purified from healthy donors) with IL-17 and BAFF is associated with acquisition of genetic alterations such as point mutations in multiple oncogenes and/or tumor suppressor genes frequently involved in B cell lymphomas. As shown in Table 1, below, sequencing of C-MYC, BCL6, PAX5 and PIM1 oncogenes and TP53 tumor suppressor following PCR amplification of genomic DNA showed acquisition of mutations in all the genes tested, TP53, C-MYC and BCL6 being the most frequently mutated genes.

**Table 1: 6 B cell clones obtained following long-term culture of human CD19⁺ peripheral B lymphocytes in the presence of IL-17 and BAFF were analysed for the potential acquisition of mutations in multiple oncogenes and/or tumor suppressor genes frequently involved in B cell lymphoma as described in Example 1.**

| | **TP53 Ex5** | **TP53 Ex6-9** | **TP53 Ex10** | **TP53 Ex11** | **C-Myc Ex2** | **C-Myc Ex3** | **BCL6** | **PAX5** | **PIM1** |
|---|---|---|---|---|---|---|---|---|---|
| **DN1#1** | - | + | - | - | + | - | - | - | + |
| **DN1#2** | - | - | - | - | - | - | - | - | - |
| **DN1#3** | + | + | - | - | + | - | + | - | - |
| **DN1#4** | - | ? | - | - | - | - | + | - | - |
| **DN1#5** | - | + | - | - | - | + | - | - | - |
| **DN1#6** | - | + | - | - | + | - | + | + | - |

In patients, lymphomas arising from plasmablasts are called "Activated B Cell - Diffuse Large B Cell Lymphoma," or "ABC-DLBCL" (Lenz G, NEJM, 2010). ABC-DLBCL is a subtype of DLBCL and is characterized by activation of STAT3 and secretion of IL-6 and IL-10 (Lam LT et al., Blood, 2007).The present inventors showed that the B cell clones generated by stimulation with IL-17 and BAFF have a plasmablast-like phenotype and share special features of ABC-DLBCL. The present inventors showed that the B cell clones express Blimp1 but not PAX5 (Figure 5A) and thus have a plasmablast-like phenotype (Montes-Moreno S, Haematologica, 2010). In line with this, human ABC-DLBCL cell lines, as well as B cell clones generated by exposure to IL-17 and BAFF, have an activated Stat3 (Figure 5B). This is in contrast to the germinal center B-like subtype of DLBCL (GCB-DLBCL, Figure 5B). Human ABC-DLBCL cell lines, as well as B cell clones generated by exposure to IL-17 and BAFF, also secrete IL-6 and IL-10 (Figure 5C). Again, this is in contrast to GCB-DLBCL and to non-DLBCL lymphoma cell lines, as shown in Figure 5C. Furthermore, the present inventors have shown for the first time that ABC-DLBCL cells, as well as B cell clones generated by exposure to IL-17 and BAFF, also secrete IL-17 and BAFF. A comparison of cytokine secretion by non-DLBCL lymphoma cell lines, GCB-DLBCL cell lines and ABC-DLBCL cell lines is shown in Figure 6. ABC-DLBCL cell lines displayed a characteristic cytokine expression profile with high levels of IL-17, BAFF, IL-6 and IL-10 that is not seen in other lymphoma cell lines, which secrete no or low levels of IL-17, BAFF, IL-6 and IL-10.

**Table 2: Classification and distribution of subtypes Non-Hodgkin's Lymphoma.**

| | |
|---|---|
| Diffuse Large B Cell (DLBCL) | 31% |
| Follicular (FL) | 22% |
| Marginal Zone B Cell (MZL), MALT | 8% |
| Small B lymphocytic | 7% |
| Peripheral T cell | 7% |
| Mantle cell | 6% |
| Marginal Zone B Cell (MZL), nodal | 3% |
| Lymphoblastic | 2% |
| Anaplastic T/null | 2% |
| Primary mediastinal large B cell | 2% |
| Burkitt | <1% |
| others | 10% |

| | |
|---|---|
| *Adapted from "A Clinical Evaluation of the International Lymphoma Study Group Classification of Non-Hodgkin's Lymphoma," Blood, 1998 | |

MALT=Mucosa associated lymphoid tissue; Representative percentages of lymphoma subtypes in adults are indicated. DLBCL is the main non-Hodgkin's lymphoma in adults.

As shown in Table 2, Diffuse Large B Cell Lymphoma (DLBCL) is the main non-Hodgkin lymphoma in adults, representing about 31% of cases. For patients with DLBCL, the subtype has important clinical implications, since the ABC subtype is refractory to standard chemotherapy such as R-CHOP treatment (rituximab + cyclophosphamide + doxorubicin + vincristin) and has the worst prognosis (Table 3). Indeed, five-year survival ranges from 59 to 62% for GCB-DLBCL patients compared to 26-31% in ABC-DLCBL patients (Table 3).

**Table 3: DLBCL Subtype and prognosis.**

| | 5 year survival | |
|---|---|---|
| | Lymphochip | Affimetrix |
| GCB-DLBCL | 59% | 62% |
| ABC-DLBCL | 31% | 26% |

ABC-DLBCL lymphomas have a much worse prognosis compared to GCB-DLBCL lymphomas. Table displaying 5-year survival rates of ABC-DLBCL and GCB-DLBCL patients. Adapted from Wright, G., "A gene expression-based method to diagnose clinically distinct subgroups of diffuse large B cell lymphoma," PNAS, 2003, which shows Kaplan Meier curves of patients with GCB-DLBCL or ABC-DLBCL. ABC or GCB subtypes were determined by gene expression profiling using Lymphochip or Affimetrix microarrays. The original standard for DLBCL classification used gene expression profiling of frozen tissues (Alizadeh, AA, Nature, 2000; Wright, G, PNAS, 2003) which nicely predicts patient outcome as exemplified in Table 3. However, such profiling is generally not feasible in the clinic. An immunohistochemistry (IHC) classification scheme based on 3 antibodies (Hans, CP et al., Blood, 2004) is used as a substitute for gene expression profiling classification. An improved IHC scheme based on 5 antibodies has also been proposed (Choi, WWL, Clin Cancer Research, 2009). However, recent clinical trials showed that IHC classification does not correlate well with gene expression profiling classification and does not predict outcome (Ott, G, Blood, 2010). Thus, accurate classification of ABC and GCB subtypes is still needed in order to predict patient prognosis and response to chemotherapy such as R-CHOP treatment.

Quantifications of IL-17, BAFF, IL-6, and IL-10 levels in blood samples from healthy donors and lymphoma patients showed that the majority of DLBCL patients in the study cohort have increased IL-17, BAFF, IL-10 and IL-6 levels (Figure 7). While ABC or GCB classification for DLBCL patients was not available in this cohort, based on the differential secretion of IL-17, BAFF, IL-6 and IL-10 in human ABC-DLBCL cell lines as compared to GCB-DLBCL and non-DLBCL cell lines *in vitro* (see Figure 6), the present inventors have identified a cytokine signature which can distinguish the ABC-DLBCL from GCB-DLBCL and from other lymphomas. This may serve a beneficial diagnostic and therapeutic purpose by allowing this subtype of patients to be readily identified, and to develop a therapeutic approach that targets the cytokines associated with this subtype: IL-17 and BAFF, along with IL-6 and/or IL-10.

As shown in Figure 8, the present inventors have shown that neutralizing antibodies to IL-17, BAFF, IL-6 and IL-10 induced cell death and sensitization to chemotherapy of ABC-DLBCL-like B cell clones. Neutralizing antibodies to IL-17, BAFF, IL-6 and IL-10 induced a certain degree of apoptosis of B cell clones generated by exposure to IL-17 and BAFF compared to medium alone. The combination of neutralizing antibodies to IL17 and BAFF further increased apoptosis of B cell clones, as did the combination of neutralizing antibodies to IL6 and IL10. However, the combination of neutralizing antibodies to IL-17 and BAFF antibodies greatly sensitized the cells to chemotherapy (doxorubicin), whereas the combination of neutralizing antibodies to IL6 and IL10 did not. The greatest level of apoptosis without chemotherapy was seen with a combination of neutralizing antibodies to all four cytokines (i.e., IL-17, BAFF, IL-6 and IL-10). Likewise, this combination of 4 neutralizing antibodies induced the greatest sensitivity to chemotherapy (doxorubicin) with the highest overall percentage of apoptosis. Figure 8 displays results obtained with one B cell clone.

As demonstrated by the above, IL-17 and BAFF play a key role in B cell lymphoma development and induce lymphoma cell survival and resistance to chemotherapy such as doxorubicin. Hence, the quantification of IL-17 and BAFF expression are useful methods of diagnosing patients with B cell lymphoma. Furthermore, antagonists to IL-17 and BAFF, such as antibodies and/or other therapies, such as small molecule antagonists, nucleic acid antagonists and gene silencers (e.g., siRNA and shRNA) are useful methods of treating patients with B cell lymphoma or to increasing response to conventional chemotherapeutic agents. More specifically, the quantification of IL-17 and BAFF expression or a combination of IL-17 and BAFF expression with IL-6 and/or IL-10 expression are useful methods of diagnosing patients with ABC-DLBCL, who have a poor prognosis with only 26-31% survival at 5 years, and who can not be diagnosed with the previously available diagnostic tools. Furthermore, a combination of antagonists to IL-17 and BAFF or a combination of antagonists to IL-17 and BAFF with IL-6 and/or IL-10, such as antibodies and/or other therapies, such as small molecule antagonists, nucleic acid antagonists and gene silencers (e.g., siRNA and shRNA) are useful methods of treating ABC-DLBCL patients who poorly respond to conventional chemotherapeutic agents (such as R-CHOP) and/or to increase response to conventional chemotherapeutic agents.

### EXAMPLE 3: IL-17 and Breast Cancer

IL-17 receptors are ubiquitously expressed at the cell surface of human cells including mammary epithelial cells. Furthermore, it was shown that Activation Induced Deaminase (AID), which is not expressed in epithelial cells, can, however, be induced in breast cells by estrogens (Pauklin S. et al., J Exp Med,, 206: 99-111, 2009) and is expressed in breast cancer cells (Babbage G. et al., Cancer Res, 66: 3996-4000, 2006). The present inventors demonstrated that exposure of human normal primary mammary epithelial cells (HMEC) (Figure 9A) or immortalized mammary epithelial cells (MCF10A) (Figure 11B) to IL-17 induces upregulation of AID and the concomitant upregulation of the transcription factor Twist-1, which, in turn, inhibits the p53 tumor suppressor. IL-17, IL-1β, TNFα and TGFβ induced AID protein in HMEC (Figure 9A) and MCF10A (Figure 9B). On the other hand, IL-17, and to a lower extent, IL-6, TNFα and TGFβ induced Twist-1 protein (Figure 9A, 9B). Induction of Twist-1 was stronger with IL-17 than with TNFα and TGFβ, and only IL-17 induced stable Twist-1 expression at later timepoints (Figure 9C). In accordance with the ability of Twist-1 to inhibit p53, IL-17-stimulated cells showed a complete inhibition of p53 and its direct target, p21, whereas IL-1β-treated, IL-6-treated, TNFα-treated, TGFβ-treated, and untreated (NT) cells did not (Fig. 9A, 9B). Also, the p53 pathway was not functional in IL-17 treated cells, as IL-17-stimulated cells were unable to upregulate p53 and p21 in response to doxorubicin (Figure 9D). The inhibition of the p53-dependant pathway by IL-17 was mediated by Twist-1 as *TWIST1* knockdown by shRNA restored p53 and p21 upregulation in response to doxorubicin in IL-17-treated cells (Figure 9D).

The present inventors demonstrated that IL-17, but not other cytokines such as TNFα and TNFβ, induces genomic instability (unrepaired DNA damage) and oncogeneic events in mammary epithelial cells. The cytokines that induced AID (i.e., TNFα, TGFβ and IL-17) generated DNA lesions in MCF10A cells as demonstrated by p-ATM and p-yH2AX nuclear foci at 48 h post stimulation, and *AICDA* knockdown prevented the appearance of such lesions (Figure 10A). Importantly, only IL-17-stimulated cells displayed persisting DNA lesions beyond 5 days (Figure 10A). As Twist-1 inhibited p53 (see Figure 9), it was thought that it mediated the maintenance of the DNA lesions. Indeed, *TWIST1* knockdown did not prevent DNA lesions occurrence but prevented their persistence (Figure 10A). Similar results were obtained in primary HMEC cells (Figure 10B). Taken together, these results demonstrate that concomitant induction of AID and Twist-1 by IL-17 generates widespread DNA lesions in mammary epithelial cells. Among the cytokines tested, only IL-17 could sustain both AID and Twist-1 expression and generate persistent DNA lesions, highlighting IL-17's specific effect on the generation of persistent DNA lesions.

The present inventors demonstrated that IL-17 induced genomic instability in mammary epithelial cells. IL-17-induced global genomic alterations were first screened for by standard R-banding karyotyping carried out on metaphasic cells. Whereas parental MCF10A cells displayed a nearly diploid karyotype with a derivative chromosome 9 translocated t(5;3;9) and trisomy for chromosomes 1q and 20 as originally described (Owell, JK et al. (2005) Cancer Genet Cytogenet 163: 23-29), IL-17 treated cells displayed composite and complex karyotypes with both gains and losses of whole chromosomes, as well as structural chromosomal aberrations (Figure 11). Chromosomal instability was further illustrated by array-based comparative genomic hybridization (a-CGH). The profile of DNA copy number gains and losses across MCF10A cells showed an important chromosomal instability associated with major amplifications and deletions (including whole chromosomes) all over the genome in IL-17-treated cells (Figure 12). To confirm that these genomic aberrations were not due to a putative transformed phenotype of the cells or an artifact of long term culture, also included were MCF10A cells transformed by defined genetic events (cMYC + TWIST1) (Valsesia-Wittmann, S et al., Cancer Cell, 2004). At 42 days post genetic transformation, these cells retained a parental a-CGH profile with only 3 small genomic amplifications detected by a-CGH (Figure 12). Thus, chromosomal alteration in IL-17-treated cells result from IL-17-mediated genomic instability.

The present inventors demonstrated that exposure to IL-17 is sufficient to disrupt normal mammary tissue homeostasis. As shown by the Matrigel assay in Figure 13A, IL-17-stimulated MCF10A cells do not form normal acinar structures. Indeed, whereas control MCF10A cells or MCF10A cells treated with TNFα or TGFβ form well-organized and delimited acini with a central lumen in 3D culture, IL-17 treated MCF10A cells had lost their ability to form acinus-like structures as they lacked the central lumen (reminiscent of the alteration of p53 (Danes, CG et al., Cancer Research, 2008)) and expanded into the extracellular matrix (ECM), suggesting invasive abilities. Furthermore, the present inventors demonstrated that when MCF10A were exposed to IL-17 for 21 days (time required to induce genomic instability) and subjected to soft-agar assay, most cells were transformed (Figure 13). As a control, TNFα or TGFβ, which were unable to induce genomic instability in mammary epithelial cells (Figure 10), gave rise to a very limited number of clones (Figure 13).

The present inventors demonstrated that exposure of mammary epithelial cells to IL-17 is sufficient to generate breast cancer cells. The present inventors demonstrated that stimulation of immortalized mammary epithelial cells MCF10A by IL-17 generates cancer cells *in vivo.* MCF10A cells were exposed for 3 weeks to IL-17 and subcutaneously engrafted in irradiated nude mice. As shown Figure 14A, all mice injected with IL-17-treated cells developed tumors, whereas mice engrafted with MCF10A cells never did so. A representative example of a tumor bearing mouse is illustrated in Figure 14B. To delineate the role of AID and Twist-1 in IL-17-induced carcinogenesis, MCF10A were first transduced with *AICDA* or *TWIST1* targeting shRNA, exposed to IL-17 for 21 days and then subjected to soft agar and tumor growth assays. In these settings, DNA lesions cannot occur (*AICDA* shRNA) or are eliminated (*TWIST1* shRNA) as shown previously (see Figure 10). *AICDA* or *TWIST1* knocked down cells had dramatically decreased ability to form clones in agar (Figure 13) and did not form tumors in mice despite 5.10⁶ cells were implanted (Figure 14C), whereas cells infected with a control shRNA gave rise to tumors as observed with uninfected cells. Thus, both AID and Twist-1 are required for IL-17-induced mammary carcinogenesis..

The present inventors have shown that stimulation of mammary epithelial cells with IL-17 induces an epithelial to mesenchymal transition (EMT) via Twist-1 upregulation which increases migration, invasion and stem cell population. The present inventors have shown that IL-17 induces an EMT in MCF10A characterized by a switch from cobblestone-like to spindle like morphology (Figure 15, upper lane) by loss of the epithelial marker E-Cadherin and gain of the mesenchymal marker Vimentin as demonstrated by immunofluorescence (Figure 15, middle lane and Figure 16A). IL-17 induced EMT was further demonstrated by the downregulation of several epithelial markers (E-Cadherin, α-Catenin and Occludin) and the upregualtion of several mesenchymal markers (N-Cadherin, Vimentin), as demonstrated by immunoblotting (Figure 16B). IL-1β, TNFα or TGFβ did not induce EMT compared to IL-17 (Figure 16A, 16B). IL-17-induced EMT was mediated by Twist-1 upregulation as *TWIST1* knocked down MCF10A cells (shTWIST1) did not undergo EMT following IL-17 stimulation (Figure 16A). As a control, *AICDA* (shAICDA) or control shRNA (ShCt) did not affect IL-17-induced EMT. IL-17-induced EMT in MCF10A cells was accompanied by a ∼16 fold increase in their ability to migrate in transwell migration assay (Figure 16C, black bars), while TNFα and TGFβ stimulation led to no (TNFα) or a weak (TGFβ) increase in cell migration. IL-17-stimulated cells also displayed a ∼10 fold increase in their invasive ability as assessed in Matrigel invasion assay (Figure 16C, white bars). IL-17 induced invasion was also demonstrated in cluster assay (figure 15, lower lane). Again, TNFα and TGFβ stimulation led to no (TNFα) or a weak (TGFβ) increase in cell invasion. Interestingly, the present inventors have shown that deprivation of exogenous IL-17 reverted EMT (the reverse process being called Mesenchymal to Epithelial Transition, MET, Figure 15, 2^{nd} to 3^{rd} column, upper and middle lanes) and abrogated invasive ability of the cells in cluster assay (Figure 15, 2^{nd} to 3^{rd} column, lower lane), which would tend to abrogate a cell's ability to invade the surrounding tissues and metastasize to distant organs. The present inventors have also shown that IL-17 exposure also increases the percentage of CD24^{low}CD44^{high} stem cells within MCF10A cell line compared to untreated cells. The percentage of CD24^{low}CD44^{high} stem cells in MCF10A was ∼16%, but increased up to 71% upon IL-17 exposure (Figure 16D).

The present inventors have shown that IL-17 increases cell migration and invasion of well-differentiated MCF7 breast cancer cells. MCF7 cancer cells retain an epithelial morphology and are poorly motile and invasive. As shown in Figure 17, MCF7 cells stimulated for 7 days with IL-17, but not with TNFα or TGFβ, underwent complete EMT (Figure 17A) that increased their migratory (∼8 fold) and invasive (∼10 fold, Figure 17B) abilities which is expected to confer metastatic capacities to non-metastatic MCF7 breast cancer cells. The present inventors have also shown that IL-17 exposure also increases the percentage of CD24^{low}CD44^{high} cancer stem cells within MCF7 breast cancer cell line compared to untreated cells. The percentage of CD24^{low}CD44^{high} cancer stem cells in MCF7 was ∼0%, but increased up to 67% upon IL-17 exposure (Figure 17C).

The present inventors have shown that IL-17 expression is increased in human breast cancers. As show in the top panel of Figure 18A, normal cells from a breast cancer patient (Patient #1) do not express IL-17, whereas IL-17 is expressed by tumor cells. As seen in the bottom panel of Figure 18A, the normal cells from Patient #2 also do not express IL-17; however, IL-17 is expressed by immune cells infiltrating the tumor stroma (indicated by arrows). Table 4 shows additional examples of IL 17 expression in normal breast tissues, one hyperplasia, breast tumor cells and cells in the tumor stroma.

**Table 4: IL-17 production by normal breast cells (HMEC) or cell line (MCF10A) and various breast cancer cell lines, along with their tumorigenic and metastatic phenotype.**

| | normal cells | | tumor cells | | | | | |
|---|---|---|---|---|---|---|---|---|
| | HMEC | MCF10A | MCF7 | T47D | MDAMB453 | MDAMB468 | MDAMB435S | MDAMB231 |
| Tumorigenic | | | +/- | + | + | +++ | +++ | +++ |
| Metastatic | | | | | | + | +++ | +++ |
| IL-17 production | | | | | +/- | +++ | +++ | +++ |

Normal cells (HMEC, MCF10A) and poorly tumorigenic cancer cells (MCF7, T47D, MDAMB453) do not secrete IL-17, whereas metastatic human breast cancer cell lines (MDAMB468, MDAMB435S and MDAMB431) secrete IL-17.

The present inventors have shown that human breast cancer cells can produce their own IL-17 (autocrine production). It was shown by the present inventors that breast cancer cells harboring a mesenchymal (MDAMB231, MDAMB435S) or "loosely adherent" (MDAMB468) phenotype that form invasive and dedifferentiated primary lesions and are metastatic in mice secreted large amounts of IL-17 (Figure 19), which is in accordance with IL-17 ability to promote EMT. On the other hand, normal cells (HMEC and MCF10A) and cancer cells that are well-differentiated (epithelial), poorly tumorigenic and non metastatic in mice (MCF7, T47D, MDAMB453) did not release IL-17. Of important note, all cell lines that produce IL-17 also displayed high levels of AID and Twist-1 proteins (Figure 19). Furthermore, inhibition of IL-17 by ShRNA in the MDAMB231 cell line was accompanied by a marked reduction of both AID and Twist-1 protein levels (Figure 19), demonstrating that both AID and Twist-1 expression is indeed driven by autocrine production of IL-17 in these cells.

The present inventors have shown that IL-17 is a therapeutic target in breast cancer. In more detail, the present inventors have shown that IL-17 inhibition or blockade in breast cancer cells that secrete IL-17 (e.g., autocrine production) induces tumor cell death, sensitize cells to chemotherapeutic agents, decreases cancer cell migration and invasion, alters the cancer stem cell pool *in vitro* and abrogates both primary tumor growth and metastasis *in vivo.* The present inventors have shown that IL-17 neutralizing antibody and or inhibition by shRNA blocks the secretion of IL-6 (Figure 20A) and each induces mesenchymal to epithelial transition (reversion of EMT) in the highly metastatic human breast cancer cell line MDAMB231 as shown by cell morphology (switch from spindle like to cobblestone-like morphology, Figure 20B). Mesenchymal to epithelial transition was also demonstrated by the increased expression of epithelial markers (E-cadherin, Occludin, and α-Catenin) and decreased expression of mesdenchymal markers (Vimentin and N-cadherin) (Figure 20C). The MDAMB231 cells in which IL-17 was inhibited by shRNA or neutralizing antibody and that have undergone mesenchymal to epithelial transition then displayed a phenotype that is characteristic of epithelial cells. The present inventors have shown that these cells had decreased motility (Figure 20D, black bars) and were not invasive, as assessed in Boyden chamber assay (Figure 20D, white bars) and in cluster assay (Figure 20E). The present inventors have shown that inhibition of IL-17 in MDAMB231 cells resulted in a drastic decrease in the percentage of CD24^{low} CD44^{high} cancer stem cells (from ∼73% to 27%). The present inventors have also shown that IL-17 (but not IL-6) neutralizing antibody or IL-17 inhibition by shRNA also induced a significant level of cancer cell death (approximately 25% of apoptotic cells in IL-17 disrupted cells *vs* ∼7% in control cells) (Figure 21A). More importantly, the present inventors have also shown that MDAMB231 breast cancer cells where IL-17 is inhibited by shRNA have drastically decreased tumorigenicity *in vivo.* Indeed, MDAMB231 cells where IL-17 was inhibited by shRNA had a significantly reduced ability to form tumors when subcutaneously engrafted in nude mice compared with cells expressing control shRNA (Figure 21B). Moreover, delayed tumors formed by IL-17 knocked down cells had, in fact, re-expressed IL-17 at much higher levels than IL-17 knocked down cells at the time of engraftment, suggesting that IL-17 is required for the *in vivo* growth of MDAMB231 cells. The present inventors have further demonstrated that antagonizing IL-17 in breast cancer cells induces tumor cell death and sensitizes cancer cells to chemotherapeutic agents. In Figure 21D, disruption of IL-17 by neutralizing antibody in MDAMB231 human breast cancer cells resulted in an increase in the percentage of apoptotic cells compared to untreated controls (NT). Furthermore, disruption of IL-17 also increased the sensitivity of the cancer cells to paclitaxel (taxol) and doxorubicin compared to untreated controls (NT). In Figure 21E and 21F, the present inventors have further shown that IL-17 inhibition abrogates orthotropic (*i.e.,* in the mammary gland) tumor growth and metastasis of MDAMB231 cell line, which is one of the most aggressive human breast cancer cell lines available for preclincical evaluation. MDAMB231 cells expressing IL-17 or Control shRNA were engrafted in the mammary fat pad of nude mice and monitored primary tumor growth and metastasis. The present inventors have demonstrated that IL17A knocked down cells did not form macroscopically detectable mammary tumors whereas mice implanted with control shRNA cells developed aggressive lesions (Figure 21E). MDAMB231 is a highly metastatic cell line that disseminates to multiple organs in mice, such as the draining lymph node, the lungs and the liver. Whereas control cells widely disseminated in the draining lymph node (4 out of 5 mice), the lungs (5 out of 5) and the liver (2 out of 5), the present inventors have further demonstrated that IL17A knocked down MDAMB231 were unable to colonize the draining lymph node and to metastasize to the lungs and the liver (Figure 21F) except for 1 positive lymph node.

The present inventors have further illustrated with two other metastatic human breast cancer cell lines that IL-17 is a therapeutic target in breast cancer. The present inventors have indeed demonstrated that IL-17 blockade by neutralizing antibody in two other breast cancer cell lines that produced IL-17 (i.e., MDAMB435S and MDAMB468) similarly induced tumor cell death, increased sensitivity to paclitaxel and doxorubicin (Figure 22A, 22B) and restored an epithelial phenotype as shown by increased expression of epithelial markers (E-cadherin, Occludin, and α-Catenin) and decreased expression of mesdenchymal markers (Vimentin and N-cadherin) (Figure 22C).

As demonstrated by the above, IL-17 plays a key role in breast cancer cell survival, resistance to chemotherapeutic agents, growth, invasion, and metastasis. IL-17 can be produced by the tumor micro-environment or by the cancer cells themselves. IL-17 antagonists can induce cancer cell death and sensitize the cells to chemotherapeutic agents. IL-17 antagonization can also abrogate primary tumor growth and metastases *in vivo* in mouse models bearing human breast tumors. Hence, IL-17 expression and/or AID and/or Twist-1 expression are useful methods of diagnosing patients with breast cancer or metastatic breast cancer, or cancer at risk of metastasizing. Antagonists to IL-17, such as antibodies and/or other therapies, such as small molecule antagonists, nucleic acid antagonists and gene silencers (e.g., siRNA and shRNA) are useful methods of treating primary breast tumors, of treating and/or preventing breast cancer invasion and metastases.

### EXAMPLE 4: IL-17 and Hepatocellular Carcinoma (HCC)

It was shown that Activation Induced Deaminase (AID), that is not expressed in primary hepatocytes, is expressed in HCC (Kou T., et al., Int J Cancer, 120: 469-476, 2007) and in human liver inflammatory disease caused by hepatitis C virus (HCV) infection (Endo Y. et al., Oncogene, 26: 5587-5595, 2007). High expression of the Twist-1 protein was also found in HCC and correlated with poor prognosis and metastasis (Niu R.F. et al., J Exp Clin Cancer Res, 26: 385-394, 2007). Moreover it has been recently reported that increased numbers of intratumoral IL17-producing cells were correlated with a poor survival in HCC patients (Zhang J.P., et al., J Hepatol, 50: 980-989, 2009). The present inventors have demonstrated that following HCV (genotype 3A) infection, human primary hepatocytes acquire the capacity to secrete IL-17 in an autocrine manner. This is illustrated in Figure 23, which shows the amount of IL-17 measured by ELISA in cell supernatants of primary human hepatocytes infected or not with HCV. In the HCV-negative hepatocytes, IL-17 production is negligible, whereas in the HCV-infected hepatocytes, IL-17 expression reached about 100 pg/mL on day 5 and about 350 pg/mL on day 10.

The present inventors have demonstrated that IL-17 induces Twist-1 expression and cooperates with HCV to further increase Twist-1 expression and inhibit the expression of the tumor suppressor gene p53 in human primary hepatocytes, whereas TNF-α and TGF-β did not cooperate. Figure 24 compares the effects of administering TNF-α, TGF-β, or IL-17 to primary human hepatocytes that were infected or not with HCV genotype 3A. Treatment of uninfected primary hepatocytes for 5 days in the presence of IL-17 (10 ng/ml), but also TNFα (100 ng/ml) and TGFβ (8 ng/ml) upregulated the expression of the DNA mutator AID. IL-17 but not TNFα and TGFβ partially decreased the expression of the tumor suppressor p53 and its target p21. In the HCV-infected cells exposed to IL-17, there was an upregulation of AID, concomitantly with a strong upregulation of Twist-1 expression and a strong inhibition of the p53 tumor suppressor and its target p21. TNFα or TGFβ did not synergize with HCV in inhibiting p53 expression. Only the combination of IL-17 and HCV was accompanied by induction of expression of some oncogenes, e.g. c-Myc.

The present inventors have demonstrated that IL-17 also cooperates with HCV to transform primary hepatocytes into cancerous cells. Figure 25 shows human primary hepatocytes, infected or not with HCV genotype 3A and exposed to TNFα (100 ng/ml), TGFβ (8 ng/ml) or IL-17 (10 ng/ml) for 21 days and subjected to foci formation assay. When cultured in the presence of cytokines alone, primary hepatocytes did not acquire a transformed phenotype. When HCV-infected primary hepatocytes were cultured in the presence of TGFβ, only rare cells displayed a phenotype characteristic of transformed cells (Figure 25). When HCV-infected primary hepatocytes are cultured in the presence of IL-17, most of the cells showed a phenotype characteristic of transformed cells as demonstrated in foci formation assay. Of note, transformation is a characteristic of cancer cells.

The present inventors also showed that IL-17 induces Epithelial to Mesenchymal Transition and further cooperates with HCV to induce EMT, a process involved in liver fibrosis (Choi S.S. et al., Hepatology 50: 2007-2013, 2009; Thiery J-P. et al., Cell, 139: 871-890, 2009) and HCC metastases (Yang MH. et al., Hepatology, 50: 1464-1474, 2009). IL-17, but not TNF-α or TGF-β, induced EMT in human primary hepatocytes as illustrated by a switch from cobblestone-like to spindle like cell morphology of human primary hepatocytes that were infected with HCV(+HCV) or not (-HCV). Figure 26B also shows the expression of various epithelial and mesenchymal cell markers. IL-17 alone, but not TNFα or TGFβ, induced partial EMT as illustrated by the partial loss of epithelial markers such as E-Cadherin, α-Catenin and Occluding and gain of mesenchymal marker such as N-Cadherin and Vimentin (Figure 26B). IL-17 further cooperated with HCV to induce complete EMT in human primary hepatocytes as illustrated by complete loss of epithelial markers such as E-Cadherin, α-Catenin and Occluding and further increased expression of mesenchymal marker such as N-Cadherin and Vimentin (Figure 28B), whereas TGFβ cooperated with HCV to induce a partial EMT and TNFα had no effect (Figure 26B).

As demonstrated by the above, IL-17 secretion is induced upon infection by HCV in human primary hepatocyte. IL-17 alone or combined with HCV induces the DNA mutator AID and inhibits the p53 tumor suppressor via Twist-1 upregulation, resulting in activation of oncogenes such as c-Myc and hepatocyte transformation, potentially generating cancer cell. IL-17 alone or combined with HCV also induces EMT, which may favor HCC cell invasion and metastasis. Hence, IL-17 expression and/or AID and/or Twist-1 expression are useful methods of diagnosing patients with HCC or metastatic HCC, or cancer at risk of metastasizing. Antagonists to IL-17, such as antibodies and/or other therapies, such as small molecule antagonists, nucleic acid antagonists and gene silencers (e.g., siRNA and shRNA) are useful methods of treating HCC, of treating and/or preventing HCC invasion and metastases.

### EXAMPLE 5: IL-17 Expression in Other Cancers

In a screen of about 150 different human cancer cell lines, representing approximately 17 different types of human cancer, the present inventors showed that IL-17 secretion by cancer cells was observed for some cancer cell lines (Figure 27A-27F). In particular, the present inventors showed a high frequency of IL-17 secreting cell lines in colon, lung, ovary, head and neck, esophageal, and melanoma cancer cell lines, and lymphoma cell lines, as well as breast cancer cell lines. Hence, IL-17 expression and/or AID and/or Twist-1 expression are useful methods of diagnosing patients with breast, colon, lung, ovary, head and neck, esophageal cancers and melanomas or metastatic cancer, or cancer at risk of metastasizing. Antagonists to IL-17, such as antibodies and/or other therapies, such as small molecule antagonists, nucleic acid antagonists and gene silencers (e.g., siRNA and shRNA) may be useful methods of treating numerous human cancers including breast, colon, lung, ovary, head and neck, esophageal cancers and melanomas, of treating and/or preventing invasion and metastases of numerous human cancers including breast, colon, lung, ovary, head and neck, esophageal cancers and melanomas.

### EXAMPLE 6: IL-17 neutralizing antibodies

The present inventors showed that IL-17 neutralizing antibodies OREGA-56-8-12 (hybridoma deposited as CNCM-I-4476), OREGA-94-9-5 (hybridoma deposited as CNCM-I-4477) and OREGA-124-8-4 (hybridoma deposited as CNCM-I-4478) sensitize MDAMB231 breast cancer cells to chemotherapy. IL-17 neutralization by neutralizing antibodies OREGA-56-8-12, OREGA-94-9-5 and OREGA-124-8-4 or the reference antibody (Ebioscience) sensitizes MDAMB231 cell to paclitaxel (taxol) or doxorubicin (Figure 28)

## Claims

1. A pharmaceutical composition comprising:
- a chemotherapeutic agent chosen in the list consisting of docetaxel, doxorubicin and paclitaxel; and
- an IL-17 neutralizing antibody or an antigen binding fragment thereof for increasing the sensitivity of cancer cells to a chemotherapeutic agent;
for use in the treatment of cancer in a subject having a cancer associated with increased expression or production of IL-17.

2. Products containing a chemotherapeutic agent chosen in the list consisting of docetaxel, doxorubicin and paclitaxel, and an IL-17 neutralizing antibody or an antigen binding fragment thereof for increasing the sensitivity of cancer cells to a chemotherapeutic agent as a combined preparation for simultaneous, separate or sequential use in the treatment of cancer in a subject having a cancer associated with increased expression or production of IL-17.

3. A pharmaceutical composition for its use according to claim 1 or products for their use according to claim 2, wherein the cancer is a solid tumor.

4. A pharmaceutical composition for its use according to claim 3 or products for their use according to claim 3, wherein the solid tumor is selected from the group consisting of breast cancer, hepatocellular carcinoma, ovarian cancer, lung cancer, colorectal cancer, melanoma, oesophageal cancer, head and neck cancer, renal cell carcinoma, cervical carcinoma, fibrosarcoma, gastric cancer and prostate cancer, advantageously breast cancer, hepatocellular carcinoma, ovarian cancer, lung cancer, colorectal cancer, melanoma, oesophageal cancer and head and neck cancer.

5. A pharmaceutical composition for its use according to any one of claims 1 and 3 or products for their use according to any one of claims 2 to 3, wherein the cancer is a lymphoproliferative disease, advantageously an haematological malignancy, more advantageously a lymphoma.

## Patentansprüche

1. Eine pharmazeutische Zusammensetzung bestehend aus:
- einem chemotherapeutischen Wirkstoff, der aus der Liste ausgesucht wird, die aus Docetaxel, Doxorubicin und Paclitaxel besteht; und
- einem IL-17 neutralisierender Antikörper oder einem Antigen-bindenden Fragment von ihm zur Steigerung der Sensitivität von Krebszellen gegenüber einem chemotherapeutischen Wirkstoff;
zur Verwendung bei der Behandlung von Krebs bei einem Patienten mit einer Krebserkrankung mit erhöhter Expression oder Produktion von IL-17.

2. Produkte, die einen chemotherapeutischen Wirkstoff, die aus der Liste ausgesucht wird, die aus Docetaxel, Doxorubicin und Paclitaxel besteht, und einen IL-17 neutralisierenden Antikörper oder einen Antigen-bindenden Fragment von ihm zur Steigerung der Sensitivität von Krebszellen gegenüber einem chemotherapeutischen Wirkstoff, als kombiniertes Präparat zum gleichzeitigen, getrennten oder aufeinanderfolgenden Einsatz bei der Behandlung von Krebs bei einem Patienten mit einer Krebserkrankung mit erhöhter Expression oder Produktion von IL-17 enthalten.

3. Pharmazeutische Zusammensetzung zur Verwendung gemäß Anspruch 1 oder Produkte für den Einsatz gemäß Anspruch 2, wobei der Krebs ein solider Tumor ist.

4. Pharmazeutische Zusammensetzung zur Verwendung gemäß Anspruch 3 oder Produkte für den Einsatz gemäß Anspruch 3, wobei der solide Tumor aus der Gruppe ausgewählt wird, die aus Brustkrebs, Leberzellkarzinom, Eierstockkrebs, Lungenkrebs, Dickdarmkrebs, Melanom, Speiseröhrenkrebs, Kopf-Hals-Karzinom, Nierenzellkarzinom, Gebärmutterhalskrebs, Fibrosarkom, Magenkrebs und Prostatakrebs, am besten Brustkrebs, Leberzellkarzinom, Eierstockkrebs, Lungenkrebs, Dickdarmkrebs, Melanom, Speiseröhrenkrebs und Kopf-Hals-Karzinom besteht.

5. Pharmazeutische Zusammensetzung zur Verwendung gemäß einem der Ansprüche 1 und 3 oder Produkte für den Einsatz gemäß Anspruch 2 bis 3, wobei der Krebs eine lymphoproliferative Erkrankung, am besten eine hämatologische Malignität, noch besser ein Lymphom ist.

## Revendications

1. Composition pharmaceutique comprenant :
- un agent chimiothérapeutique choisi dans la liste constituée du docétaxel, de la doxorubicine et du paclitaxel ; et
- un anticorps neutralisant de IL-17 ou un fragment de celui-ci se liant à l'antigène pour augmenter la sensibilité des cellules cancéreuses à un agent chimiothérapeutique,
pour utilisation dans le traitement du cancer chez un sujet ayant un cancer associé à une augmentation de l'expression ou de la production de IL-17.

2. Produits contenant un agent chimiothérapeutique choisi dans la liste constituée du docétaxel, de la doxorubicine et du paclitaxel, et un anticorps neutralisant de IL-17 ou un fragment de celui-ci se liant à l'antigène pour augmenter la sensibilité des cellules cancéreuses à un agent chimiothérapeutique, comme préparation combinée pour utilisation simultanée, séparée ou séquentielle dans le traitement du cancer chez un sujet ayant un cancer associé à une augmentation de l'expression ou de la production de IL-17.

3. Composition pharmaceutique pour son utilisation selon la revendication 1 ou produits pour leur utilisation selon la revendication 2, dans lesquelles le cancer est une tumeur solide.

4. Composition pharmaceutique pour son utilisation selon la revendication 3 ou produits pour leur utilisation selon la revendication 3, dans lesquelles la tumeur solide est choisie dans le groupe constitué du cancer du sein, carcinome hépatocellulaire, cancer ovarien, cancer du poumon, cancer colorectal, mélanome, cancer de l'oesophage, cancer de la tête et du cou, carcinome à cellule rénales, carcinome cervical, fibrosarcome, cancer gastrique et cancer de la prostate, avantageusement cancer du sein, carcinome hépatocellulaire, cancer ovarien, cancer du poumon, cancer colorectal, mélanome, cancer de l'oesophage, cancer de la tête et du cou.

5. Composition pharmaceutique pour son utilisation selon l'une des revendications 1 à 3 ou produits pour leur utilisation selon l'une des revendications 2 à 3, dans lesquelles le cancer est une maladie lymphoproliférative, avantageusement une malignité hématologique, encore plus avantageusement un lymphome.


### SEQUENCE LISTING

<110> OREGA BIOTECH
   INSTITUT NATIONAL DE LA SANTE ET DE LA RECHERCHE MEDICALE
<120> Methods of Treating And/or Preventing Cell Proliferation Disorders with IL-17 Antagonists
<130> O57-B-33883 PCT
<150> 61/334,979
   <151> 2010-05-14
<160> 8
<170> PatentIn version 3.5
<210> 1
   <211> 155
   <212> PRT
   <213> Homo sapiens
<220>
   <223> HUMAN IL-17 AMINO ACID
<400> 1
   Met Thr Pro Gly Lys Thr Ser Leu Val Ser Leu Leu Leu Leu Leu Ser
   1               5                   10                  15
   Leu Glu Ala Ile Val Lys Ala Gly Ile Thr Ile Pro Arg Asn Pro Gly
               20                  25                  30
   Cys Pro Asn Ser Glu Asp Lys Asn Phe Pro Arg Thr Val Met Val Asn
           35                  40                  45
   Leu Asn Ile His Asn Arg Asn Thr Asn Thr Asn Pro Lys Arg Ser Ser
       50                  55                  60
   Asp Tyr Tyr Asn Arg Ser Thr Ser Pro Trp Asn Leu His Arg Asn Glu
   65                  70                  75                  80
   Asp Pro Glu Arg Tyr Pro Ser Val Ile Trp Glu Ala Lys Cys Arg His
                   85                  90                  95
   Leu Gly Cys Ile Asn Ala Asp Gly Asn Val Asp Tyr His Met Asn Ser
               100                 105                 110
   Val Pro Ile Gln Gln Glu Ile Leu Val Leu Arg Arg Glu Pro Pro His
           115                 120                 125
   Cys Pro Asn Ser Phe Arg Leu Glu Lys Ile Leu Val Ser Val Gly Cys
       130                 135                 140
   Thr Cys Val Thr Pro Ile Val His His Val Ala
   145                 150                 155
<210> 2
   <211> 468
   <212> DNA
   <213> Homo sapiens
<220>
   <223> HUMAN IL-17 mRNA
<400> 2
   atgactcctg ggaagacctc attggtgtca ctgctactgc tgctgagcct ggaggccata        60
   gtgaaggcag gaatcacaat cccacgaaat ccaggatgcc caaattctga ggacaagaac       120
   ttcccccgga ctgtgatggt caacctgaac atccataacc ggaataccaa taccaatccc       180
   aaaaggtcct cagattacta caaccgatcc acctcacctt ggaatctcca ccgcaatgag       240
   gaccctgaga gatatccctc tgtgatctgg gaggcaaagt gccgccactt gggctgcatc       300
   aacgctgatg ggaacgtgga ctaccacatg aactctgtcc ccatccagca agagatcctg       360
   gtcctgcgca gggagcctcc acactgcccc aactccttcc ggctggagaa gatactggtg       420
   tccgtgggct gcacctgtgt caccccgatt gtccaccatg tggcctaa                    468
<210> 3
   <211> 137
   <212> PRT
   <213> Artificial Sequence
<220>
   <223> Recombinant human IL-17
<400> 3
   Met Ile Val Lys Ala Gly Ile Thr Ile Pro Arg Asn Pro Gly Cys Pro
   1               5                   10                  15
   Asn Ser Glu Asp Lys Asn Phe Pro Arg Thr Val Met Val Asn Leu Asn
               20                  25                  30
   Ile His Asn Arg Asn Thr Asn Thr Asn Pro Lys Arg Ser Ser Asp Tyr
           35                  40                  45
   Tyr Asn Arg Ser Thr Ser Pro Trp Asn Leu His Arg Asn Glu Asp Pro
       50                  55                  60
   Glu Arg Tyr Pro Ser Val Ile Trp Glu Ala Lys Cys Arg His Leu Gly
   65                  70                  75                  80
   Cys Ile Asn Ala Asp Gly Asn Val Asp Tyr His Met Asn Ser Val Pro
                   85                  90                  95
   Ile Gln Gln Glu Ile Leu Val Leu Arg Arg Glu Pro Pro His Cys Pro
               100                 105                 110
   Asn Ser Phe Arg Leu Glu Lys Ile Leu Val Ser Val Gly Cys Thr Cys
           115                 120                 125
   Val Thr Pro Ile Val His His Val Ala
       130                 135
<210> 4
   <211> 285
   <212> PRT
   <213> Homo sapiens
<220>
   <223> Human BAFF Isoform 1
<400> 4
   Met Asp Asp Ser Thr Glu Arg Glu Gln Ser Arg Leu Thr Ser Cys Leu
   1               5                   10                  15
   Lys Lys Arg Glu Glu Met Lys Leu Lys Glu Cys Val Ser Ile Leu Pro
               20                  25                  30
   Arg Lys Glu Ser Pro Ser Val Arg Ser Ser Lys Asp Gly Lys Leu Leu
           35                  40                  45
   Ala Ala Thr Leu Leu Leu Ala Leu Leu Ser Cys Cys Leu Thr Val Val
       50                  55                  60
   Ser Phe Tyr Gln Val Ala Ala Leu Gln Gly Asp Leu Ala Ser Leu Arg
   65                  70                  75                  80
   Ala Glu Leu Gln Gly His His Ala Glu Lys Leu Pro Ala Gly Ala Gly
                   85                  90                  95
   Ala Pro Lys Ala Gly Leu Glu Glu Ala Pro Ala Val Thr Ala Gly Leu
               100                 105                 110
   Lys Ile Phe Glu Pro Pro Ala Pro Gly Glu Gly Asn Ser Ser Gln Asn
           115                 120                 125
   Ser Arg Asn Lys Arg Ala Val Gln Gly Pro Glu Glu Thr Val Thr Gln
       130                 135                 140
   Asp Cys Leu Gln Leu Ile Ala Asp Ser Glu Thr Pro Thr Ile Gln Lys
   145                 150                 155                 160
   Gly Ser Tyr Thr Phe Val Pro Trp Leu Leu Ser Phe Lys Arg Gly Ser
                   165                 170                 175
   Ala Leu Glu Glu Lys Glu Asn Lys Ile Leu Val Lys Glu Thr Gly Tyr
                   180                 185                 190
   Phe Phe Ile Tyr Gly Gln Val Leu Tyr Thr Asp Lys Thr Tyr Ala Met
           195                 200                 205
   Gly His Leu Ile Gln Arg Lys Lys Val His Val Phe Gly Asp Glu Leu
       210                 215                 220
   Ser Leu Val Thr Leu Phe Arg Cys Ile Gln Asn Met Pro Glu Thr Leu
   225                 230                 235                 240
   Pro Asn Asn Ser Cys Tyr Ser Ala Gly Ile Ala Lys Leu Glu Glu Gly
                   245                 250                 255
   Asp Glu Leu Gln Leu Ala Ile Pro Arg Glu Asn Ala Gln Ile Ser Leu
               260                 265                 270
   Asp Gly Asp Val Thr Phe Phe Gly Ala Leu Lys Leu Leu
           275                 280                 285
<210> 5
   <211> 858
   <212> DNA
   <213> Homo sapiens
<220>
   <223> Human BAFF Isoform 1 mRNA
<400> 5
   atggatgact ccacagaaag ggagcagtca cgccttactt cttgccttaa gaaaagagaa        60
   gaaatgaaac tgaaggagtg tgtttccatc ctcccacgga aggaaagccc ctctgtccga       120
   tcctccaaag acggaaagct gctggctgca accttgctgc tggcactgct gtcttgctgc       180
   ctcacggtgg tgtctttcta ccaggtggcc gccctgcaag gggacctggc cagcctccgg       240
   gcagagctgc agggccacca cgcggagaag ctgccagcag gagcaggagc ccccaaggcc       300
   ggcctggagg aagctccagc tgtcaccgcg ggactgaaaa tctttgaacc accagctcca       360
   ggagaaggca actccagtca gaacagcaga aataagcgtg ccgttcaggg tccagaagaa       420
   acagtcactc aagactgctt gcaactgatt gcagacagtg aaacaccaac tatacaaaaa       480
   ggatcttaca catttgttcc atggcttctc agctttaaaa ggggaagtgc cctagaagaa       540
   aaagagaata aaatattggt caaagaaact ggttactttt ttatatatgg tcaggtttta       600
   tatactgata agacctacgc catgggacat ctaattcaga ggaagaaggt ccatgtcttt       660
   ggggatgaat tgagtctggt gactttgttt cgatgtattc aaaatatgcc tgaaacacta       720
   cccaataatt cctgctattc agctggcatt gcaaaactgg aagaaggaga tgaactccaa       780
   cttgcaatac caagagaaaa tgcacaaata tcactggatg gagatgtcac attttttggt       840
   gcattgaaac tgctgtga                                                     858
<210> 6
   <211> 266
   <212> PRT
   <213> Homo sapiens
<220>
   <223> Human BAFF Isoform 2
<400> 6
   Met Asp Asp Ser Thr Glu Arg Glu Gln Ser Arg Leu Thr Ser Cys Leu
   1               5                   10                  15
   Lys Lys Arg Glu Glu Met Lys Leu Lys Glu Cys Val Ser Ile Leu Pro
               20                  25                  30
   Arg Lys Glu Ser Pro Ser Val Arg Ser Ser Lys Asp Gly Lys Leu Leu
           35                  40                  45
   Ala Ala Thr Leu Leu Leu Ala Leu Leu Ser Cys Cys Leu Thr Val Val
       50                  55                  60
   Ser Phe Tyr Gln Val Ala Ala Leu Gln Gly Asp Leu Ala Ser Leu Arg
   65                  70                  75                  80
   Ala Glu Leu Gln Gly His His Ala Glu Lys Leu Pro Ala Gly Ala Gly
                   85                  90                  95
   Ala Pro Lys Ala Gly Leu Glu Glu Ala Pro Ala Val Thr Ala Gly Leu
               100                 105                 110
   Lys Ile Phe Glu Pro Pro Ala Pro Gly Glu Gly Asn Ser Ser Gln Asn
           115                 120                 125
   Ser Arg Asn Lys Arg Ala Val Gln Gly Pro Glu Glu Thr Gly Ser Tyr
       130                 135                 140
   Thr Phe Val Pro Trp Leu Leu Ser Phe Lys Arg Gly Ser Ala Leu Glu
   145                 150                 155                 160
   Glu Lys Glu Asn Lys Ile Leu Val Lys Glu Thr Gly Tyr Phe Phe Ile
                   165                 170                 175
   Tyr Gly Gln Val Leu Tyr Thr Asp Lys Thr Tyr Ala Met Gly His Leu
               180                 185                 190
   Ile Gln Arg Lys Lys Val His Val Phe Gly Asp Glu Leu Ser Leu Val
           195                 200                 205
   Thr Leu Phe Arg Cys Ile Gln Asn Met Pro Glu Thr Leu Pro Asn Asn
   210 215 220
   Ser Cys Tyr Ser Ala Gly Ile Ala Lys Leu Glu Glu Gly Asp Glu Leu
   225 230 235 240
   Gln Leu Ala Ile Pro Arg Glu Asn Ala Gln Ile Ser Leu Asp Gly Asp
   245 250 255
   Val Thr Phe Phe Gly Ala Leu Lys Leu Leu
   260 265
<210> 7
   <211> 801
   <212> DNA
   <213> Homo sapiens
<220>
   <223> Human BAFF Isoform 2 mRNA
<400> 7
   atggatgact ccacagaaag ggagcagtca cgccttactt cttgccttaa gaaaagagaa 60
   gaaatgaaac tgaaggagtg tgtttccatc ctcccacgga aggaaagccc ctctgtccga 120
   tcctccaaag acggaaagct gctggctgca accttgctgc tggcactgct gtcttgctgc 180
   ctcacggtgg tgtctttcta ccaggtggcc gccctgcaag gggacctggc cagcctccgg 240
   gcagagctgc agggccacca cgcggagaag ctgccagcag gagcaggagc ccccaaggcc 300
   ggcctggagg aagctccagc tgtcaccgcg ggactgaaaa tctttgaacc accagctcca 360
   ggagaaggca actccagtca gaacagcaga aataagcgtg ccgttcaggg tccagaagaa 420
   acaggatctt acacatttgt tccatggctt ctcagcttta aaaggggaag tgccctagaa 480
   gaaaaagaga ataaaatatt ggtcaaagaa actggttact tttttatata tggtcaggtt 540
   ttatatactg ataagaccta cgccatggga catctaattc agaggaagaa ggtccatgtc 600
   tttggggatg aattgagtct ggtgactttg tttcgatgta ttcaaaatat gcctgaaaca 660
   ctacccaata attcctgcta ttcagctggc attgcaaaac tggaagaagg agatgaactc 720
   caacttgcaa taccaagaga aaatgcacaa atatcactgg atggagatgt cacatttttt 780
   ggtgcattga aactgctgtg a 801
<210> 8
   <211> 153
   <212> PRT
   <213> Artificial Sequence
<220>
   <223> Recombinant human soluble BAFF
<400> 8
   Met Ala Val Gln Gly Pro Glu Glu Thr Val Thr Gln Asp Cys Leu Gln
   1               5                   10                  15
   Leu Ile Ala Asp Ser Glu Thr Pro Thr Ile Gln Lys Gly Ser Tyr Thr
               20                  25                  30
   Phe Val Pro Trp Leu Leu Ser Phe Lys Arg Gly Ser Ala Leu Glu Glu
           35                  40                  45
   Lys Glu Asn Lys Ile Leu Val Lys Glu Thr Gly Tyr Phe Phe Ile Tyr
       50                  55                  60
   Gly Gln Val Leu Tyr Thr Asp Lys Thr Tyr Ala Met Gly His Leu Ile
   65                  70                  75                  80
   Gln Arg Lys Lys Val His Val Phe Gly Asp Glu Leu Ser Leu Val Thr
                   85                  90                  95
   Leu Phe Arg Cys Ile Gln Asn Met Pro Glu Thr Leu Pro Asn Asn Ser
               100                 105                 110
   Cys Tyr Ser Ala Gly Ile Ala Lys Leu Glu Glu Gly Asp Glu Leu Gln
           115                 120                 125
   Leu Ala Ile Pro Arg Glu Asn Ala Gln Ile Ser Leu Asp Gly Asp Val
       130                 135                 140
   Thr Phe Phe Gly Ala Leu Lys Leu Leu
   145                 150